(19) Europäisches Patentamt
European Patent Office
Office européen des brevets

(11) EP 4 029 858 A1

(12) EUROPEAN PATENT APPLICATION
published in accordance with Art. 153(4) EPC

(43) Date of publication:
**20.07.2022 Bulletin 2022/29**

(21) Application number: **20861117.8**

(22) Date of filing: **04.09.2020**

(51) International Patent Classification (IPC):
***C07D 255/00*** $^{(2006.01)}$ ***C07D 255/02*** $^{(2006.01)}$
***C07D 401/02*** $^{(2006.01)}$ ***C07D 401/12*** $^{(2006.01)}$
***C07D 403/12*** $^{(2006.01)}$ ***A61K 31/506*** $^{(2006.01)}$
***A61P 35/00*** $^{(2006.01)}$ ***A61P 19/02*** $^{(2006.01)}$

(52) Cooperative Patent Classification (CPC):
**A61K 31/506; A61P 1/16; A61P 3/00; A61P 3/10; A61P 9/00; A61P 11/00; A61P 13/12; A61P 17/00; A61P 19/02; A61P 25/00; A61P 25/04; A61P 29/00; A61P 35/00; A61P 37/00; C07D 255/00;** (Cont.)

(86) International application number:
**PCT/CN2020/113474**

(87) International publication number:
**WO 2021/043260 (11.03.2021 Gazette 2021/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.09.2019 CN 201910841159**
**06.09.2019 CN 201910846545**

(71) Applicant: **Wuhan Humanwell Innovative Drug Research and Development Center Limited Company**
**Wuhan, Hubei 430075 (CN)**

(72) Inventors:
- **ZHANG, Xuejun**
  **Wuhan, Hubei 430075 (CN)**
- **LI, Lie**
  **Wuhan, Hubei 430075 (CN)**
- **SHEN, Jie**
  **Wuhan, Hubei 430075 (CN)**
- **WEI, Wenjun**
  **Wuhan, Hubei 430075 (CN)**
- **LEI, Sijun**
  **Wuhan, Hubei 430075 (CN)**
- **DING, Xiaohua**
  **Wuhan, Hubei 430075 (CN)**
- **ZANG, Yang**
  **Wuhan, Hubei 430075 (CN)**
- **SUN, Hongna**
  **Wuhan, Hubei 430075 (CN)**
- **FU, Qiangqiang**
  **Wuhan, Hubei 430075 (CN)**

(74) Representative: **Habermann, Hruschka & Schnabel**
**Patentanwälte**
**Montgelasstraße 2**
**81679 München (DE)**

# (54) PYRIMIDINE COMPOUND AND PREPARATION METHOD THEREFOR

(57) The present disclosure relates to pyrimidine compounds, and more specifically, relates to pyrimidine compounds, preparation methods thereof, and uses thereof in manufacture of medicaments. Provided is a compound represented by Formula (I) or a tautomer, stereoisomer, hydrate, solvate, pharmaceutical acceptable salt, or prodrug of the compound represented by Formula (I). The compound has a significant inhibitory effect on ATX enzyme, exhibits excellent liver metabolism stability, is metabolized more slowly in the human body, and has higher exposure.

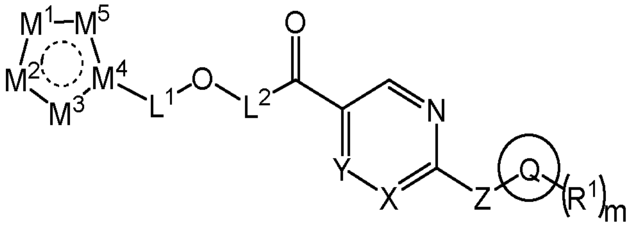

(I)

(52) Cooperative Patent Classification (CPC): (Cont.)
**C07D 255/02; C07D 401/02; C07D 401/12; C07D 401/14; C07D 403/12; C07D 403/14**

## Description

## CROSS-REFERENCE TO RELATED APPLICATIONS

This application claims priority to Chinese Patent Application No. 201910841159.9, titled "PYRIMIDINE COMPOUND AND PREPARATION METHOD THEREFOR", and Chinese Patent Application No. 201910846545.7, titled "PYRIMIDINE DERIVATIVES AND USE THEREOF", both filed on September 6, 2019 with the China National Intellectual Property Administration, the entire disclosures of which are hereby incorporated by their references.

## FIELD

The present disclosure belongs to the field of medicinal chemistry. Specifically, the present disclosure relates to pyrimidine compounds, and more specifically, the present disclosure relates to pyrimidine compounds, preparation methods therefor, and uses thereof in preparation of medicaments.

## BACKGROUND

Autotaxin (abbreviated as ATX) is a secreted glycoprotein having phosphodiesterase (PDE) activity, and is a member of the extracellular pyrophosphatase/phosphodiesterase (ENPP) family. Thus, autotaxin is also called ENPP2. ATX also has lysophospholipase D (LysoPLD) activity, and can hydrolyze lysophosphatidylcholine (LPC) into lysophosphatidic acid (LPA) having biological activity. LPA is an intracellular lipid mediator that affects many biological and biochemical processes.

Studies have indicated that, under pathological conditions, a level of LPA can be lowered by inhibiting ATX, thereby providing therapeutic benefits for unmet clinical needs. The related diseases include cancer, lymphocyte homing, chronic inflammation, neuropathic pain, fibrosis, thrombosis, cholestatic itching, or fibrosis diseases that are induced, mediated, and/or spread through an elevated level of LPA and/or an activation of ATX.

Up-regulation of the ATX-LPA signaling pathway can be observed in various inflammatory conditions. For example, the pro-inflammatory effects of LPA include mast cell degranulation, smooth muscle cell contraction, and release of cytokine from dendritic cells. As a manifestation of a general role of LPA in inflammation, the up-regulation of the ATX-LPA signaling pathway was observed in a mouse carrageenan air pouch model, which is adopted to develop anti-inflammatory drugs, including cyclooxygenase inhibitors for arthritis (Hidenobu Kanda, Rebecca Newton, Russell Klein et al., Autotaxin, an ectoenzyme that produces lysophosphatidic acid, promotes the entry of lymphocytes into secondary lymphoid organs[J] Nature Immunology. 2008, 9(4):415-423.). In addition, a reduction of LPA in plasma and in an air pouch has been observed in a rat carrageenan air pouch model using an ATX inhibitor, which confirms the role of ATX as the main source of LPA during inflammation. As another general role of LPA in inflammatory diseases, a "synergistic effect" between LPA and lymphocyte migration chemokines has been confirmed. High ATX expression can be found in chronic inflammation sites. It has been confirmed that the homing of T-cells to lymphatic tissues is inhibited through intravenously injecting the inactivated ATX, which may be achieved by competing with endogenous ATX and exerting a dominant negative effect. In some cases, ATX facilitates the entry of lymphocytes into lymphoid organs. Therefore, ATX inhibitors can block the migration of lymphocytes into secondary lymphoid organs and bring benefits for autoimmune diseases.

In rheumatoid arthritis, it has been confirmed that ATX expression is increased in synovial fibroblasts from patients with rheumatoid arthritis (RA), and the elimination of ATX expression in interstitial cells (including synovial fibroblasts) results in symptom weakening in a mouse model of rheumatoid arthritis. As such, the role of autotaxin in rheumatoid arthritis has been fully established.

LPA can also up-regulate pain-related proteins through one of its homologous receptors, $LPA_1$. A targeted inhibition against ATX-mediated biosynthesis of LPA can serve as a mechanism for preventing neuropathic pain caused by nerve damage, such as pain associated with osteoarthritis. It has been observed that autotaxin inhibitors reduce LPA and PGE2 and also alleviate the inflammatory pain. It has also been indicated through research that the targeted inhibition against ATX-mediated biosynthesis of LPA can be a new mechanism for preventing neuropathic pain caused by nerve damage.

After the inflammation subsides and the tissue damage is repaired, the tissue usually recovers to its original state. When no longer needed, excessive and uncontrolled tissue repair may lead to commonly called fibrosis. Fibrosis is characterized by excessive deposition of extracellular matrix components and excessive growth of fibroblasts. Fibrosis may occur in all tissues, but it is especially common in organs that are frequently chemically and biologically damaged, including lungs, skin, digestive tract, kidneys, and liver. Fibrosis often seriously harms the normal functions of organs.

In some cases, LPA stimulates the proliferation of hepatic stellate cells while inhibiting DNA synthesis in hepatocytes. An LPA level and a serum ATX activity are elevated in patients with chronic hepatitis C. In the blood of rabbits

with different liver injuries, a plasma LPA concentration and a serum ATX activity are relatively higher in carbon tetrachloride-induced liver fibrosis. The plasma LPA concentration and the serum ATX activity increase with the severity of different liver injuries.

Pulmonary fibrosis is the end-stage change of a large group of lung diseases characterized by the proliferation of fibroblasts and the accumulation of a large amount of extracellular matrix accompanied by inflammatory damage and destruction of tissue structure, i.e., structural abnormality (scar formation) caused by abnormal repair after the normal alveolus tissues are damaged. When the lung damage is attributed to various causes, the interstitial tissues will secrete collagen for repair. If it is repaired excessively, i.e., fibroblasts are excessively proliferated and a large amount of extracellular matrix is accumulated, the pulmonary fibrosis will be developed.

LPA signal has the effect of promoting fibrosis on epithelial cells, endothelial cells and fibroblasts specifically through the $LPA_1$ receptor: genetic deletion of this receptor reduces epithelial cell apoptosis, vascular leakage and fibroblast accumulation in the pulmonary fibrosis model.

Idiopathic pulmonary fibrosis (IPF) is a chronic, progressive, and fibrotic interstitial pneumonia with unknown etiology, characterized by diffuse alveolitis and alveolar structural disorders, and typically revealing as ordinary interstitial pneumonia in imaging and pathologic histology. As the course of the disease progresses, the pulmonary fibrosis occurs, the lung tissue of the patient becomes thicker and harder, resulting in permanent scars, or the formation of honeycomb-like lung of the patient, also vividly referred to as "honeycomb lung" or "luffa lung". This chronic progressive disease will cause an irreversible and continuous decline in lung function. 50% of the patients may have an average survival time of only 2.8 years since they were diagnosed. Thus, the idiopathic pulmonary fibrosis is also referred to as "tumor-like disease." At present, the existing drug treatments have the problems such as many adverse reactions, poor therapeutic effects; and the non-drug treatments mainly include lung transplantation, but organ transplantation is expensive and limited in resources, and certain clinical risks accompanies.

There is evidence demonstrating that the proliferation and contraction of fibroblasts and extracellular matrix secretion stimulated by LPA promote fibrous proliferation in other airway diseases, such as peribronchial fibrosis present in chronic bronchitis and interstitial lung disease as well as severe asthma. LPA plays a role in fibrosis interstitial lung disease and bronchiolitis obliterans, in which collagen and myofibroblasts are both increased. Studies related to idiopathic pulmonary fibrosis (IPF) have indicated that the LPA level in patients' bronchoalveolar lavage fluid is increased. Further research on LPA1 knockout and inhibitors has revealed that LPA plays a key role in the process of lung fibrosis, which is supplemented by the investigation using cell-specific knockout mice lacking ATX in bronchial epithelial cells and macrophages. It has been indicated that these mice were less sensitive to the lung fibrosis model. The role of LPA in other fibrosis diseases (kidney and skin) is based on similar observations. The role of LPA in lung remodeling is related to the effects of LPA on both lung fibroblasts (via LPA1) and epithelial cells (via LPA2). It has been indicated that LPA2 plays a pivotal role in the activation of TGF$\beta$ in epithelial cells in the case of fibrosis disorders. The roles of LPA in remodeling and fibrosis are related to COPD, IPF and asthma, and lung remodeling, as a long-term result, will limit lung function. Finally, for the focus on lung diseases, ATX is one of the three main quantitative trait loci that seem to be associated with lung function differences in mice.

The study found that LPA concentration increases in plasma and ascites in patients with ovarian cancer at the early and late stages. The elevated LPA level and changes in expression and response of LPA receptors may be one of the causes for the onset, progression or outcome of ovarian cancer. LPA is also associated with prostate cancer, breast cancer, melanoma cancer, head and neck cancer, bowel cancer, brain cancer and thyroid cancer. LPA is involved in tumor cell proliferation and invasion of adjacent tissues, leading to metastasis. These biological and pathobiological processes are initiated by activation of G protein-coupled receptors with LPA. The LPA level can be lowered by inhibiting enzymes involving in the LPA biosynthesis, such as ATX, for treatment of tumor patients.

In the process of angiogenesis, ATX and other angiogenic factors together lead to angiogenesis. The tumor can be nourished through the angiogenesis during tumor growth. Accordingly, an important starting point for cancer and tumor treatment is to inhibit the angiogenesis. Patent Application WO2014202458A1 recites the effects of ATX-LPA signaling in different pathophysiological conditions, such as proliferative diseases, neuropathic pain, inflammation, autoimmune diseases, fibrosis, lymphocyte tracing in lymph nodes, obesity, diabetes, or embryos blood vessel formation.

At present, certain progresses in the treatments of cancer, fibrosis diseases, proliferative diseases, inflammatory diseases, autoimmune diseases, respiratory diseases, cardiovascular diseases, neurodegenerative diseases, dermatological disorders, and/or abnormal angiogenesis-related diseases have been made, but they are still insufficient. The currently marketed IPF therapeutic drugs include Pirfenidone and Nintedanib. Pirfenidone may result in liver damage (such as liver failure, jaundice), hypersensitivity reactions (such as facial swelling, laryngeal edema, dyspnea, wheezing, etc.), and severe gastrointestinal reactions, and photogenotoxicity assays demonstrated that it may cause structural abnormality of chromosomes and may cause skin cancer under light exposure. Nintedanib has adverse reactions such as diarrhea, nausea, and abdominal pain, the incidence of gastrointestinal reactions can be as high as 50%, and common adverse reactions thereof include weight loss, loss of appetite, liver damage, and bleeding, etc. Among the patients who were taking Pirfenidone and Nintedanib, the probability of discontinuation due to serious adverse events was 20.9%

and 26.3%, respectively (Toby M Maher, et al. Rationale, design and objectives of two phase III, randomised, placebocontrolled studies of GLPG1690, a novel autotaxin inhibitor, in idiopathic pulmonary fibrosis (ISABELA 1 and 2)[J]. BMJ Open Respiratory Research. 2019, 21;6(1).). The living quality of IPF patients will be severely affected, and neither Pirfenidone nor Nintedanib could improve the living quality of the patients in clinical trials. Although both of these two drugs may improve overall results, they can only delay the course of the disease but cannot reverse pulmonary fibrosis. In this regard, the patients with severe specific pulmonary fibrosis may not benefit therefrom. GLPG-1690, as one of the medicaments for treating IPF that is now under development and has made rapid progress, exhibits a trend of reversing the course of the disease, but it has the problems of low enzyme activity, large dosage of clinical medication, and poor medication compliance. Therefore, the currently existing therapies are still unsatisfactory. There are still a large number of patients who are in need of new treatments with higher activity and better efficacy, in order to slow down the course of the disease to a greater extent or even reverse the course of the disease, improve medication compliance, and allow more patients with idiopathic pulmonary fibrosis to benefit therefrom.

In view of the above, the present disclosure, on the basis of the prior art, designs a compound represented by Formula (I) to provide an ATX inhibitor with novel structure, better pharmacokinetic properties, better pharmacodynamics, and strong druggability, for effective treatment of ATX-related diseases and conditions, including but not limited to cancer, metabolic diseases, kidney diseases, liver diseases, fibrotic diseases, interstitial lung diseases, pulmonary fibrosis, liver fibrosis, proliferative diseases, inflammatory diseases, pain, osteoarthritis-related pain, autoimmune diseases, respiratory diseases, cardiovascular diseases, neurodegenerative diseases, dermatological disorders and/or abnormal angiogenesis related diseases.

## SUMMARY

The present disclosure aims to solve at least one of the above technical problems or at least provide a useful business option.

In one aspect of the present disclosure, the present disclosure provides a compound, which is a compound represented by Formula (I), or a tautomer, stereoisomer, hydrate, solvate, pharmaceutical acceptable salt, or prodrug of the compound represented by Formula (I):

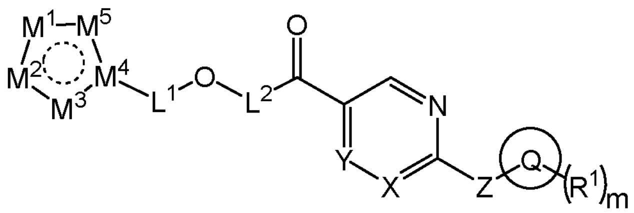

(I)

in which Q is selected from $C_3$-$C_{10}$ cycloalkyl, a three- to ten-membered heterocyclic group, $C_6$-$C_{10}$ aryl, or a five- to ten-membered heteroaryl; the $C_3$-$C_{10}$ cycloalkyl, the three- to ten-membered heterocyclic group, the $C_6$-$C_{10}$ aryl, and the five- to ten-membered heteroaryl are optionally substituted by m groups $R^1$, m is an integer selected from 0 to 9;

each of the m groups $R^1$ is independently selected from hydrogen, halogen, -CN, -OH, -SH, $-NO_2$, $C_1$-$C_{10}$ alkyl, $C_3$-$C_{10}$ cycloalkyl, or $C_1$-$C_{10}$ alkoxy;
X and Y are independently selected from -N= or $-C(R^2)=$;
Z is selected from -O-, -S-, or $-N(R^3)-$;
$L^1$ is selected from a single bond or

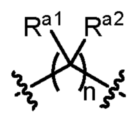

;

$L^2$ is selected from a single bond or

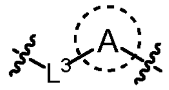;

$L^3$ is selected from a single bond or

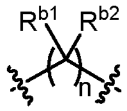;

$R^{a1}$, $R^{a2}$, $R^{b1}$, and $R^{b2}$ are each independently selected from hydrogen, halogen, cyano, -OH, or $C_1$-$C_3$ alkyl;

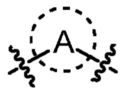

is selected from $C_3$-$C_8$ cycloalkyl, $C_4$-$C_8$ heterocycloalkyl, or a seven- to eight-membered N-spirocyclic group, and the $C_3$-$C_8$ cycloalkyl, the $C_4$-$C_8$ heterocycloalkyl, and the seven- to eight-membered N-spirocyclic group are optionally substituted with at least one $R^C$;
each of the at least one $R^C$ is independently selected from -H, halogen, -OH, -CN, or $C_1$-$C_3$ alkyl;
n is an integer selected from 0 to 3;
$M^1$, $M^2$, $M^3$, $M^4$, and $M^5$ are each independently selected from -N=, -N($R^4$)-, or -C($R^5$)=, at least one of $M^1$, $M^2$, $M^3$, $M^4$, or $M^5$ is -N= or -N($R^4$)-, and at least one of $M^1$, $M^2$, $M^3$, $M^4$, or $M^5$ is -C($R^5$)=;
$R^2$ and $R^5$ are each independently selected from hydrogen, halogen, -CN, -OH, -SH, or $C_1$-$C_3$ alkyl;
$R^3$ and $R^4$ are each independently selected from hydrogen or $C_1$-$C_3$ alkyl; and
the compound includes none of the following compounds or enantiomers or stereoisomers thereof:

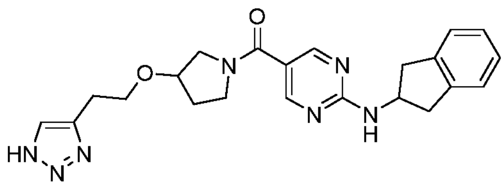 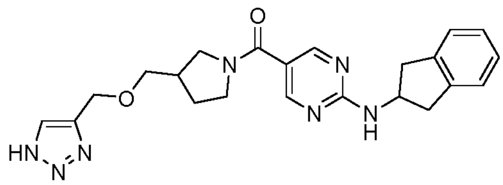

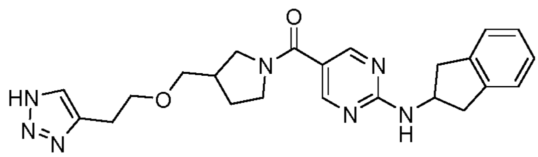 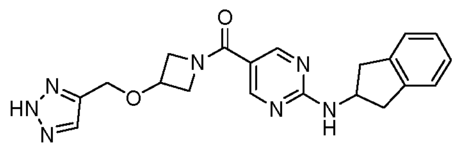

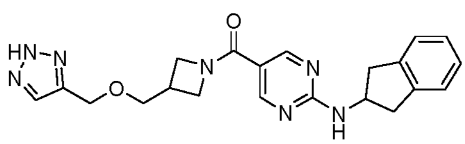 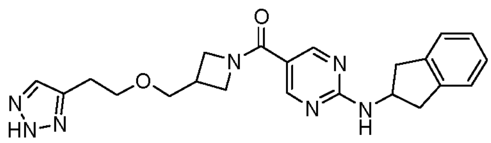

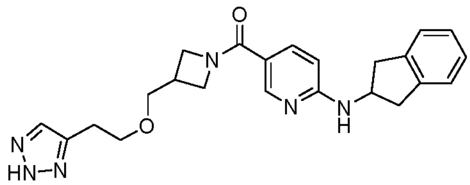 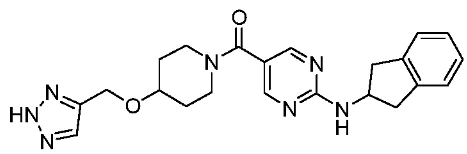

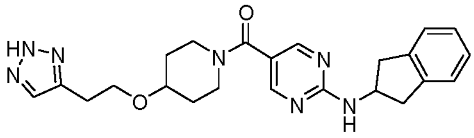

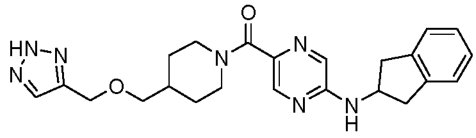

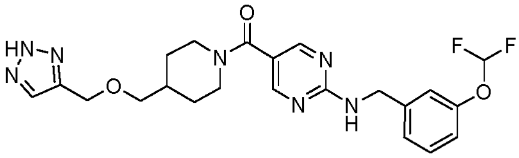

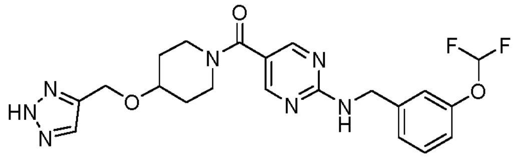

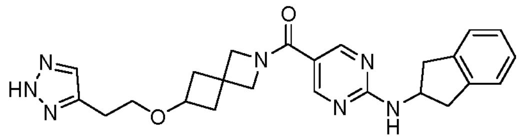

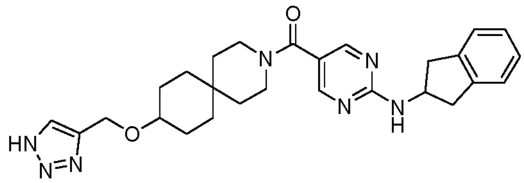

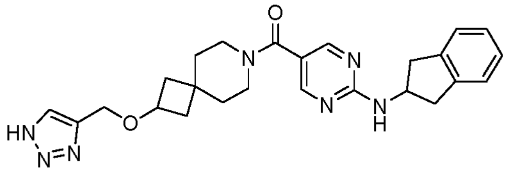

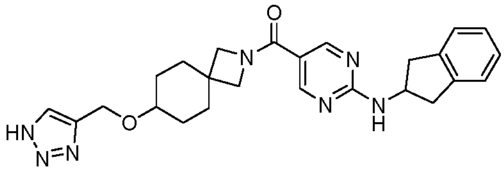

.

The inventors synthesized a new compound represented by Formula (I) and found that the compound or a pharmaceutically acceptable salt, tautomer, stereoisomer, hydrate, solvate or prodrug thereof has significant inhibitory effect on ATX enzyme, and exhibits excellent liver metabolism stability, and is metabolized more slowly in the human body and has higher exposure.

According to exemplary embodiments of the present disclosure, in the compound represented by Formula (I), Q is selected from cyclopropyl, cyclobutyl, cyclopentyl, cyclohexyl, azetidinyl, pyrrolidinyl, tetrahydrofuranyl, piperidinyl, piperazinyl, morpholinyl, phenyl, indenyl, naphthyl, pyrrolyl, pyrazolyl, imidazolyl, triazolyl, tetrazolyl, furyl, thienyl, thiazolyl, oxazolyl, pyridinyl, pyrimidinyl, pyrazinyl, pyridazinyl, benzimidazolyl, indolyl, or quinolinyl; m is 0, 1, 2 or 3; and $M^1$, $M^2$, and $M^3$ are all selected from -N= or -N($R^4$)-, and each of $M^4$ and $M^5$ is -C($R^5$)=.

According to embodiments of the present disclosure, each of the m groups $R^1$ is independently selected from hydrogen, halogen, or $C_1$-$C_3$ alkyl, and preferably, $R^1$ is selected from H, F, Cl, methyl, or ethyl; and preferably, $R^1$ is selected from H, F, Cl, methyl, or ethyl;

optionally, when Q is indenyl, the indenyl is optionally unsubstituted with $R^1$ or substituted with one or two groups $R^1$;

optionally, when X is -N=, Y is -C($R^2$)=; or when X is -C($R^2$)=, Y is -N=;

optionally, Z is selected from -O-, -S-, or -N($R^3$)-; when Z is -N($R^3$)-, $R^3$ is hydrogen or $C_1$-$C_3$ alkyl;

optionally, when $L^1$ is

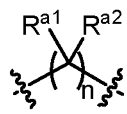

,

n is 1 or 2;

optionally, when $L^1$ is

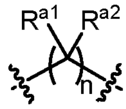,

$R^{a1}$ and $R^{a2}$ are each independently selected from hydrogen, F, Cl, -CN, methyl, or ethyl;

optionally, when $L^1$ is

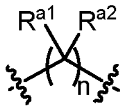,

$R^{a1}$ and $R^{a2}$ are each independently selected from hydrogen, F, Cl, -CN, methyl, or ethyl, and at least one $R^{a1}$ is hydrogen;

optionally, when $L^3$ is

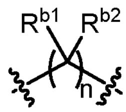,

n is 1 or 2;

optionally, when $L^3$ is

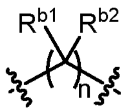,

$R^{b1}$ and $R^{b2}$ are each independently selected from hydrogen, -F, -C1, -CN, methyl, or ethyl;

optionally, when $L^3$ is

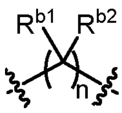,

$R^{b1}$ and $R^{b2}$ are each independently selected from hydrogen, -F, -Cl, -CN, methyl, or ethyl, and at least one $R^{b1}$ is hydrogen;

optionally, when said $L^2$ is

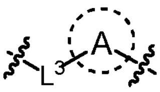, 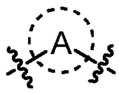

is selected from the following groups unsubstituted or optionally substituted with at least one $R^C$: piperidinyl, or a seven- to eight-membered N-spirocyclic group;

optionally, when said $L^2$ is

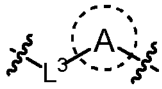, 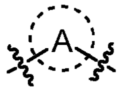

is piperidinyl unsubstituted or optionally substituted with at least one $R^C$, said $L^3$ and N on the piperidinyl are independently in an ortho, meta or para position relative to each other;

optionally, when said $L^2$ is

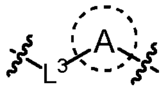, 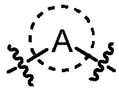

is piperidinyl unsubstituted or optionally substituted with at least one $R^C$, the at least one $R^C$ and N on the piperidinyl are independently in an ortho or meta position relative to each other;

optionally, when said $L^2$ is

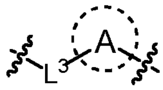, 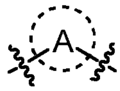

is piperidinyl unsubstituted or optionally substituted with at least one $R^c$, the at least one $R^c$ is selected from -H, -F, -Cl, methyl, or ethyl;

optionally, when said $L^2$ is

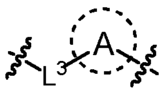

and $L^3$ is a single bond,

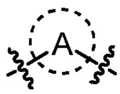

is selected from seven- to eight-membered N-spirocyclic groups unsubstituted or optionally substituted with at least one $R^C$;

optionally, when said

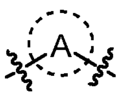

is selected from the seven- to eight-membered N-spirocyclic groups unsubstituted or optionally substituted with at least one $R^C$, the N-spirocylic group is selected from

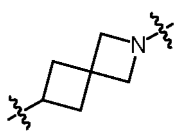

or

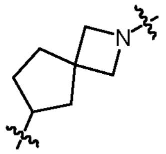

; and

optionally, $M^1$, $M^2$, and $M^3$ are each selected from -N= or -N($R^4$)-, $M^4$ and $M^5$ are both -C($R^5$)=, $R^4$ is hydrogen, and $R^5$ is hydrogen.

According to exemplary embodiments of the present disclosure, the compound represented by Formula (I) is a compound represented by the following Formula (I-0):

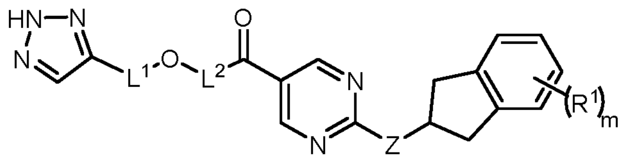

(I-0)

in which $R^1$ is -F or methyl;
optionally, m is 0, 1, or 2;
optionally, Z is -NH-;
optionally, $L^1$ is selected from

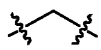 or 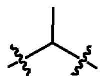;

optionally, $L^2$ is selected from

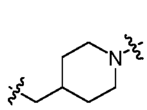, 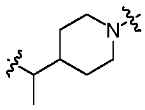, 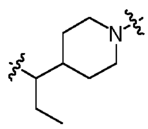, 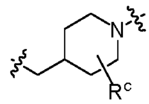,

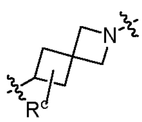, or 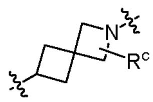,

where $R^C$ is selected from -H, -F, -Cl, methyl, or ethyl.

In another aspect, the present disclosure provides a compound represented by Formula (1-1), or a tautomer, stereoisomer, hydrate, solvate, pharmaceutical acceptable salt, or prodrug of the compound represented by Formula (I-1):

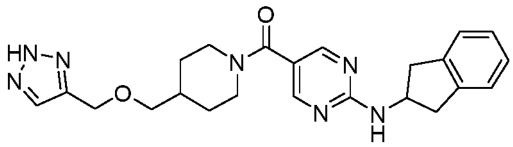

I-1

.

In yet another aspect, the present disclosure provides a compound represented by Formula (1-9), or a tautomer, stereoisomer, hydrate, solvate, pharmaceutical acceptable salt, or prodrug of the compound represented by Formula (1-9):

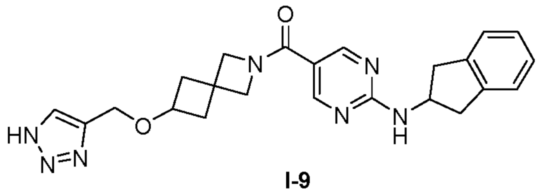

I-9

.

According to embodiments of the present disclosure, the compound represented by Formula (I) is any one of the following compounds:

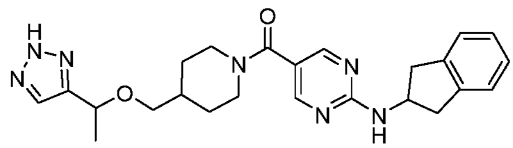

I-2

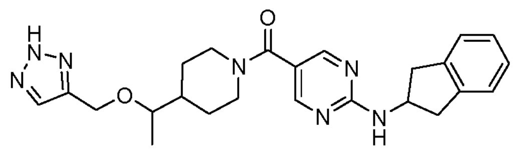

I-3

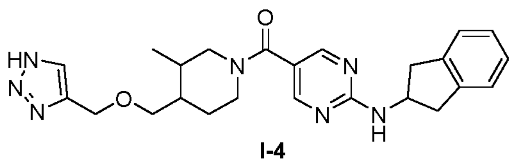

I-4

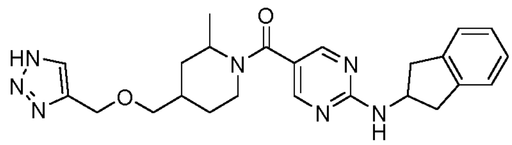

I-5

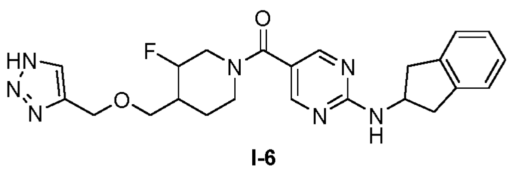

I-6

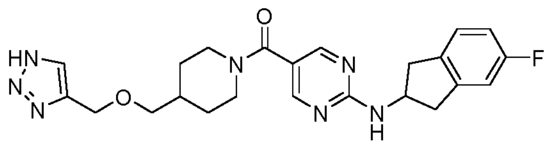

I-7

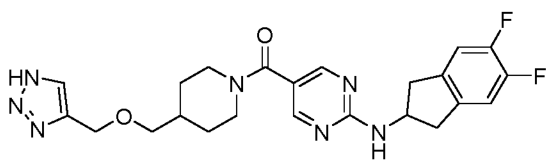

I-8

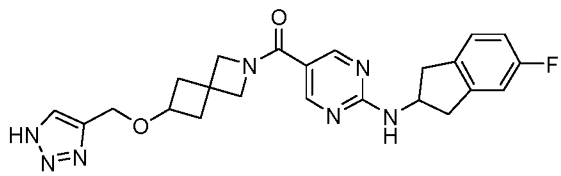

I-10

.

The compounds of the present disclosure may have tautomerism. The present disclosure includes all tautomeric forms of the compounds, either they are in an equilibrium state or one form is dominant, the present disclosure includes respective tautomeric forms.

In yet another aspect of the present disclosure, the present disclosure provides a pharmaceutical composition, which includes an effective dose of the compound represented by Formula (I).

In yet another aspect of the present disclosure, the present disclosure provides a use of the compound represented by Formula (I) or the tautomer, stereoisomer, hydrate, solvate, pharmaceutical acceptable salt, or prodrug thereof

in manufacture of a medicament for treating an ATX-related disease.

According to embodiments of the present disclosure, the ATX-related disease is selected from cancer, metabolic diseases, kidney diseases, liver diseases, fibrosis diseases, interstitial lung diseases, proliferative diseases, inflammatory diseases, pain, autoimmune diseases, respiratory diseases, cardiovascular diseases, neurodegenerative diseases, dermatological disorders, and/or diseases related to abnormal angiogenesis.

According to embodiments of the present disclosure, the ATX-related disease is selected from interstitial lung diseases, pulmonary fibrosis, liver fibrosis, or renal fibrosis, and preferably, the ATX-related disease is idiopathic pulmonary fibrosis. According to the embodiments of the present disclosure, the compounds of the present disclosure have significant advantages in the treatment of pulmonary fibrosis, especially idiopathic pulmonary fibrosis.

According to embodiments of the present disclosure, the ATX-related disease is a metabolic disease, and preferably, selected from type II diabetes or non-alcoholic steatohepatitis. According to the embodiments of the present disclosure, the compounds of the present disclosure have significant advantages in the treatment of the metabolic disease, especially, type II diabetes and non-alcoholic steatohepatitis.

According to embodiments of the present disclosure, the ATX-related disease is selected from neuropathic pain or inflammatory pain, and preferably, the ATX-related disease is osteoarthritis-related pain. According to the embodiments of the present disclosure, the compounds of the present disclosure have significant advantages in the treatment of osteoarthritis-related pain.

According to embodiments of the present disclosure, the ATX-related disease is cancer. According to the embodiments of the present disclosure, the compounds of the present disclosure have significant advantages in the treatment of cancer.

In yet another aspect of the present disclosure, the present disclosure provides a method for treating an ATX-related disease, and the method includes: administering, to a patient suffering from the ATX-related disease, a pharmaceutical composition containing an effective dose of the compound represented by Formula (I).

According to embodiments of the present disclosure, the ATX-related disease is selected from cancer, metabolic diseases, kidney diseases, liver diseases, fibrosis diseases, interstitial lung diseases, proliferative diseases, inflammatory diseases, pain, autoimmune diseases, respiratory diseases, cardiovascular diseases, neurodegenerative diseases, dermatological disorders, and/or diseases related to abnormal angiogenesis.

According to embodiments of the present disclosure, the ATX-related disease is selected from interstitial lung diseases, pulmonary fibrosis, liver fibrosis, or renal fibrosis, and preferably, the ATX-related disease is idiopathic pulmonary fibrosis.

According to embodiments of the present disclosure, the ATX-related disease is a metabolic disease, and preferably, selected from type II diabetes or non-alcoholic steatohepatitis.

According to embodiments of the present disclosure, the ATX-related disease is selected from neuropathic pain or inflammatory pain, and preferably, the ATX-related disease is osteoarthritis-related pain.

According to embodiments of the present disclosure, the ATX-related disease is cancer.

## Term Definitions and Explanations

Unless otherwise stated, the definitions of groups and terms described in the specification and claims include actual definitions, exemplary definitions, preferred definitions, definitions recorded in tables, and definitions of specific compounds in the examples, etc., which can be arbitrarily combined and integrated with each other. The group definitions and compound structures that are combined and integrated should fall within the scope of the present disclosure.

Unless otherwise defined, all scientific and technological terms of the present disclosure have the same meanings as those commonly understood by those skilled in the art to which the subjects of the claims belong. Unless otherwise specified, all granted patents, patent applications, and published documents cited in the present disclosure are incorporated into the present disclosure by reference in their entireties. If a term has multiple definitions in the present disclosure, the definitions in this section shall prevail.

Unless otherwise specified, conventional methods in the technical scope of the related art, such as mass spectrometry, NMR, IR and UV/Vis spectroscopy, and pharmacological methods are used. Unless specific definitions are provided, the terms used in the present disclosure relating to analytical chemistry, synthetic organic chemistry, and pharmaceuticals and medicinal chemistry are known in the art. Standard techniques can be used in chemical synthesis, chemical analysis, drug preparation, formulation and delivery, and treatment of patients. For example, reaction and purification can be carried out according to the manufacturer's instructions for use of the kit or in a manner known in the related art or the description of the present disclosure. Generally, the above-mentioned techniques and methods can be implemented according to the descriptions in a number of summary and more specific documents cited and discussed in the present disclosure according to conventional methods well-known in the related art. In the present specification, groups and their substituents can be selected by those skilled in the art to provide stable structural moieties and compounds. When a substituent is described by a conventional chemical formula written from left to right, the substituent

also includes the chemically equivalent substituent obtained when the structural formula is written from right to left. For example, $CH_2O$ is equivalent to $OCH_2$.

When the numerical range described in the specification and claims of the present disclosure is understood as "integers", it should be understood as recording the two endpoints of the range and all integers in the range. For example, an "integer from 1 to 6" should be understood as recording the integers 0, 1, 2, 3, 4, 5, and 6. An "integer from 0 to 9" should be understood as recording the integers 0, 1, 2, 3, 4, 5, 6, 7, 8, and 9. When the numerical range is understood as a "numerical number", it should be understood to record the two endpoints of the range, respective integers in the range and respective decimals in the range. For example, a "number from 1 to 10" should be understood as not only recording the respective integers 1, 2, 3, 4, 5, 6, 7, 8, 9 and 10, but also at least recording sums of each of the integers and 0.1, 0.2, 0.3, 0.4, 0.5, 0.6, 0.7, 0.8, and 0.9 respectively.

The term "pharmaceutically acceptable" refers to the compounds, materials, compositions and/or dosage forms that are suitable for use in contact with human and animal tissues without excess toxicity, irritation, allergic reactions or other problems or complications within the scope of reliable medical judgment, and are commensurate with a reasonable benefit/risk ratio.

The term "pharmaceutically acceptable salt" refers to a pharmaceutically acceptable salt of a non-toxic acid or base, including salts of inorganic acids and bases, as well as organic acids and bases. Salts derived from inorganic bases include, but are not limited to, metal salts formed by Al, Ca, Li, Mg, K, Na, and Zn. Salts derived from organic bases include, but are not limited to, salts of primary, secondary or tertiary amines, including organic salts formed by naturally occurring substituted or unsubstituted amines, cyclic amines, and basic ion exchange resins, for example, organic salts formed by ammonium, isopropylamine, trimethylamine, diethylamine, triethylamine, tripropylamine, diethanolamine, ethanolamine, dimethylethanolamine, 2-dimethylaminoethanol, 2-diethylaminoethanol, dicyclohexylamine, caffeine, procaine, choline, betaine, benethamine penicillin, ethylenediamine, glucosamine, methylglucamine, theobromine, triethanolamine, trometamol, purine, piperazine, piperidine, N-ethylpiperidine, or polyamine resin. Salts derived from inorganic and organic acids include, but are not limited to, organic salts formed by sulfuric acid, phosphoric acid, nitric acid, hydrobromic acid, hydrochloric acid, formic acid, acetic acid, propionic acid, benzenesulfonic acid, benzoic acid, phenylacetic acid, salicylic acid, alginic acid, anthranilic acid, camphoric acid, citric acid, vinylsulfonic acid, formic acid, fumaric acid, furoic acid, gluconic acid, glucuronic acid, glutamic acid, glycolic acid, isethionic acid, lactic acid, maleic acid, malic acid, mandelic acid, mucic acid, pamoic acid, pantothenic acid, stearic acid, succinic acid, sulphanilic acid, tartaric acid, p-toluenesulfonic acid, malonic acid, 2-hydroxypropionic acid, oxalic acid, glycolic acid, glucuronic acid, galacturonic acid, citric acid, lysine, arginine, aspartic acid, cinnamic acid, p-toluenesulfonic acid, methanesulfonic acid, ethanesulfonic acid, trifluoromethanesulfonic acid, etc.

In addition to the pharmaceutically acceptable salts, other salts may be adopted in the present disclosure, and they can serve as intermediates in the purification of compounds or in the preparation of other pharmaceutically acceptable salts or can be used for identifying, characterizing, or purifying the compounds of the present disclosure.

The term "stereoisomer" refers to an isomer produced by a different spatial arrangement of atoms in the molecule, including cis- and trans-isomers, enantiomers, diastereomers, and conformational isomers. The definitions and rules of stereochemistry used in the present disclosure generally follow "S.P.Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994".

Depending on the selected raw materials and methods, the compounds of the present disclosure may exist in the form of one of the possible isomers or their mixtures, for example, as pure optical isomers, or as a mixture of isomers such as racemic and diastereomeric mixtures, depending on the number of asymmetric carbon atoms. When describing optically active compounds, the prefixes *D* and *L* or *R* and *S* are used to denote the absolute configurations of the molecule with respect to one or more chiral centers. The prefixes D and L or (+) and (-) are symbols used to specify a rotation of plane-polarized light caused by a compound, where (-) or L indicates that the compound is levorotatory, and the prefix (+) or D indicates that the compound is dextrorotatory. For a given chemical structure, these stereoisomers are identical except that these stereoisomers are mirror images of each other. The specific stereoisomers can be referred to as enantiomers, and a mixture of such isomers is usually called an enantiomeric mixture. A mixture of enantiomers in 50:50 is called a racemic mixture or a racemate, which may occur when there is no stereoselectivity or stereospecificity in a chemical reaction or process. Many geometric isomers of olefins, C=N double bonds, etc. can also be included in the compounds described herein, and all such stable isomers are considered in the present disclosure. When the compounds described herein contain olefinic double bonds, unless otherwise specified, such double bonds include *E* and *Z* geometric isomers. If the compound contains a disubstituted cycloalkyl group, the substituent of the cycloalkyl group may has a *cis*- or *trans*-configuration.

When the bond with a chiral carbon in the formula of the present disclosure is depicted in a straight line, it should be understood that the two configurations (R) and (S) of the chiral carbon and both the resulting enantiomerically pure compound and mixture are included in the scope defined by the general formula. The diagrammatic presentation of the racemate or pure enantiomeric compound herein is from Maehr, J. Chem. Ed. 1985, 62: 114-120. Unless otherwise

specified, the wedge bond and the dashed bond are used to represent the absolute configuration of a stereocenter.

Optically active (R)- or (S)-isomers can be prepared using chiral synthons or chiral preparations, or resolved using conventional techniques. The compounds of the present disclosure containing asymmetrically substituted carbon atoms can be separated in an optically active form or in a racemic form. The resolution of a racemic mixture of a compound can be carried out with any of a variety of methods known in the art. For example, the methods include fractional recrystallization using chiral resolving acids, which are optically active salt-forming organic acids. For example, the suitable resolving agents for fractional recrystallization are optically active acids, such as tartaric acid, diacetyl tartaric acid, dibenzoyl tartaric acid, mandelic acid, malic acid, lactic acid or various optically active camphorsulfonic acids such as the *D* and *L* forms of β-camphorsulfonic acid. Other resolving agents suitable for fractional crystallization include α-methyl-benzylamine in a stereoisomerically pure form (for example, *S* and *R* forms or a diastereomerically pure form), 2-phenylglycinol, norephedrine, ephedrine, N-methylephedrine, cyclohexylethylamine, 1,2-diaminocyclohexane, etc. The resolution of the racemic mixture can also be carried out by eluting a column filled with an optically active resolving agent (for example, dinitrobenzoylphenylglycine). High performance liquid chromatography (HPLC) or supercritical fluid chromatography (SFC) can also be employed. The specific method, elution conditions, and the chromatographic columns can be selected by those skilled in the art according to the structures of the compounds and the experimental results. Further, optically pure starting materials or reagents with known configuration can also be used to obtain any enantiomers or diastereomers of the compounds described in the present disclosure through stereoorganic synthesis.

The term "tautomer" refers to an isomer of a functional group resulting from a rapid movement of an atom between two positions in a molecule. The compound of the present disclosure may exhibit tautomerism. Tautomeric compounds can be present in two or more mutually convertible species. The prototropy tautomer is resulted from a transfer of covalently bonded hydrogen atoms between two atoms. The tautomer generally exist in an equilibrium form. When trying to separate a single tautomer, a mixture is usually produced, the physical and chemical properties of which are consistent with the mixture of compounds. The position of equilibrium depends on the intramolecular chemical properties. For example, for many aliphatic aldehydes and ketones, such as acetaldehyde, the ketonic form is dominant; and for phenols, the enol form is dominant. All tautomeric forms of the compounds are included in the present disclosure.

In the examples of the present disclosure, protons may occupy two or more positions of the cyclic form of a heterocyclic ring system, for example, 1H- and 3H-imidazole, 1H-, 2H- and 4H-1,2,4-triazole, 1H- and 2H-isoindole, and 1H- and 2H-pyrazole. The tautomeric forms can be in equilibrium or sterically fixed in one form by appropriate substitution, for example:

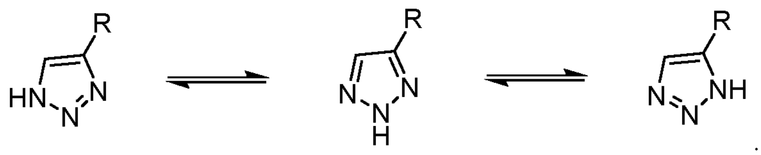

Due to resonance, a hydrogen atom on nitrogen of triazole may be located on any one of the three nitrogen atoms of the triazole, thus the names thereof are different, but these three forms actually represent one same compound.

The term "pharmaceutical composition" refers to a mixture of one or more of the compounds described herein or physiologically/pharmaceutically acceptable salts or prodrugs thereof and other chemical components. The other chemical components can be, for example, physiologically/pharmaceutically acceptable carriers and excipients. The pharmaceutical composition aims to facilitate the administration of the compound to an organism.

As for drugs or pharmacologically active agents, the term "effective dose", "effective amount" or "therapeutically effective amount" refers to a sufficient amount of a drug or agent that is non-toxic but can achieve the desired effect. For the oral dosage form of the present disclosure, the "effective amount" of one active substance in the composition refers to the amount required to achieve the desired effect when combined with another active substance in the composition. The effective amount may vary from person to person, depending on the age and general conditions of the subjects, as well as the specific active substance. The appropriate effective amount in a specific case can be determined by those skilled in the art according to routine experiments.

The term "active ingredient", "therapeutic agent", "active substance" or "active agent" refers to a chemical entity that can effectively treat the target disorder, disease or condition.

The term "solvate" refers to the compound of the present disclosure or a salt thereof including a stoichiometric or non-stoichiometric solvent bonded through an intermolecular non-covalent force. When the solvent is water, the solvate is a hydrate.

The term "prodrug" can be converted into the compound of the present disclosure having biological activity under physiological conditions or through solvolysis. The prodrug of the present disclosure is prepared by modifying the functional groups in the compound, and the modification can be removed by conventional operations or *in vivo,* so as to obtain the parent compound. The prodrug includes a compound, which is formed by connecting a moiety to a hydroxyl

group or amino group in the compound of the present disclosure. When the prodrug of the compound of the present disclosure is administered to a mammal individual, the prodrug is dissociated to form a free hydroxyl or amino group.

The compound of the present disclosure may contain an unnatural ratio of atomic isotopes on one or more of the atoms constituting the compound. For example, the compound may be labeled with a radioisotope, such as deuterium ($^2H$), tritium ($^3H$), iodine-125 ($^{125}I$) or C-14 ($^{14}C$). The transformations of all isotopic compositions of the compounds of the present disclosure, whether radioactive or not, are included within the scope of the present disclosure.

The term "excipient" refers to a pharmaceutically acceptable inert ingredient. Examples of the "excipient" include, but are not limited to, binders, disintegrants, lubricants, glidants, stabilizers, fillers, diluents, etc. Excipients can improve operation properties of the pharmaceutical formulation, i.e., allowing the formulation to be more suitable for direct compression by increasing fluidity and/or adhesion. Examples of typical "pharmaceutically acceptable carriers" suitable for the above formulations are: sugars such as lactose, sucrose, mannitol, and sorbitol; starches such as corn starch, tapioca starch and potato starch; cellulose and its derivatives, such as sodium carboxymethyl cellulose, ethyl cellulose, and methyl cellulose; calcium phosphates, such as dicalcium phosphate, and tricalcium phosphate; sodium sulfate; calcium sulfate; polyvinylpyrrolidone; polyvinyl alcohol; stearic acid; alkaline earth metal salts of stearic acid, such as magnesium stearate and calcium stearate; stearic acid; vegetable oils such as peanut oil, cottonseed oil, sesame oil, olive oil and corn oil; non-ionic, cationic, and anionic surfactants; ethylene glycol polymers; fatty alcohols; and grain hydrolyzed solids and other non-toxic compatible fillers, binders, disintegrants, buffers, preservatives, antioxidants, lubricants, coloring agents, and other excipients commonly used in drug formulations.

The term "$C_1$-$C_{10}$ alkyl" should be understood to indicate a straight-chain or branched-chain saturated monovalent hydrocarbon group having 1, 2, 3, 4, 5, 6, 7, 8, 9 or 10 carbon atoms. The alkyl group is, for example, methyl, ethyl, propyl, butyl, pentyl, hexyl, isopropyl, isobutyl, sec-butyl, tert-butyl, isoamyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl, etc., or isomers thereof. In particular, the alkyl group has 1, 2, 3, 4, 5, or 6 carbon atoms ("$C_1$-$C_6$ alkyl"), such as methyl, ethyl, propyl, butyl, isopropyl, isobutyl, sec-butyl, tert-butyl, and more particularly, the alkyl group has 1, 2 or 3 carbon atoms ("$C_1$-$C_3$ alkyl"), such as methyl, ethyl, n-propyl or isopropyl.

The term "$C_3$-$C_{10}$ cycloalkyl" should be understood to indicate a saturated monocyclic or bicyclic monovalent hydrocarbon ring having 3 to 10 carbon atoms, including a fused or bridged polycyclic system, for example, cyclopropyl, cyclobutyl, cyclopentyl, cyclohexyl, cycloheptyl, cyclooctyl, cyclonononyl or cyclodecyl, or bicyclic hydrocarbon groups such as a decahydronaphthalene ring.

The term "three- to ten-membered heterocyclic group" should be understood to indicate a saturated, unsaturated, or partially saturated monocyclic, bicyclic or tricyclic ring having 3 to 10 atoms, in which 1, 2, 3, 4 or 5 ring atoms are selected from N, O or S, and they can be connected by carbon or nitrogen, unless otherwise stated. The $-CH_2-$ group in the heterocyclic group is selectively replaced by -C(O)-. Unless otherwise, the nitrogen atom or sulfur atom in the ring is selectively oxidized to form N-oxide or S-oxide, or the nitrogen atom in the ring is selectively quaternized. -NH in the ring is selectively substituted by acetyl, formyl, methyl or methylsulfonyl. The ring is selectively substituted with one or more halogens. It should be understood that when the total number of S atoms and O atoms in the heterocyclic group exceeds 1, these heteroatoms are not adjacent to each other. If the heterocyclic group is bicyclic or tricyclic, at least one ring may selectively be a heteroaromatic ring or an aromatic ring, provided that at least one ring is non-heteroaromatic. If the heterocyclic group is monocyclic, it must not be aromatic. Examples of heterocyclic groups include, but are not limited to, piperidinyl, N-acetylpiperidinyl, N-methylpiperidinyl, N-formylpiperazinyl, N-methanesulfonylpiperazinyl, homopiperazinyl, piperazinyl, azetidinyl, oxetanyl, morpholinyl, tetrahydroisoquinolinyl, tetrahydroquinolinyl, indolinyl, tetrahydropyranyl, dihydro-2H-pyranyl, tetrahydrofuranyl, tetrahydrothiopyranyl, tetrahydrothiopyran-1-oxide, tetrahydrothiopyran-1,1-dioxide, 1H-pyridin-2-one, and 2,5 -dioxoimidazolidinyl.

The term "$C_1$-$C_{10}$ alkoxy" should be understood as -O-( $C_1$-$C_{10}$ alkyl), where "$C_1$-$C_{10}$ alkyl" has the definition described above.

The term "$C_6$-$C_{10}$ aryl" group should be understood as a monovalent aromatic or partially aromatic monocyclic, bicyclic or tricyclic hydrocarbon ring having 6 to 10 carbon atoms, especially a ring having 6 carbon atoms ("$C_6$ aryl"), such as phenyl; or biphenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl. When said $C_6$-$C_{10}$ aryl is substituted, it may be mono-substituted or multi-substituted. In addition, the substitution site is not limited, for example, ortho, para, or meta substitution may be adopted.

The term "$C_6$-$C_{10}$ aryloxy" should be understood as -O-( $C_6$-$C_{10}$ aryl), where $C_6$-$C_{10}$ aryl has the above definition.

The term "five- to ten-membered heteroaryl" should be understood as a monocyclic, bicyclic or tricyclic monovalent aromatic ring group having 5 to 10 ring atoms and containing 1 to 5 heteroatoms independently selected from N, O or S. For example, "five- to fourteen-membered heteroaryl" should be understood as a monocyclic, bicyclic or tricyclic monovalent aromatic ring group having 5, 6, 7, 8, 9, 10, 11, 12, 13 or 14 ring atoms, especially 5 or 6 or 9 or 10 carbon atoms, and containing 1 to 5, preferably 1 to 3 heteroatoms selected from N, O or S; and additionally, in each case, it

can be benzo-fused. In particular, the heteroaryl group is selected from the group consisting of thienyl, furyl, pyrrolyl, oxazolyl, thiazolyl, imidazolyl, pyrazolyl, isoxazolyl, isothiazolyl, oxadiazolyl, triazolyl, thiadiazolyl, etc. and benzo-fused derivatives thereof, such as benzofuranyl, benzothienyl, benzoxazolyl, benzisoxazolyl, benzimidazolyl, benzotriazolyl, indazolyl, indolyl, isoindolyl, etc.; or pyridinyl, pyridazinyl, pyrimidinyl, pyrazinyl, triazinyl, etc., and their benzo derivatives, such as quinolinyl, quinazolinyl, iso-quinolinyl, etc.; or azocinyl, indolizinyl, purinyl, etc. and their benzo derivatives; or cinolinyl, phthalazinyl, quinazolinyl, quinoxalinyl, naphthyridinyl, pteridyl, carbazolyl, acridinyl, phenazinyl, phenothiazinyl, phenoxazinyl, etc.

The term "spirocylic" refers to a polycyclic group that shares one carbon atom (called a spiro atom) between five- to twenty-membered monocyclic rings. The spirocylic group may include one or more double bonds, but none of the rings has a fully conjugated $\pi$ electron system. The rings are preferably six- to fourteen-membered, more preferably sevento eight-membered. Based on the number of shared spiro atoms between the rings, a spirocycloalkyl group can be classified into a monospirocycloalkyl group, a bispirocycloalkyl group or a polyspirocycloalkyl group, and the monospirocycloalkyl group and the bispirocycloalkyl group are preferable. More preferably, the spirocycloalkyl is a 4-membered/4-membered, 4-membered/5-membered, 4-membered/6-membered, 5-membered/5-membered, or 5-membered/6-membered monospirocycloalkyl. Examples of the spirocycloalkyl include, but are not limited to:

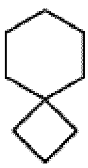 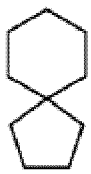 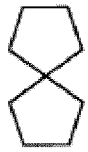 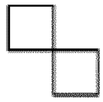 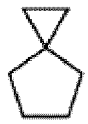

The spirocycloalkyl group in which a monospirocycloalkyl and a heterocycloalkyl share a spiro atom is also included, and the examples thereof include, but are not limited to:

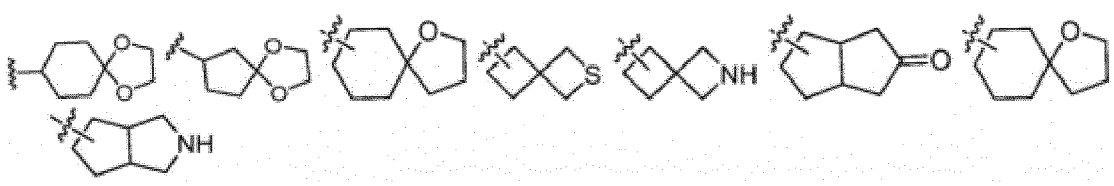.

The term "halogenated group" or "halogen" refers to fluorine, chlorine, bromine and iodine.

"Halogenated alkyl" refers to a branched and straight chain saturated aliphatic hydrocarbon group having a specified number of carbon atoms and substituted with one or more halogen atoms (such as -CvFw, where v=1 to 3, w=1 to (2v+1)). Examples of halogenated alkyl include, but are not limited to, trifluoromethyl, trichloromethyl, pentafluoroethyl, pentachloroethyl, 2,2,2-trifluoroethyl, heptafluoropropyl, and heptachloropropyl.

## Beneficial effects

According to the specific examples of the present disclosure, the compound represented by Formula (I) of the present disclosure or the pharmaceutically acceptable salt, tautomer, stereoisomer, hydrate, solvate or prodrug thereof has significant inhibitory effect on the ATX enzyme.

According to the specific examples of the present disclosure, the compound of the present disclosure has a good inhibitory effect on ATX enzyme, and the compound of the present disclosure exhibits excellent liver metabolism stability, is metabolized more slowly in the human body, and has higher exposure.

The additional aspects and advantages of the present disclosure will be partly given in the following description, and part of them will become apparent from the following description or can be understood through the implementation of the present disclosure.

## DESCRIPTION OF EMBODIMENTS

The solutions of the present disclosure will be explained below in conjunction with examples. Those skilled in the art will understand that the following examples are merely used to illustrate the present disclosure, and should not be construed as limiting the scope of the present disclosure. The examples without indication of specific techniques or conditions shall be implemented in accordance with the technology or conditions described in the literatures in the related art or in accordance with the product instructions. The reagents or instruments are all conventional products that are

commercially available where the manufacturers thereof are not specified.

The embodiments of the present disclosure provide compounds represented by Formula (I) or tautomers, stereoisomers, hydrates, solvates, pharmaceutically acceptable salts or prodrugs thereof; methods and intermediates for preparing the compounds represented by Formula (I) or the tautomers, stereoisomers, hydrates, solvates, pharmaceutically acceptable salts or prodrugs thereof; pharmaceutical compositions; and uses of the compounds and the pharmaceutical compositions of the present disclosure in manufacture of medicaments.

Reaction solvents used in reaction steps of the present disclosure are not particularly limited, and any solvent that can dissolve the starting materials to a certain extent and does not inhibit the reaction shall be included in the present disclosure. In addition, many similar modifications, equivalent substitutions, solvent combinations equivalent to the solvents described in the present disclosure, and different ratios of the solvent combinations are all deemed to be included in the scope of the present disclosure.

Structures of the compounds are identified by nuclear magnetic resonance (NMR) and/or mass spectrometry (MS). The unit of NMR shift is $10^{-6}$ (ppm). The NMR shift is described in unit of $10^{-6}$(ppm). The solvent for NMR measurement is deuterated dimethyl sulfoxide, deuterated chloroform, deuterated methanol, etc., and the internal standard is tetramethylsilane (TMS).

Liquid chromatography-mass spectrometry (LC-MS) measurement was conducted by using Waters Acquity H-class Uplc-QDA mass spectrometer, and monitored by using ACQUITY UPLC BEH C18, 2.1*50mm, 1.7 $\mu$m chromatographic column. Gradient elution conditions: at a flow rate of 1.0 mL/min, 95-5% solvent A1 and 5-95% solvent B1, then 95% B1 and 5% A1 for 0.5 min, in which the percentage was a percentage of a volume of a certain solvent in the total solvent volume. Among them, the solvent A1 was 0.1% formic acid in water solution, and the solvent B1 was 0.1% formic acid in acetonitrile solution. The percentage is the volume percentage of the solute in the solution.

The abbreviations in the present disclosure are defined as follows:

DIPEA: also referred to as DIEA, diisopropylethylamine, i.e., N,N-diisopropylethylamine
DMF: N,N-dimethylformamide
$Et_3N$: triethylamine
MeOH: methanol
N: equivalent concentration, for example, 2N hydrochloric acid means 2 mol/L hydrochloric acid solution
NADPH: reduced coenzyme II
NaH: sodium hydride
NMM: N-methylmorpholine
NMP: N-methylpyrrolidone
SFC: supercritical fluid chromatography
T3P: propylphosphonic anhydride, i.e., 2,4,6-tripropyl-1,3,5,2,4,6-trioxatriphosphine-2,4,6-trioxide or 1-propylphosphoric anhydride
THF: tetrahydrofuran
$TMSN_3$: Azidotrimethylsilane
$IC_{50}$: Half inhibitory concentration, which refers to a concentration at which half of the maximum inhibitory effect is reached

Unless otherwise indicated, the compounds exemplified herein are named and numbered with ChemBioDraw Ultra 13.0.

A control compound 1 used in the following tests of the present disclosure has a structure of:

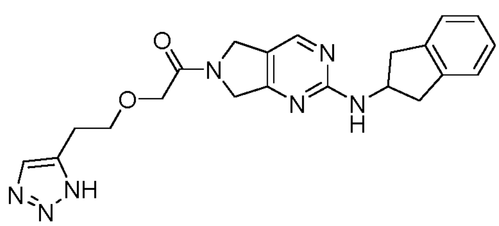

Control compound 1.

The control compound was synthesized with reference to patent application WO2014110000A1.

A control compound 2 used in the following tests of the present disclosure has a

structure of:

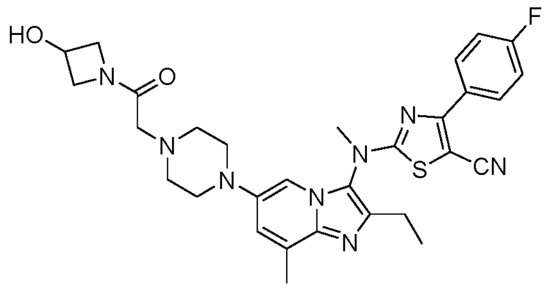

Control compound 2

The control compound 2 was synthesized with reference to patent application WO2014139882A1.
The control compounds 1 and 2 are known to have good inhibitory effect against ATX enzyme.

Preparation example 1: Preparation of intermediate A

2-((2,3-dihydro-1H-inden-2-yl)amino)pyrimidine-5-carboxylic acid (intermediate A)

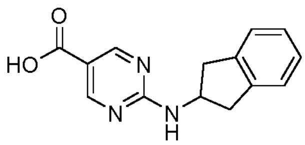

The synthetic scheme of the intermediate A is illustrated as below:

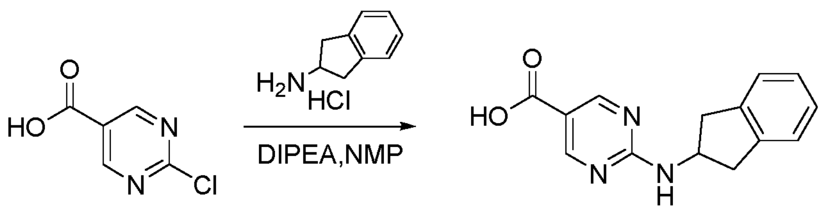


2-chloropyrimidine-5-carboxylic acid (2 g, 12.61 mmol) was dissolved in N-methylpyrrolidone (10 mL), and 2-aminoindan hydrochloride (2.57 g, 15.14 mmol) and N, N-diisopropylethylamine (8.15 g, 63.1 mmol) were added. The mixture was heated to 100°C and reacted for 24h. The reaction solution was evaporated under an oil pump to remove the solvent, the residue was added with ethyl acetate (30 mL) for dispersing and filtration, the filter cake was pulped with water (30 mL), filtered, and air-dried at 50°C for 3h to obtain a gray solid, i.e., 2-((2,3-dihydro-1H-inden-2-yl)amino)pyrimidine-5-carboxylic acid (2.4 g, yield 74.5%). The intermediate A used in the following examples can be obtained by referring to the above synthetic scheme and preparation method.
LC-MS m/z: 256.2[M+H]+

Preparation example 2: Preparation of intermediate B

4-(((1H-1,2,3-triazol-4-yl)methoxy)methyl)piperidine hydrochloride (intermediate B)

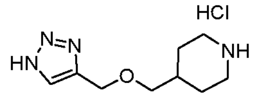


B

The synthetic scheme of the intermediate B is illustrated as below:

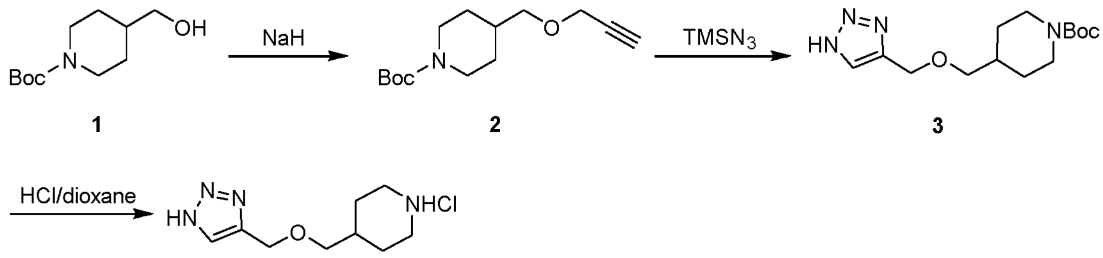


1 2 3

B

Step 1: Synthesis of tert-butyl 4-((prop-2-yn-1-yloxy)methyl)piperidine-1-carboxylate (2)

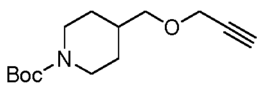

2

Tert-butyl 4-(hydroxymethyl)piperidine-1-carboxylate (150 g, 697 mmol) was added to THF (1000 mL) at 0° C. Then, sodium hydride (33.4 g, 836 mmol, purity 60%) was added batchwise, stirred at room temperature for 1h, and then cooled to 0°C. 3-bromopropyne (104 g, 871 mmol) was slowly added dropwise, and stirred at room temperature for 8h after the addition was complete. After the reaction was completed, the reaction solution was poured into saturated aqueous ammonium chloride solution THF (1000 mL), and extracted with ethyl acetate (300 mLx3), and the organic layers were combined to obtain a crude product. The crude product was separated and purified with a silica gel column (petroleum ether: ethyl acetate (V/V)=20:1) to obtain a colorless oily compound, i.e., tert-butyl 4-((prop-2-yn-1-yloxy)methyl)piperidine-1-carboxylate (135g, yield 76%).

Step 2: Synthesis of tert-butyl 4-(((1H-1,2,3-triazol-4-yl)methoxy)methyl)piperidine-1-carboxylate (3)

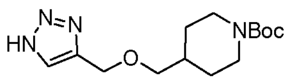

3

The substrate, tert-butyl 4-((prop-2-yn-1-yloxy)methyl)piperidine-1-carboxylate (60 g, 237 mmol), was dissolved in a mixed solvent DMF/MeOH (400 mL /100mL), and copper (I) iodide (2.26 g, 11.84 mmol) was added. After the mixture was cooled to 0°C, azidotrimethylsilane (40.9 g, 355 mmol) was slowly added dropwise, and after the addition was complete, the reaction mixture was slowly heated to 100°C and stirred for 18h. After the reaction was completed, the reaction mixture was cooled to room temperature and diluted with MeOH, and then the precipitate was filtered out. The filtrate was concentrated under reduced pressure to obtain a crude product. The crude product was separated and purified with a silica gel column (petroleum ether: ethyl acetate (V/V)=5:1) to obtain a yellow oily compound, i.e., tert-butyl 4-(((1H-1,2,3-triazol-4-yl)methoxy)methyl)piperidine-1-carboxylate (55 g, yield 78%).

LC-MS, M/Z: 297.1 $[M+H]^+$

Step 3: Synthesis of 4-(((1H-1,2,3-triazol-4-yl)methoxy)methyl)piperidine hydrochloride (**intermediate B**)

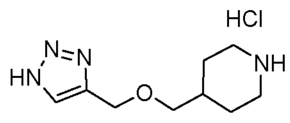

B

The substrate, tert-butyl 4-((prop-2-yn-1-yloxy)methyl)piperidine-1-carboxylate (55 g, 186 mmol), was dissolved in 1,4-dioxane (90 mL). Then, a 1,4-dioxane solution of hydrogen chloride (93 mL, 371 mmol, 4 M) was slowly dropwise added under stirring, and then stirred at room temperature for 2h. After the reaction was complete, the reaction solution was diluted with tert-butyl methyl ether (200 mL) and stirred well for 2h. The solid formed was quickly filtered with suction to obtain 4-(((1H-1,2,3-triazol-4-yl)methoxy)methyl)piperidine hydrochloride (42 g, yield 84%).

LC-MS, M/Z: 197.1 $[M+H]^+$

Example 1: Preparation of target compound I-1

(4-(((2H-1,2,3-triazol-4-yl)methoxy)methyl)piperidin-1-yl)(2-((2,3-dihydro-1H-inde n-2-yl)amino)pyrimidin-5-yl)methanone **(target compound I-1)**

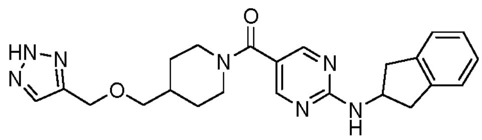

**I-1**

The synthetic scheme of the target compound **I-1** is illustrated as below:

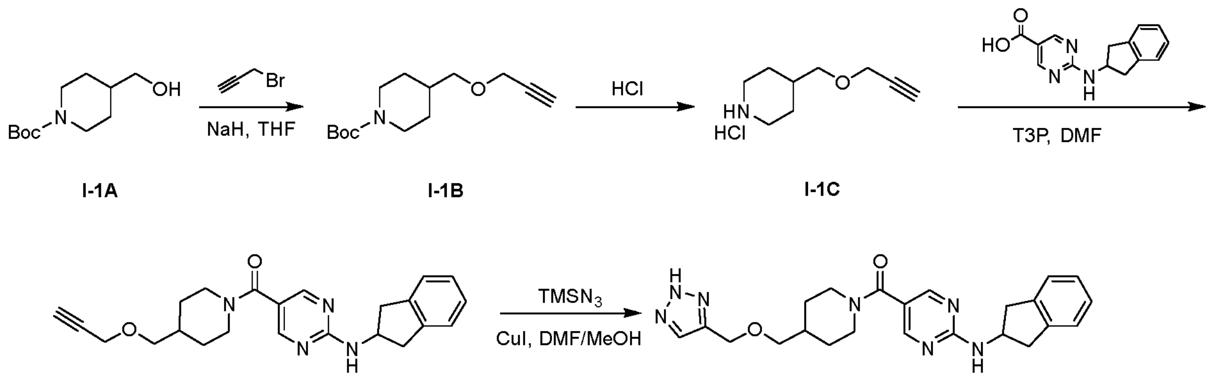


Step 1: Synthesis of tert-butyl 4-((prop-2-yn-1-yloxy)methyl)piperidine-1-carboxylate (**I-1B**)

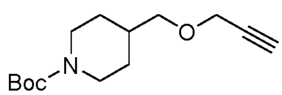

**I-1B**

Tert-butyl 4-(hydroxymethyl)piperidine-1-carboxylate (I-1A) (2 g, 9.29 mmol) was dissolved in tetrahydrofuran (20 mL), and cooled to 0° C. Then, sodium hydride (409 mg, 10.22 mmol, 60%) was added, followed by dropwise addition of 3-bromopropyne (1.66 g, 14 mmol), and after the addition was complete, the mixture reacted at room temperature for 18 hours. The reaction was quenched with water (50 mL), and extracted with ethyl acetate (50 mLx3), and the organic phases were combined and dried with anhydrous sodium sulfate, followed by filtering and concentrating. The residue was separated and purified by silica gel column (petroleum ether: ethyl acetate (V/V) =100: 1) to obtain the yellow liquid, i.e., tert-butyl 4-((prop-2-yn-1-yloxy)methyl)piperidine-1-carboxylate (**I-1B**) (1.6 g, yield 68%).

Step 2: Synthesis of 4-((prop-2-yn-1-yloxy)methyl)piperidine hydrochloride (**I-1C**)

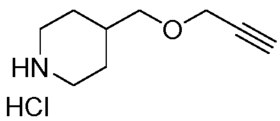

**I-1C**

A dioxane solution of hydrogen chloride (20 mL) was added into tert-butyl 4-((prop-2-yn-1-yloxy)methyl)piperidine-1-carboxylate (**I-1B**) (1.6 g, 6.32 mmol), and stirred at room temperature to react for 2h. The solvent was removed under reduced pressure to obtain a crude product, which was directly used in the next step of reaction without purification.

Step 3: Synthesis of 2-(2,3-dihydro-1H-inden-2-ylamino)pyrimidine-5-carboxylic acid

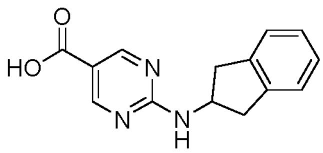

2-chloropyrimidine-5-carboxylic acid (2 g, 12.61 mmol) was dissolved in N-methylpyrrolidone (10 mL), and 2-aminoindan hydrochloride (2.57 g, 15.14 mmol) and N, N-diisopropylethylamine (8.15 g, 63.1 mmol) were added. The mixture was heated to 100°C and reacted for 24h. The reaction solution was evaporated under an oil pump to remove the solvent, the residue was added with ethyl acetate (30 mL) for dispersing and filtering, and the filter cake was pulped with water (30 mL), filtered, and air-dried at 50°C for 3h to obtain a gray solid, i.e., 2-(2,3-dihydro-1H-inden-2-ylamino)pyrimidine-5-carboxylic acid (2.4 g, yield 74.5%).

LC-MS, M/Z (ESI): 256.2 (M+H).

Step 4: Synthesis of (2-((2,3-dihydro-1H-inden-2-yl)amino)pyrimidin-5-yl)(4-((prop-2-yn-1-yloxy)methyl)piperid in-1-yl)methanone (**I-1D**)

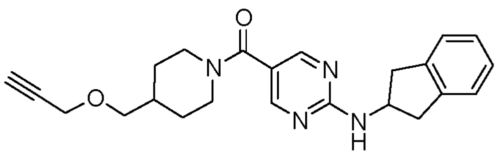

**I-1D**

N,N-dimethylformamide (10 mL), N, N-diisopropylethylamine (1.02 g, 7.91 mmol), and 2-(2,3-dihydro-1H-inden-2-ylamino) pyrimidine-5-carboxylic acid (424 mg, 1.66 mmol) were added into the crude product obtained from the previous step, i.e., 4-((prop-2-yn-1-yloxy)methyl)piperidine hydrochloride (**I-1C**) (300 mg, 1.58 mmol). The reaction solution was cooled to about 0°C, and 2,4,6-tripropyl-1,3,5,2,4,6-trioxatriphosphorinane-2,4,6-trioxide (1.31g, 2.06mmol,

50% N,N-dimethylformamide solution) was added dropwise. After the addition was complete, the mixture reacted at room temperature for 16h. The reaction solution was quenched by adding water (60 mL), and extracted with ethyl acetate (30 mLx3). The organic phases were combined, dried over anhydrous sodium sulfate, filtered, and concentrated. The residue was separated and purified with a silica gel plate (petroleum ether: ethyl acetate) (V/V)=5:1) to obtain a white solid, i.e., (2-((2,3-dihydro-1H-inden-2-yl)amino)pyrimidin-5-yl)(4-((prop-2-yn-1-yloxy)methyl)piperid in-1-yl)methanone (**I-1D**) (400 mg, yield 64.8%).

LC-MS, M/Z (ESI): 391.4 (M+1).

Step 5: Synthesis of (4-(((2H-1,2,3-triazol-4-yl)methoxy)methyl)piperidin-1-yl)(2-((2,3-dihydro-1H-inden-2-yl)a mino)pyrimidin-5-yl)methanone (**I-1**)

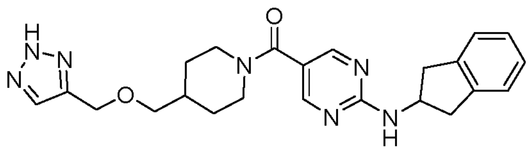

**I-1**

Under nitrogen protection, (2-((2,3-dihydro-1H-inden-2-yl)amino)pyrimidin-5-yl)(4-((prop-2-yn-1-yloxy)methyl)piperid in-1-yl)methanone (**I-1D**) (200 mg, 0.512 mmol) was dissolved in N,N-dimethylformamide (4 mL) and methanol (2 mL). Sodium L-ascorbate (203 mg, 1.024 mmol) was added, and then azidotrimethylsilane (590 mg, 5.12 mmol) and copper sulfate pentahydrate (32.7 mg, 0.205 mmol) were added. The mixture was heated to 90°C and reacted for 4 h. The reaction solution was cooled to room temperature and added with water (40 mL), and extracted with ethyl acetate (30 mLx3). The organic phases were combined, dried over anhydrous sodium sulfate, filtered, and concentrated. The residue was separated and purified with a silica gel plate (ethyl acetate: methanol (V/V)=10:1, aqueous ammonia) to obtain a white solid, i.e., (4-(((2H-1,2,3-triazol-4-yl)methoxy)methyl)piperidin-1-yl)(2-((2,3-dihydro-1H-inden-2-yl)a mino)pyrimidin-5-yl)methanone (**I-1**) (19 mg, yield 8.56%).

$^{1}H$ NMR (400 MHz, DMSO-d6): δ8.37 (s, 2H), 7.96 (d, 1H), 7.82 (s, 1H), 7.25-7.10 (m, 4H), 4.75-4.60 (m, 1H), 4.54 (s, 2H), 4.05 (m, 2H), 3.32-3.20 (m, 4H), 2.97-2.85 (m, 4H), 1.83 (m, 1H), 1.75-1.60 (m, 2H), 1.2-1.05 (m, 2H).

LC-MS, M/Z (ESI): 434.4 (M+1).

Example 2: Preparation of target compound 1-2

(4-((1-(2H-1,2,3-triazol-4-yl)ethoxy)methyl)piperidin-1-yl)(2-((2,3-dihydro-1H-inde n-2-yl)amino)pyrimidin-5-yl)methanone (target compound **I-2**)

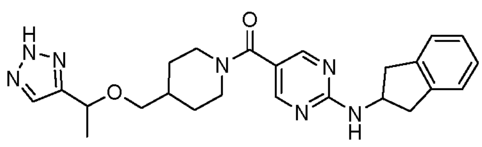

**I-2**

The synthetic scheme of the target compound **I-2** is illustrated as below:

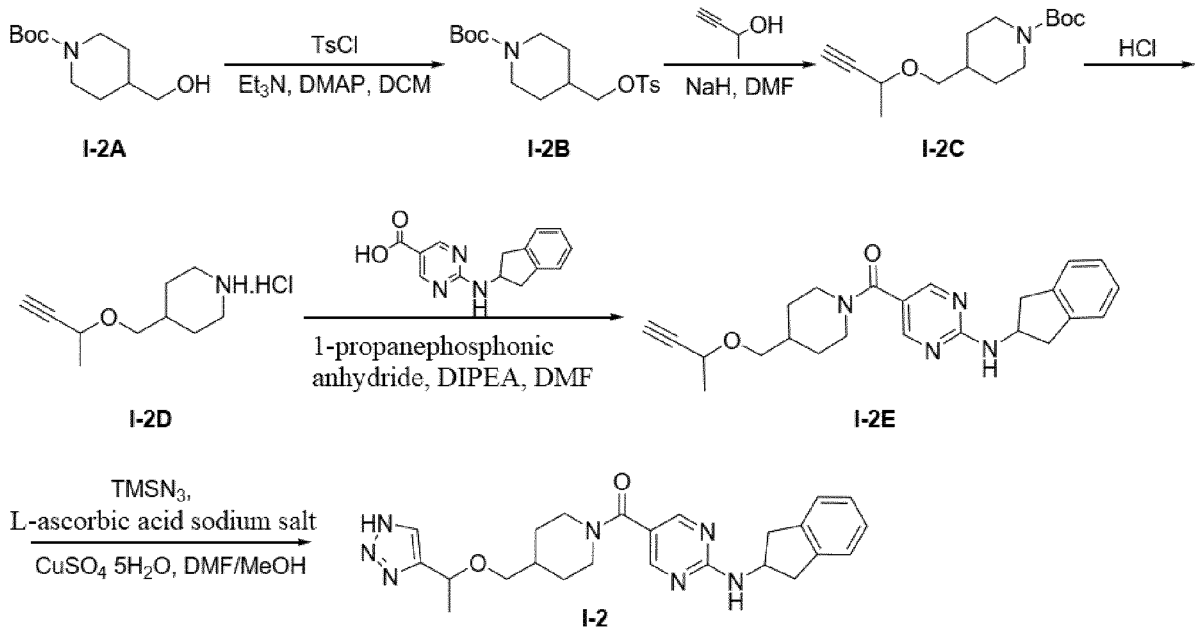


I-2A I-2B I-2C

I-2D I-2E

I-2

Step 1: Synthesis of tert-butyl 4-((tosyloxy)methyl)piperidine-1-carboxylate (**I-2B**)

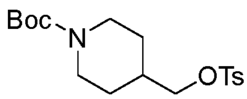

**I-2B**

Tert-butyl 4-(hydroxymethyl)piperidine-1-carboxylate (1.5 g, 6.97 mmol), triethylamine (1.41 g, 13.93 mmol), 4-dimethylaminopyridine (0.426 g, 3.48 mmol), and dichloromethane (20 mL) were added into a single-neck flask and dissolved, and p-toluenesulfonyl chloride (1.73 g, 9.06 mmol) was added at 0°C. The mixture was stirred and reacted at room temperature for 12 hours, TLC (petroleum ether: ethyl acetate (V/V) =5:1) indicated that the reaction was complete, concentration was conducted, and the residue was purified by silica gel column (petroleum ether: ethyl acetate (V/V) = 5:1) to obtain a colorless solid, tert-butyl 4-((tosyloxy)methyl)piperidine-1-carboxylate (1 g, 2.71 mmol, yield 38.8%).

Step 2: Synthesis of tert-butyl 4-((but-3-yn-2-yloxy)methyl)piperidine-1-carboxylate (I-2C)

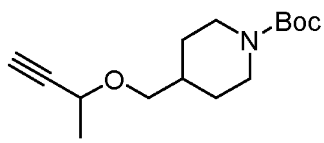

**I-2C**

But-3-yn-2-ol (0.285 g, 4.06 mmol) was added in a single-neck flask and dissolved with N,N-dimethylformamide (10 mL). 60% sodium hydride (0.162 g, 4.06 mmol) was added. The mixture was stirred at room temperature for 0.5 hours, and tert-butyl 4-((tosyloxy)methyl)piperidine-1-carboxylate (1 g, 2.71 mmol) was added, and stirred for reaction at 80°C for 12 hours. TLC (petroleum ether: ethyl acetate (V/V) =3:1) indicated that the reaction was complete, methanol (10 mL) was added to quench the reaction and concentration was conducted. The residue was purified using a silica gel column (petroleum ether: ethyl acetate (V/V) = 10:1) to obtain colorless oily product, tert-butyl 4-((but-3-yn-2-yloxy)methyl)piperidine-1-carboxylate (0.25 g, 0.935 mmol, yield 34.5%).

Step 3: Synthesis of 4-((but-3-yn-2-yloxy)methyl)piperidine hydrochloride (I-2D)

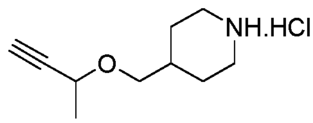

I-2D

Tert-butyl 4-((but-3-yn-2-yloxy)methyl)piperidine-1-carboxylate (0.25 g, 0.935 mmol) and hydrogen chloride/l,4-dioxane (2.5 M, 2 mL) were added into a single-neck flask, stirred at room temperature for 3 hours, and concentrated to dryness to obtain a colorless solid crude product, i.e., 4-((but-3-yn-2-yloxy)methyl)piperidine hydrochloride ( 0.204g).

Step 4: Synthesis of (4-((but-3-yn-2-yloxy)methyl)piperidin-1-yl)(2-((2,3-dihydro-1H-inden-2-yl)amino)pyrimidin -5-yl)methanone (I-2E)

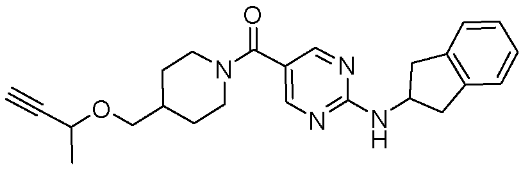

I-2E

4-((but-3-yn-2-yloxy)methyl)piperidine hydrochloride (204mg, l.Ommol), 2-((2,3-dihydro-1H-inden-2-yl)amino)pyrimidine-5-carboxylic acid (142 mg, 0.555 mmol), diisopropylethylamine (0.717 g, 5.55 mmol), and N,N-dimethylformamide (4 mL) were added into a single-neck flask, dissolved at room temperature, and then cooled to 0°C. 50% 1-propanephosphonic anhydride/N,N-dimethylformamide solution (0.529 g, 0.832 mmol) was added dropwise, and reacted at room temperature overnight after the dropwise addition was completed. TLC (methanol: dichloromethane (V/V) = 1:10) indicated that the reaction was completed, water (10 mL) was added for dilution, and the aqueous phase was extracted with dichloromethane (50 mLx2). The organic phases were combined and washed with saturated salt solution (50 mLx2) for liquid separation. The organic phase was dried over anhydrous sodium sulfate, filtered, and concentrated, and the residue was purified by a silica gel column (methanol: dichloromethane (V/V) = 1:10) to obtain a white solid, (4-((but-3-yn-2-yloxy)methyl)piperidin-1-yl)(2-((2,3-dihydro-1H-inden-2-yl)amino)pyrimidin -5-yl)methanone (200 mg, 0.494 mmol, yield 89%).

LC-MS m/z: 405.51[M+H]$^+$

Step 5: Synthesis of (4-((1-(2H-1,2,3-triazol-4-yl)ethoxy)methyl)piperidin-1-yl)(2-((2,3-dihydro-1H-inden-2-yl)a mino)pyrimidin-5-yl)methanone (target compound 1-2)

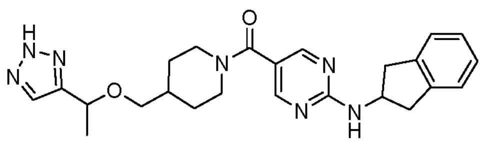

I-2

L(+)ascorbic acid sodium salt (196 mg, 1.0 mmol), (4-((but-3-yn-2-yloxy)methyl)piperidin-1-yl)(2-((2,3-dihydro-1H-inden-2-yl)amino)pyrimidin -5-yl)methanone (200 mg, 0.494 mmol), azidotrimethylsilane (0.171 g, 1.48 mmol), copper sulfate pentahydrate (25 mg, 0.099 mmol), N, N-dimethylformamide (4 mL), and methanol (0.4 mL) were added into a single-neck flask and heated to 90°C for reaction 2 hours. LCMS indicated that most of the raw materials were reacted. The reaction solution was cooled to room temperature, added with saturated salt solution (10 mL), and extracted with

ethyl acetate (50 mLx2). The organic phases were washed with saturated salt solution twice (20 mL×2), dried over anhydrous sodium sulfate, filtered, and concentrated, and the residue was separated by preparative chromatography to obtain a white solid, (4-((1-(2H-1,2,3-triazol-4-yl)ethoxy)methyl)piperidin-1-yl)(2-((2,3-dihydro-1H-inden-2-yl)a mino)pyrimidin-5-yl)methanone (target compound **I-2**, 30 mg, 0.067 mmol, yield 13.6%).

$^1$H NMR (400 MHz,DMSO-d6) δ8.33 (s, 2H), 7.92-7.93 (d, 1H), 7.74 (s, 1H), 7.16-7.19 (m, 2H), 7.09-7.13 (m, 2H), 4.56-4.64 (m, 2H), 3.09-3.25 (m, 5H), 2.85-2.90(q, 4H), 1.63-1.74 (m, 3H), 1.38-1.40 (d, 3H),1.03-1.12 (m, 3H).

LC-MS m/z: 448.54[M+H]$^+$

Example 3: Preparation of target compound 1-3

(4-(1-((1H-1,2,3-triazol-4-yl)methoxy)ethyl)piperidin-1-yl)(2-((2,3-dihydro-1H-inde n-2-yl)amino)pyrimidin-5-yl)methanone (target compound **I-3**)

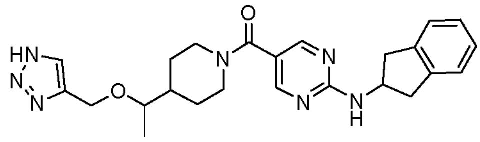

**I-3**

, i.e.,

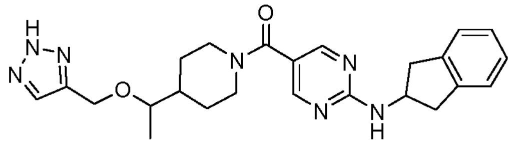

**I-3**

The synthetic scheme of the target compound **I-3** is illustrated as below:

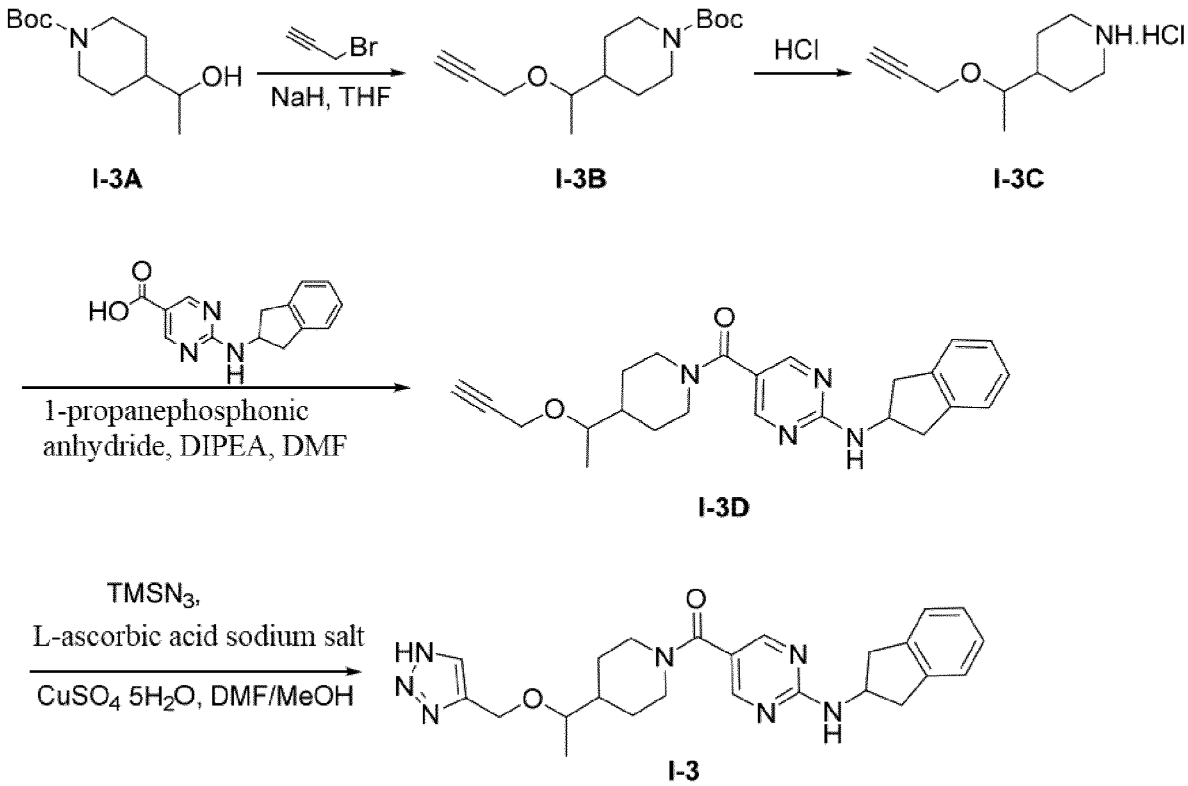


**I-3A** **I-3B** **I-3C** **I-3D** **I-3**

Step 1: Synthesis of tert-butyl 4-(1-(prop-2-yn-1-yloxy)ethyl)piperidine-1-carboxylate (I-3B)

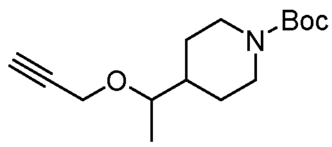

**I-3B**

Tert-butyl 4-(1-hydroxyethyl)piperidine-1-carboxylate (2.18 g, 5.34 mmol) was added in a single-neck flask and dissolved with tetrahydrofuran (10 mL), and 60% sodium hydride (0.131 g, 3.27 mmol) was added at room temperature. The mixture was stirred at room temperature for 0.5 hour, bromopropyne (0.778 g, 6.54 mmol) was added, and stirred at room temperature for 12 hours. TLC (petroleum ether: ethyl acetate (V/V) = 3:1) indicated that the reaction was complete. The reaction was quenched by adding methanol (10 mL), followed by concentration. The residue was purified through a silica gel column (petroleum ether: ethyl acetate (V/V) = 3:1) to obtain a colorless oily product, tert-butyl 4-(1-(prop-2-yn-1-yloxy)ethyl)piperidine-1-carboxylate (300 mg, 1.12 mmol, yield 51.5%).

Step 2: Synthesis of 4-(1-(prop-2-yn-1-yloxy)ethyl)piperidine hydrochloride (I-3C)

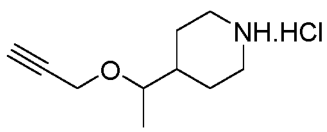

**I-3C**

Tert-butyl 4-(1-(prop-2-yn-1-yloxy)ethyl)piperidine-1-carboxylate (300 mg, 1.12 mmol) and hydrogen chloride/1,4-dioxane solution (2.5M, 2 mL) were added in a single-neck flask, stirred at room temperature for 3 hours, and concentrated to dryness to obtain a white solid crude product, i.e., 4-(1-(prop-2-yn-1-yloxy)ethyl)piperidine hydrochloride (0.22 g)

Step 3: Synthesis of (2-((2,3-dihydro-1H-inden-2-yl)amino)pyrimidin-5-yl)(4-(1-(prop-2-yn-1-yloxy)ethyl)piperid in-1-yl)methanone (I-3D)

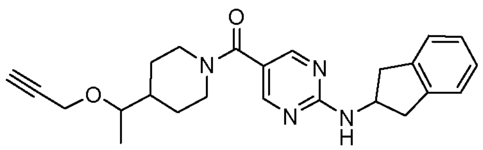

**I-3D**

The crude product, i.e., 4-(1-(prop-2-yn-1-yloxy)ethyl)piperidine hydrochloride (0.22 g, 1.08 mmol), 2-((2,3-dihydro-1H-inden-2-yl)amino)pyrimidine-5-carboxylic acid (153 mg, 0.56 mmol), diisopropylethylamine (0.774g, 5.99 mmol), and N,N-dimethylformamide (4 mL) were added into a single-neck flask, dissolved at room temperature, and then cooled to 0°C. 50% 1-propanephosphonic anhydride/N,N-dimethylformamide solution (0.572 g, 0.90 mmol) was added dropwise, and reacted overnight at room temperature after the dropwise addition was complete. TLC (methanol: dichloromethane (V/V) = 1:10) indicated that the reaction was complete. The reaction solution was diluted with water (10 mL), and the aqueous phase was extracted with dichloromethane (50 mLx2). The organic phases were combined and washed with saturated salt water (50 mLx2) for liquid separation. The organic phase was dried over anhydrous sodium sulfate, filtered, and concentrated, and the residue was purified by a silica gel column (methanol: dichloromethane (V/V) = 1:10) to obtain a white solid, i.e., (2-((2,3-dihydro-1H-inden-2-yl)amino)pyrimidin-5-yl)(4-(1-(prop-2-yn-1-yloxy)ethyl)piperid in-1-yl)methanone (150 mg, 0.37 mmol, yield 61.9%).

LC-MS m/z: 405.51$[M+H]^+$

Step 4: Synthesis of (4-(1-((1H-1,2,3-triazol-4-yl)methoxy)ethyl)piperidin-1-yl)(2-((2,3-dihydro-1H-inden-2-yl)a mino)pyrimidin-5-yl)methanone (**target compound I-3**)

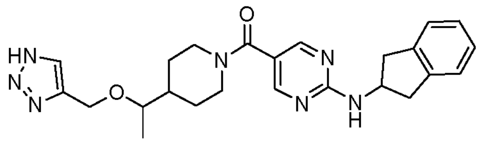

**I-3**

L(+)ascorbic acid sodium salt (147 mg, 0.74 mmol), (2-((2,3-dihydro-1H-inden-2-yl)amino)pyrimidin-5-yl)(4-(1-(prop-2-yn-1-yloxy)ethyl)piperid in-1-yl)methanone (150 mg, 0.37 mmol), azidotrimethylsilane (0.13 g, 1.12 mmol), copper sulfate pentahydrate (19 mg, 0.074 mmol), N, N-dimethylformamide (4 mL), and methanol (0.4 mL) were added into a single-neck flask and heated to 90°C for reaction 2 hours. LCMS indicated that most of the raw materials were reacted. The reaction solution was cooled to room temperature, added with saturated salt water (10 mL), and extracted with ethyl acetate (50 mLx2). The organic phases were washed with saturated salt water twice (20 mLx2), dried over anhydrous sodium sulfate, filtered, and concentrated, and the residue was separated by preparative chromatography to obtain an off-white solid, (4-(1-((1H-1,2,3-triazol-4-yl)methoxy)ethyl)piperidin-1-yl)(2-((2,3-dihydro-1H-inden-2-yl)a mino)pyrimidin-5-yl)methanone (57 mg, 0.127 mmol, yield 34.3%).

1H NMR (400 MHz, DMSO-d6) δ8.33 (s, 2H), 7.92-7.93 (d, 1H), 7.77 (s, 1H), 7.16-7.19 (m, 2H), 7.10-7.13 (m, 2H), 4.57-4.66(m,,2H), 4.42-4.46 (d, 2H), 3.31-3.30(q, 4H), 2.85-2.91(q, 3H), 1.53-1.75 (m, 4H),1.05-1.30 (m, 6H).

LC-MS m/z: 448.54$[M+H]^+$

Example 4: Preparation of target compound **I-4**

(4-(((1H-1,2,3-triazol-4-yl)methoxy)methyl)-3-methylpiperidin-1-yl)(2-((2,3-dihydro -1H-inden-2-yl)amino)pyrimidin-5-yl)methanone (target compound **I-4**)

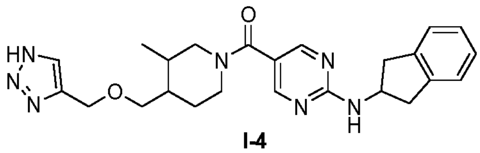

**I-4**

The synthetic scheme of the target compound **I-4** is illustrated as below:

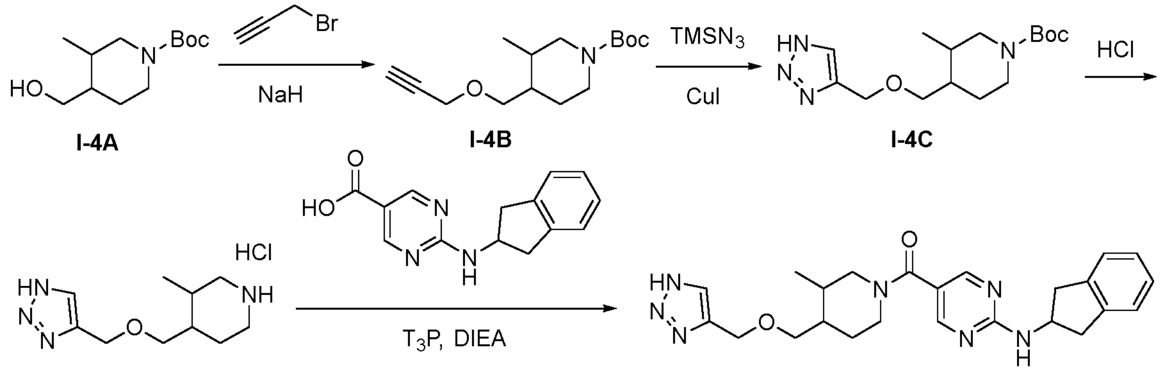


**I-4A** **I-4B** **I-4C** **I-4D** **I-4**

.

Step 1: Synthesis of tert-butyl 3-methyl-4-((prop-2-yn-1-yloxy)methyl)piperidine-1-carboxylate (I-4B)

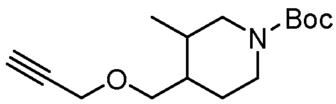

**I-4B**

Tert-butyl 4-(1-hydroxymethyl)-3-methylpiperidine-1-carboxylate **(I-4A)** (1.2 g, 5.23 mmol) was dissolved with dry tetrahydrofuran (15 mL) and cooled to 0 to 5°C. Sodium hydride (60% purity, 0.209 g, 5.23 mmol) was added, and the reaction solution was recovered to room temperature and stirred for 30 min. 3-bromopropyne (0.934 g, 7.85 mmol) was added, and after the addition was complete, reaction was continued at room temperature for 20h. The reaction was quenched by adding saturated ammonium chloride solution (50 mL), and extracted with ethyl acetate (30 mLx3). The organic phases were dried over anhydrous sodium sulfate, filtered, and concentrated. The residue was purified with a silica gel column (petroleum ether: ethyl acetate (V/V)=50:1) to obtain a yellow oily substance, tert-butyl 3-methyl-4-((prop-2-yn-1-yloxy)methyl)piperidine-1-carboxylate **(I-4B)** (450 mg, yield 32.3%)

Step 2: Synthesis of tert-butyl-4-(((1H-1,2,3-triazol-4-yl)methoxy)methyl)-3-methylpiperidine-1-carboxylate **(I-4C)**

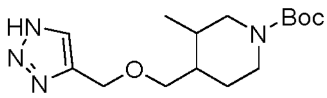

**I-4C**

Tert-butyl 3-methyl-4-((prop-2-yn-1-yloxy)methyl)piperidine-1-carboxylate (**I-4B**) (450 mg, 1.683 mmol) was dissolved in N,N-dimethylformamide (5 mL) and methanol (0.5 mL), and copper iodide (96 mg, 0.505 mmol) was added. The mixture was cooled to 0 to 5°C, azidotrimethylsilane (291 mg, 2.52 mmol) was added dropwise, and after the dropwise addition was complete, nitrogen displacement was performed three times, and then the mixture was heated to 95°C and reacted for 18h. The reaction solution was cooled to room temperature, and concentrated, and the residue was purified by a silica gel column (petroleum ether: ethyl acetate (V/V) = 1: 1) to obtain light yellow oily substance, tert-butyl-4-(((1H-1,2,3-triazol-4-yl)methoxy)methyl)-3-methylpiperidine-1-carboxylate

**(I-4C)** (95 mg, yield 18.2%).

Step 3: Synthesis of 4-(((1H-1,2,3-triazol-4-yl)methoxy)methyl)-3-methylpiperidine hydrochloride (**I-4D**)

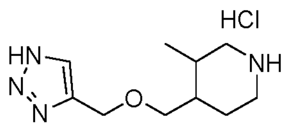

**I-4D**

4M hydrogen chloride in 1,4-dioxane solution (2 mL, 8 mmol) was added in tert-butyl-4-(((1H-1,2,3-triazol-4-yl)methoxy)methyl)-3-methylpiperidine-1-carboxylate **(I-4C)** (95 mg, 0.306 mmol), and reacted at room temperature for 2h. The reaction solution was concentrated, and the crude product was directly used for the next step of reaction.

Step 4: Synthesis of (4-(((1H-1,2,3-triazol-4-yl)methoxy)methyl)-3-methylpiperidin-1-yl)(2-((2,3-dihydro-1H-ind en-2-yl)amino)pyrimidin-5-yl)methanone (**I-4**)

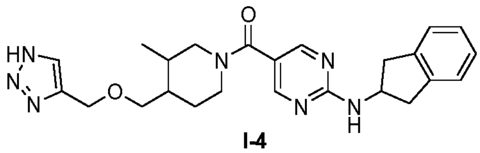

I-4

N,N-dimethylformamide (5 mL), 2-((2,3-dihydro-1H-indan-2-yl)amino)pyrimidine-5-carboxylic acid (79 mg, 0.308 mmol), and N,N-diisopropylethylamine (199 mg, 1.54 mmol) were added in the crude product obtained in the previous step, i.e., 4-(((1H-1,2,3-triazol-4-yl)methoxy)methyl)-3-methylpiperidine hydrochloride (**I-4D**). The reaction solution was cooled to about 0°C, and $T_3P$ (294 mg, 0.462 mmol, 50% N,N-dimethylformamide solution) was added dropwise. After the dropwise addition was completed, the mixture reacted at room temperature for 4h. The reaction was quenched by adding water (20 mL) into the reaction solution, the reaction solution was extracted with dichloromethane (15 mLx3), and the organic phases were dried over anhydrous sodium sulfate, filtered, and concentrated to dryness. The residue was separated and purified with a silica gel plate (ethyl acetate: methanol (V/V)=10 :1) to obtain white solid, (4-(((1H-1,2,3-triazol-4-yl)methoxy)methyl)-3-methylpiperidin-1-yl)(2-((2,3-dihydro-1H-ind en-2-yl)amino)pyrimidin-5-yl)methanone (**I-4**) (21 mg, yield 15.2%).

$^1$H NMR (400 MHz, DMSO-$d_6$): δ 8.33 (s, 2H), 7.94-7.92 (d, 1H), 7.80 (s, 1H), 7.21-7.18 (m, 2H), 7.14-7.11 (m, 2H), 4.65-4.48 (m, 3H), 3.28-3.20 (m, 2H), 2.99-2.86 (m, 3H), 2.43-2.37 (m, 1H), 2.01-1.89 (m, 2H), 1.43-1.32 (m, 3H), 121-1.20 (m, 1H), 0.94-0.93 (d, 3H), 0.71-0.70 (d, 2H).

LC-MS, M/Z: 448.3 $[M+H]^+$.

Example 5: Preparation of target compound **I-5**

(4-(((1H-1,2,3-triazol-4-yl)methoxy)methyl)-2-methylpiperidin-1-yl)(2-((2,3-dihydro -1H-inden-2-yl)amino)pyrimidin-5-yl)methanone (target compound **I-5**)

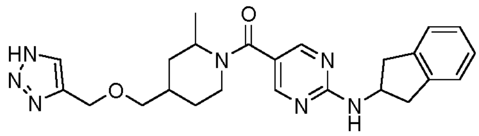

I-5

The synthetic scheme of the target compound **I-5** is illustrated as below:

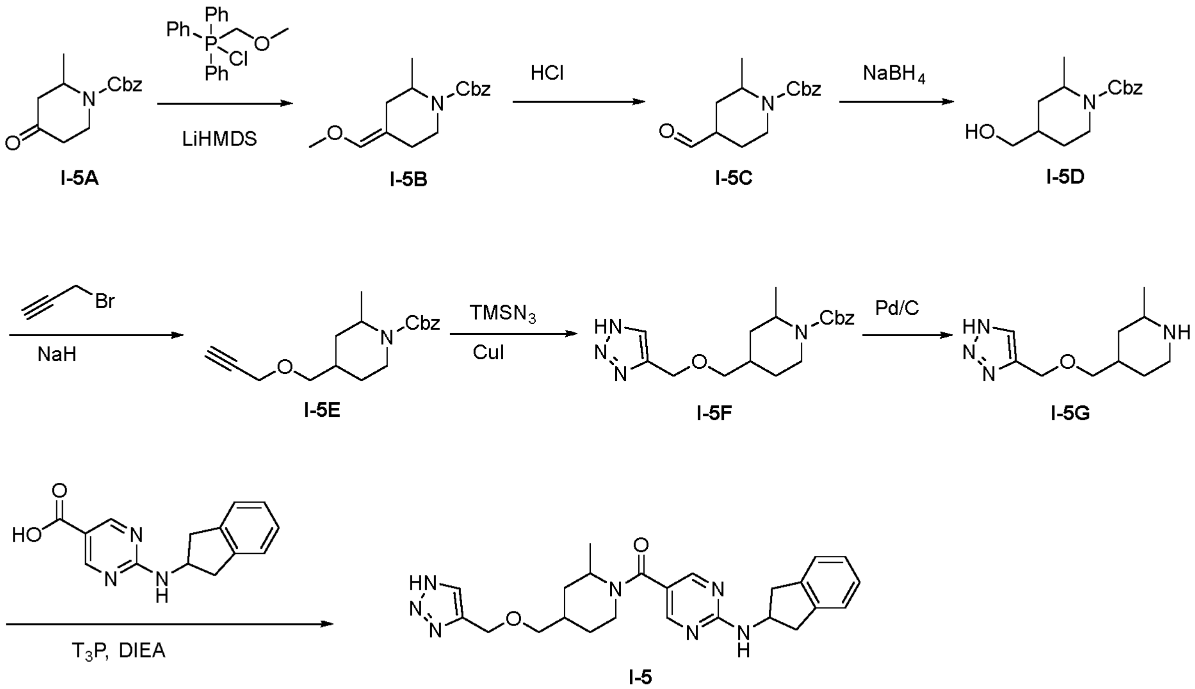


Step 1: Synthesis of benzyl (Z)-4-(methoxymethylene)-2-methylpiperidine-1-carboxylate (**I-5B**)

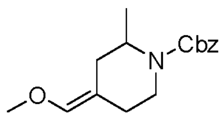

**I-5B**

Under nitrogen protection, chloro(methoxymethyl)triphenylphosphine (5.53 g, 16.13 mmol) was dissolved in dry tetrahydrofuran (15 mL), the mixture was cooled to 0°C, and lithium bis(trimethylsilyl)amide solution (1M, 16.13 mL, 16.13 mmol) was added dropwise, and thereafter, the temperature was maintained at 0°C to react for 30 min. Then, a tetrahydrofuran solution (10 mL) of benzyl 2-methyl-4-oxopiperidine-1-carboxylate (3 g, 12.13 mmol) was added dropwise, and after the addition, the reaction continued at 0 to 5°C for 1 h. The reaction was quenched by adding saturated ammonium chloride solution (50 mL), and the reaction solution was extracted with ethyl acetate (50 mLx3), the organic phases were combined and dried over anhydrous sodium sulfate, and concentrated. The residue was separated and purified by column chromatography (petroleum ether: ethyl acetate (V/V)=50:1) to obtain a light yellow oily substance, benzyl (Z)-4-(methoxymethylene)-2-methylpiperidine-1-carboxylate (**I-5B**) ( 3.37 g, yield 101%).

Step 2: Synthesis of benzyl 4-formyl-2-methylpiperidine-1-carboxylate (**I-5C**)

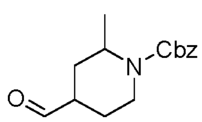

**I-5C**

Benzyl (Z)-4-(methoxymethylene)-2-methylpiperidine-1-carboxylate (**I-5B**) (3.37 g, 12.24 mmol) was dissolved in acetone (20 mL), concentrated hydrochloric acid (0.245 g, 2.448 mmol) was added, and the mixture was heated to

50°C and reacted for 20h. The reaction solution was cooled to room temperature, and saturated sodium bicarbonate solution was added to adjust pH to be greater than 8. The reaction solution was extracted with ethyl acetate (10 mLx3), and the organic phases were combined, dried over anhydrous sodium sulfate, and concentrated to obtain a yellow oily substance, benzyl 4-formyl-2-methylpiperidine-1-carboxylate (**I-5C)** (3.08 g, yield 96 %).

Step 3: Synthesis of benzyl 4-(hydroxymethyl)-2-methylpiperidine-1-carboxylate (I-5D)

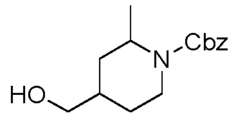

**I-5D**

Benzyl 4-formyl-2-methylpiperidine-1-carboxylate (**I-5C**) (3.08 g, 11.79 mmol) was dissolved in methanol (30 mL). Then, sodium borohydride (0.892 g, 23.57 mmol) was added batchwise, and after the addition was complete, the mixture was stirred at room temperature and reacted for 18h. Then, the reaction was quenched by adding water (50 mL). The reaction solution was extracted with ethyl acetate (30 mLx3), the organic phases were combined, dried over anhydrous sodium sulfate, concentrated, and separated and purified through column chromatography (petroleum ether: ethyl acetate (V/V)=3:1) to obtain a pale yellow oily substance, benzyl 4-(hydroxymethyl)-2-methylpiperidine-1-carboxylate (**I-5D**) (2.44 g, yield 79%).

Step 4: Synthesis of benzyl 2-methyl-4-((prop-2-yn-1-yloxy)methyl)piperidine-1-carboxylate (**I-5E)**

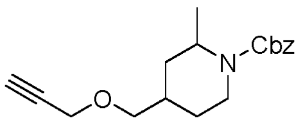

**I-5E**

Benzyl 4-(hydroxymethyl)-2-methylpiperidine-1-carboxylate(**I-5D**) (2.44 g, 9.27 mmol) was dissolved with dry tetrahydrofuran (25 mL) and cooled to about 0°C. Sodium hydride (60% purity, 0.371 g, 9.27 mmol) was added, and the reaction solution was recovered to room temperature to react for 30 min. 3-bromopropyne (1.653 g, 13.90 mmol) was added dropwise, and after the addition was complete, the mixture reacted at room temperature for 20h. The reaction was quenched by adding saturated ammonium chloride solution (50 mL), and extracted with ethyl acetate (50 mLx3). The organic phases were combined and dried over anhydrous sodium sulfate, and concentrated. The residue was separated and purified by column chromatography (petroleum ether: ethyl acetate (V/V)=10:1) to obtain a yellow liquid, benzyl 2-methyl-4-((prop-2-yn-1-yloxy)methyl)piperidine-1-carboxylate (**I-5E**) (2.48 g, yield 89%).

Step Synthesis of benzyl 4-(((1H-1,2,3-triazol-4-yl)methoxy)methyl)-2-methylpiperidine-1-carboxylate (**I-5F**)

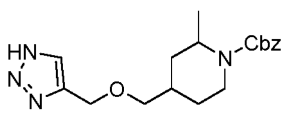

**I-5F**

Benzyl 2-methyl-4-((prop-2-yn-1-yloxy)methyl)piperidine-1-carboxylate (**I-5E**) (1.2 g, 3.98 mmol) was dissolved in N,N-dimethylformamide (10 mL) and methanol (1 mL), cuprous iodide (0.227 g, 1.195 mmol) was added, and the mixture was cooled to about 0°C. Azidotrimethylsilane (0.688 g, 5.97 mmol) was added dropwise, and thereafter, nitrogen replacement was performed for three times, the temperature was raised to 95°C to react for 20h. The reaction solution

was concentrated, and the residue was separated and purified by column chromatography (petroleum ether: ethyl acetate (V/V) = 1: 1) to obtain a brown oily substance, benzyl 4-(((1H-1,2,3-triazol-4-yl)methoxy)methyl)-2-methylpiperidine-1-carboxylate (**I-5F**) (0.45 g, yield 32.8%).

Step 6: Synthesis of 4-(((1H-1,2,3-triazol-4-yl)methoxy)methyl)-2-methylpiperidine (I-5G)

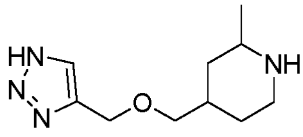

**I-5G**

Benzyl 4-(((1H-1,2,3-triazol-4-yl)methoxy)methyl)-2-methylpiperidine-1-carboxylate (**I-5F**) (450 mg, 1.307 mmol) was dissolved in methanol (10 mL), dry palladium on carbon (10%, 278 mg, 0.261 mmol) was added, and hydrogen replacement was performed for three times. The mixture reacted at room temperature under the protection of a hydrogen balloon for 48h, then filtered through diatomite, and concentrated to obtain a crude product directly for the next step of reaction.

Step 7: Synthesis of (4-(((1H-1,2,3-triazol-4-yl)methoxy)methyl)-2-methylpiperidin-1-yl)(2-((2,3-dihydro-1H-ind en-2-yl)amino)pyrimidin-5-yl)methanone (**I-5**)

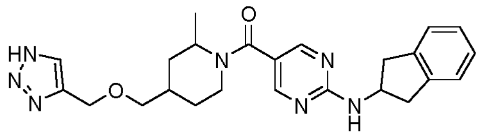

**I-5**

N,N-dimethylformamide (10 mL), 2-((2,3-dihydro-1H-indan-2-yl)amino)pyrimidine-5-carboxylic acid (334 mg, 1.308 mmol), and N,N-diisopropylethylamine (507 mg, 3.92 mmol) were added into the crude product obtained in the previous step, i.e., 4-(((1H-1,2,3-triazol-4-yl)methoxy)methyl)-2-methylpiperidine (**I-5G**). The reaction solution was cooled to about 0°C, and $T_3P$ (1.248 g, 1.962 mmol, 50% N,N-dimethylformamide solution) was added dropwise. After the addition was completed, the mixture reacted at room temperature for 4h. The reaction was quenched by adding water (50 mL) to the reaction solution, and the reaction solution was extracted with ethyl acetate (30 mLx3). The organic phases were dried over anhydrous sodium sulfate, filtered, and concentrated to dryness. The residue was separated and purified with a silica gel plate (ethyl acetate: methanol (V/V)=10: 1) to obtain a white solid, (4-(((1H-1,2,3-triazol-4-yl)methoxy)methyl)-2-methylpiperidin-1-yl)(2-((2,3-dihydro-1H-ind en-2-yl)amino)pyrimidin-5-yl)methanone (**I-5**) (48 mg, yield 8.2%).

$^1H$ NMR (400 MHz, DMSO-$d_6$) $\delta$ 8.33 (s, 2H), 7.93-7.91 (d, 1H), 7.80 (s, 1H), 7.21-7.17 (m, 2H), 7.14-7.10 (m, 2H), 4.67-4.58 (m, 1H), 4.51 (s, 2H), 3.29-3.20 (m, 7H), 2.92-2.86 (dd, 2H), 2.03 (s, 1H), 1.68-1.53 (dd, 2H), 1.38-1.04 (m, 5H).

LC-MS, M/Z: 448.3 $[M+H]^+$.

Example 6: Preparation of target compound 1-6

(4-(((1H-1,2,3-triazol-4-yl)methoxy)methyl)-3-fluoropiperidin-1-yl)(2-((2,3-dihydro-1H-inden-2-yl)amino)pyrimidin-5-yl)methanone (target compound **I-6**)

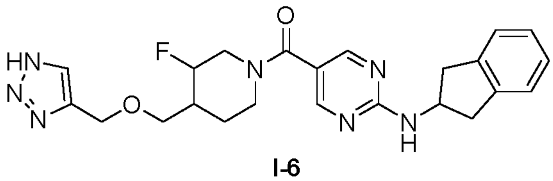

**I-6**

The synthetic scheme of the target compound **I-6** is illustrated as below:

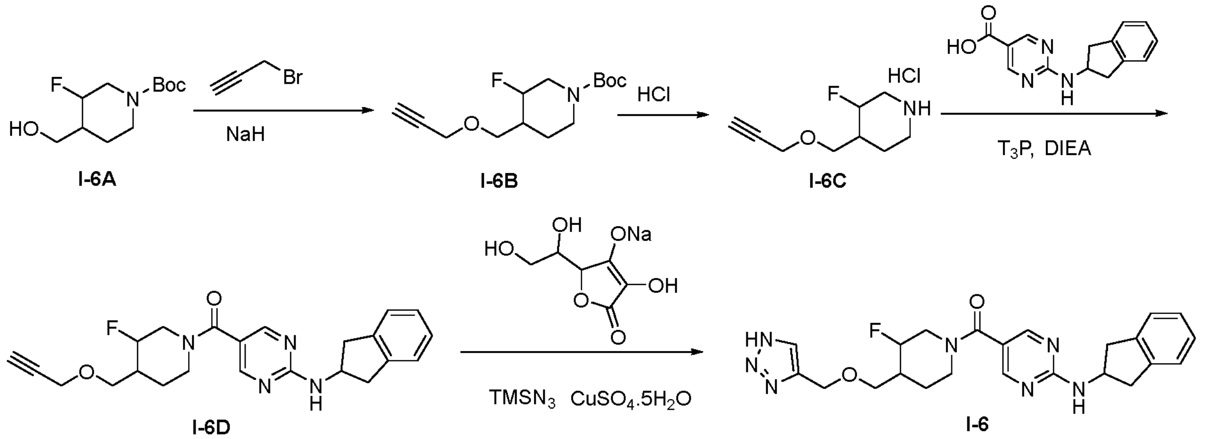


**I-6A** **I-6B** **I-6C** **I-6D** **I-6**

Step 1: Synthesis of tert-butyl 3-fluoro-4-((prop-2-yn-1-yloxy)methyl)piperidine-1-carboxylate (**I-6B**)

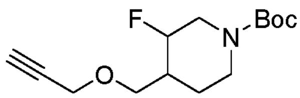

**I-6B**

Tert-butyl 3-fluoro-4-(hydroxymethyl)piperidine-1-carboxylate (**I-6B**) (500 mg, 2.143 mmol) was dissolved in dry tetrahydrofuran (5 mL) and cooled to about 0°C, sodium hydride (60% purity, 86 mg, 2.143 mmol) was added, and temperature of the mixture was recovered to room temperature to react for 30 min. 3-bromopropyne (382 mg, 3.22 mmol) was added dropwise, and thereafter, the reaction continued at room temperature for 18h. The reaction was quenched by adding saturated ammonium chloride solution (30 mL) to the reaction solution, and the reaction solution was extracted with ethyl acetate (20 mLx3). The organic phases were combined, dried over anhydrous sodium sulfate, and concentrated. The residue was separated and purified by column chromatography (petroleum ether: ethyl acetate (V/V)=20: 1) to obtain a yellow oily substance, tert-butyl 3-fluoro-4-((prop-2-yn-1-yloxy)methyl)piperidine-1-carboxylate (**I-6B**) (450 mg, yield 77%).

Step 2: Synthesis of 3-fluoro-4-((prop-2-yn-1-yloxy)methyl)piperidine hydrochloride (I-6C)

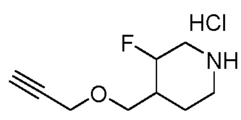

**I-6C**

4M hydrogen chloride in 1,4-dioxane solution (5 mL, 20 mmol) was added into tert-butyl 3-fluoro-4-((prop-2-yn-1-yloxy)methyl)piperidine-1-carboxylate (**I-6B**) (200 mg, 0.737 mmol), and reacted at room temperature for 1h. The reaction solution was concentrated, and the crude product was directly fed to the next step of reaction.

Step 3: Synthesis of (2-((2,3-dihydro-1H-inden-2-yl)amino)pyrimidin-5-yl)(3-fluoro-4-((prop-2-yn-1-yloxy)methy 1)piperidin-1-yl)methanone (**I-6D)**

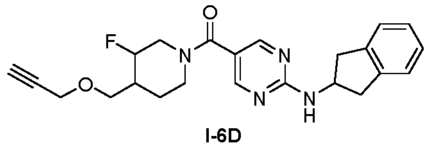

**I-6D**

N,N-dimethylformamide (5 mL), 2-((2,3-dihydro-1H-indan-2-yl)amino)pyrimidine-5-carboxylic acid (188 mg, 0.737 mmol), and N,N-diisopropylethylamine (476 mg, 3.68 mmol) were added into the crude product obtained in the previous step, i.e., 3-fluoro-4-((prop-2-yn-1-yloxy)methyl)piperidine hydrochloride (**I-6C)**. The reaction solution was cooled to about 0°C. $T_3P$ (703 mg, 1.105 mmol, 50% N,N-dimethylformamide solution) was added dropwise. After the dropwise addition was complete, the mixture reacted at room temperature for 4h. The reaction was quenched by adding water (50 mL) to the reaction solution, and the reaction solution was extracted with ethyl acetate (30 mLx3). The organic phases were dried with anhydrous sodium sulfate, filtered, and concentrated to dryness. The residue was separated and purified by column chromatography (petroleum ether: ethyl acetate (V/V) = 1:2) to obtain a colorless oily substance, (2-((2,3-dihydro-1H-inden-2-yl)amino)pyrimidin-5-yl)(3-fluoro-4-((prop-2-yn-1-yloxy)methy 1)piperidin-1-yl)methanone (**I-6D**) (250 mg, yield 83%).

LC-MS, M/Z: 409.3 $[M+H]^+$.

Step 4: Synthesis of (4-(((1H-1,2,3 -triazol-4-yl)methoxy)methyl)-3 -fluoropiperi din-1-yl) (2-((2,3-dihydro-1H-inde n-2-yl)amino)pyrimidin-5-yl)methanone (**I-6**)

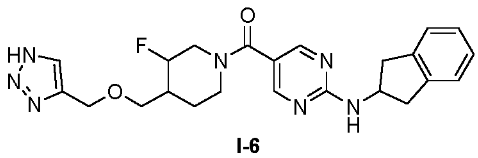

**I-6**

(2-((2,3-dihydro-1H-inden-2-yl)amino)pyrimidin-5-yl)(3-fluoro-4-((prop-2-yn-1-ylo xy)methyl)piperidin-1-yl)methanone (**I-6D**) (230 mg, 0.563 mmol) was dissolved in N,N-dimethylformamide (5 mL) and methanol (2 mL). Ascorbic acid sodium salt (223 mg, 1.126 mmol), copper sulfate pentahydrate (28.1 mg, 0.113 mmol), and azidotrimethylsilane (324 mg, 2.82 mmol) were added sequentially, nitrogen replacement was performed for three times, the reaction solution was heated to 95°C and reacted for 3h. The reaction solution was cooled to room temperature, added with water (50 mL), and extracted with dichloromethane (30 mLx3). The organic phases were combined, dried over anhydrous sodium sulfate, and concentrated. The residue was purified on a silica gel plate (ethyl acetate: methanol (V/V)=20:1) to obtain a white solid, (4-(((1H-1,2,3-triazol-4-yl)methoxy)methyl)-3-fluoropiperidin-1-yl)(2-((2,3-dihydro-1H-inde n-2-yl)amino)pyrimidin-5-yl)methanone (**I-6**) (140 mg, yield 55.1%).

$^1$H NMR (400 MHz, DMSO-$d_6$) δ 8.37 (s, 2H), 8.02-8.00 (d, 1H), 7.84 (s, 1H), 7.24-7.20 (m, 2H), 7.17-7.13 (m, 2H), 4.88 (s, 0.5H), 4.76 (s, 0.5H), 4.69-4.64 (m, 1H), 4.58 (s, 2H), 3.48-3.44(t, 4H), 3.29-3.23 (m, 4H), 2.95-2.89 (dd, 2H), 2.16-2.00 (m, 1H), 1.59-1.55 (m, 1H), 1.42-1.35 (m, 1H).

LC-MS, M/Z: 452.3 $[M+H]^+$.

Example 7: Preparation of target compound **I-7**

(4-(((1H-1,2,3-triazol-4-yl)methoxy)methyl)piperidin-1-yl)(2-((5-fluoro-2,3-dihydro-1H-inden-2-yl)amino)pyrimidin-5-yl)methanone (target compound 1-7)

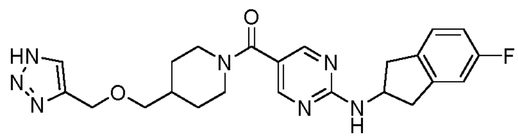

I-7

The synthetic scheme of the target compound **I-7** is illustrated as below:

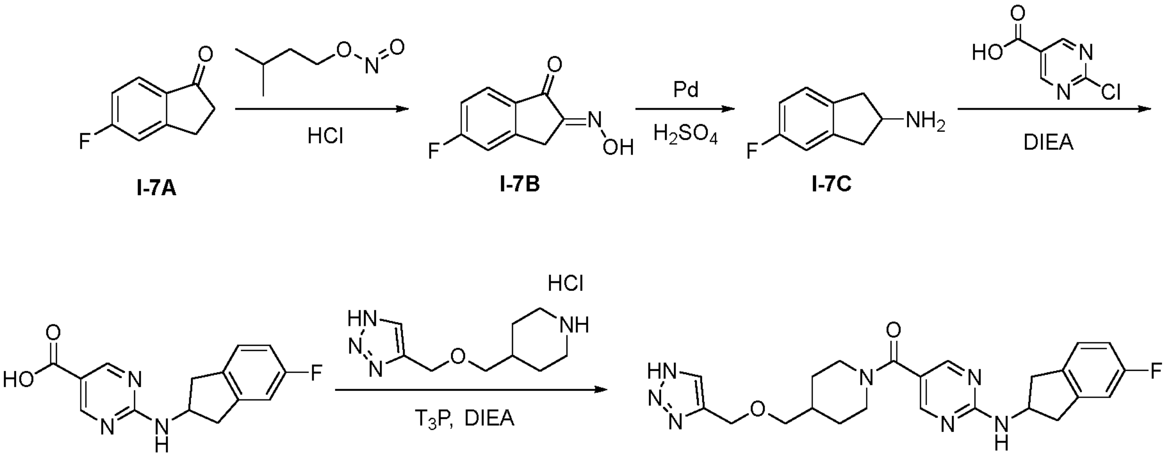


I-7A I-7B I-7C I-7D I-7

Step 1: Synthesis of (E)-5-fluoro-2-(hydroxyimino)-2,3-dihydro-1H-inden-1-one (I-7B)

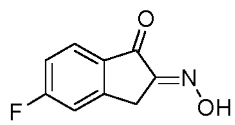

I-7B

5-fluoro-2,3-dihydro-1H-inden-1-one (**I-7A**) (5 g, 33.3 mmol) was dissolved in methanol (50 mL), the reaction solution was heated to 40°C, isopentyl nitrite (6.6 mL, 5.74 mmol) was added, and concentrated hydrochloric acid (5 g, 50.1 mmol) was added dropwise. Thereafter, the mixture reacted at 40 to 50°C for 30 min. A solid was precipitated, filtered, and dried to obtain a white solid (E)-5-fluoro-2-(hydroxyimino)-2,3-dihydro-1H-inden-1-one (**I-7B**) (4.2 g, yield 70.4%).

LC-MS, M/Z: 180.1 $[M+H]^+$.

Step 2: Synthesis of 5-fluoro-2,3-dihydro-1H-inden-2-amine (**I-7C**)

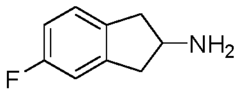

I-7C

(E)-5-fluoro-2-(hydroxyimino)-2,3-dihydro-1H-inden-1-one (**I-7B**) (3.1 g, 17.30 mL) was dissolved in acetic acid (90 mL), 10% dry palladium on carbon (5.52 g, 5.19 mmol) and concentrated sulfuric acid (6.06 g, 60.6 mmol) were added, and hydrogen replacement was performed for three times. Under the protection of a hydrogen balloon, the reaction solution was heated to 60°C and reacted for 20 h. The reaction solution was cooled to room temperature, and

filtered with diatomite, the filter cake was washed with water (20 mL), the filtrate was concentrated. 50% sodium hydroxide solution was added to the residue to adjust pH to be greater than 11, and the reaction solution was extracted with chloroform (30 mL*5). The organic phases were combined, dried over anhydrous sodium sulfate, and concentrated. The residue was separated and purified by column chromatography (dichloromethane: methanol (V/V)=50:1) to obtain a dark brown oily substance, 5-fluoro-2,3-dihydro-1H-inden-2-amine (**I-7C**) (1 g, yield 38.2%).

LC-MS, M/Z: 152.2 [M+H]$^+$.

Step 3: Synthesis of 2-((5-fluoro-2,3-dihydro-1H-inden-2-yl)amino)pyrimidine-5-carboxylic acid (**I-7D**)

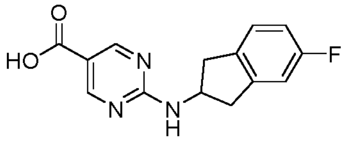

**I-7D**

5-fluoro-2,3-dihydro-1H-inden-2-amine (**I-7C**) (524 mg, 3.47 mmol) and 2-chloropyrimidine-5-carboxylic acid (500 mg, 3.15 mmol) were dissolved in N-methylpyrrolidone (5 mL), and N,N-diisopropylethylamine (2.038 g, 15.77 mmol) was added. The mixture was heated to 100°C and reacted for 20h. Then, the reaction solution was cooled, and concentrated by removing the solvent, and the residue was added with isopropyl acetate (5 mL), pulped, and filtered. The filter cake was washed with water (10 mL), and dried to obtain a gray solid, 2-((5-fluoro-2,3-dihydro-1H-inden-2-yl)amino)pyrimidine-5-carboxylic acid (**I-7D**) (650 mg, yield 75%).

LC-MS, M/Z: 274.2 [M+H]$^+$.

Step 4: Synthesis of (4-(((1H-1,2,3-triazol-4-yl)methoxy)methyl)piperidin-1-yl)(2-((5-fluoro-2,3-dihydro-1H-inde n-2-yl)amino)pyrimidin-5-yl)methanone (1-7)

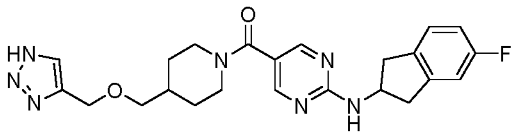

**I-7**

2-((5-fluoro-2,3-dihydro-1H-inden-2-yl)amino)pyrimidine-5-carboxylic acid (**I-7D**) (200 mg, 0.732 mmol) and 4-(((1H-1,2,3-triazol-4-yl)methoxy)methyl)piperidine hydrochloride (170 mg, 0.732 mmol) were dissolved in dry N,N-dimethylformamide (5 mL), and N,N-diisopropylethylamine (473 mg, 3.66 mmol) was added. The reaction solution was cooled to about 0°C, $T_3P$ (699 mg, 1.098 mmol, 50% N,N-dimethylformamide solution) was added dropwise. After the dropwise addition was complete, the mixture reacted at room temperature for 3 h. The reaction was quenched by adding water (20 mL), and the reaction solution was extracted with dichloromethane (30 mLx3).The organic phases were dried with anhydrous sodium sulfate, filtered, and concentrated to dryness. The residue was separated and purified with a silica gel plate (ethyl acetate: methanol (V/V)=10:1) to obtain a white solid, (4-(((1H-1,2,3-triazol-4-yl)methoxy)methyl)piperidin-1-yl)(2-((5-fluoro-2,3-dihydro-1H-inde n-2-yl)amino)pyrimidin-5-yl)methanone (**I-7**) (146 mg, yield 44.2%).

$^1$H NMR (400 MHz, DMSO-$d_6$) δ 8.36 (s, 2H), 7.98-7.96 (d, 1H), 7.81 (bs, 1H), 7.23-7.20 (m, 1H), 7.06-7.03 (dd, 1H), 6.97-6.92 (m, 1H), 4.71-4.62 (m, 1H), 4.53 (s, 2H), 3.29-3.18 (m, 6H), 2.94-2.83 (m, 4H), 1.86-1.79 (m, 1H), 1.70-1.67 (d, 2H), 1.23-1.07 (m, 2H).

LC-MS, M/Z: 452.4 [M+H]$^+$.

Example 8: Preparation of target compound **I-8**

(4-(((1H-1,2,3-triazol-4-yl)methoxy)methyl)piperidin-1-yl)(2-((5,6-difluoro-2,3-dihy dro-1H-inden-2-yl)amino)pyrimidin-5-yl)methanone (target compound **I-8**)

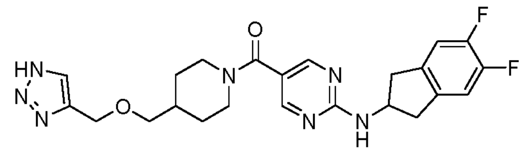

**I-8**

The synthetic scheme of the target compound **I-8** is illustrated as below:

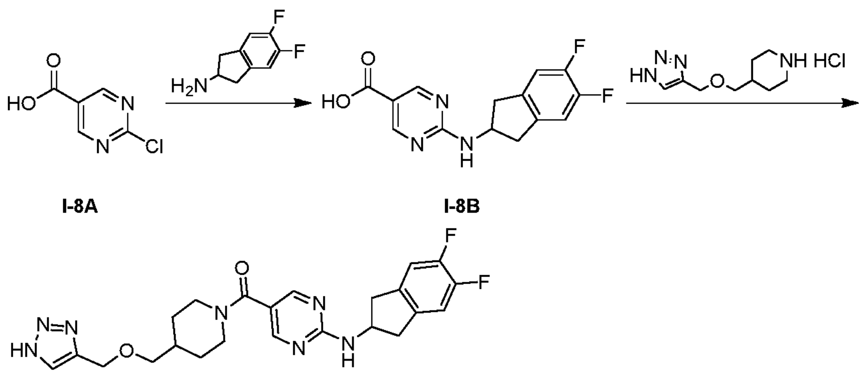

**I-8A** **I-8B**

**I-8**

Step 1: Synthesis of 2-((5,6-difluoro-2,3-dihydro-1H-inden-2-yl)amino)pyrimidine-5-carboxylic acid (I-8B)

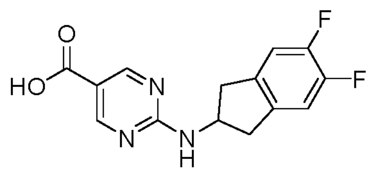

**I-8B**

5,6-difluoro-2,3-dihydro-1H-indene-2-amine (150 mg, 0.887 mmol), 2-chloropyrimidine-5-carboxylic acid (141 mg, 0.887 mmol), and DIEA (229 mg, 1.773 mmol) were added to NMP (2 mL). The system was heated to 95°C, and stirred overnight. After the reaction was completed, the reaction solution was concentrated to dryness, and pulped by adding isopropyl acetate, and then filtered to obtain the product, 2-((5,6-difluoro-2,3-dihydro-1H-inden-2-yl)amino)pyrimidine-5-carboxylic acid (I-8B) (200 mg, yield 77 %).

Step 2: Synthesis of (4-(((1H-1,2,3-triazol-4-yl)methoxy)methyl)piperidin-1-yl)(2-((5,6-difluoro-2,3-dihydro-1H-i nden-2-yl)amino)pyrimidin-5-yl)methanone (1-8)

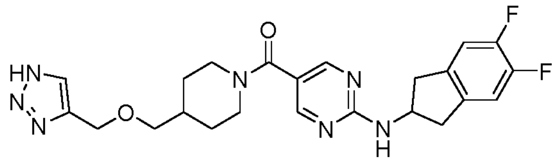

**I-8**

4-(((1H-1,2,3-triazol-4-yl)methoxy)methyl)piperidine hydrochloride (162 mg, 0.824 mmol), 2-((5, 6-difluoro-2,3-dihydro-1H-indan-2-yl)amino)pyrimidine-5-carboxylic acid (200 mg, 0.687 mmol), and DIEA (133 mg, 1.030 mmol) were sequentially added to DMF solution (3 mL). $T_3P$ (437 mg, 0.687 mmol, 50% DMF solution) was added dropwise under ice bath, and after the dropwise addition was complete, the mixture reacted overnight at room temperature. After the reaction was complete, the reaction solution was concentrated by removing DMF, added with 10 mL water solution of sodium bicarbonate, extracted with dichloromethane (10 mL×3), dried, concentrated, and separated on the preparation plate to obtain a pale yellow solid, (4-(((1H-1,2,3-triazol-4-yl)methoxy)methyl)piperidin-1-yl)(2-((5,6-difluoro-2,3-dihydro-1H-i nden-2-yl)amino)pyrimidin-5-yl)methanone (1-8) (70 mg, yield 21.7%).

LC-MS, M/Z: 470.3 $[M+H]^+$

$^1H$ NMR (400 MHz, DMSO-d6) δ 8.32 (s, 2H), 7.94 (s, 1H), 7.22 (s, 2H), 4.72-4.62 (m, 3H), 3.99 (b, 1H), 3.18 (m, 4H), 2.85 (m, 4H), 1.75-1.61 (m, 3H), 1.16-1.10 (m, 4H). Example 9: Preparation of target compound 1-9

(6-((1H-1,2,3-triazol-4-yl)methoxy)-2-azaspiro[3.3]heptan-2-yl)(2-((2,3-dihydro-1H-inden-2-yl)amino)pyrimidin-5-yl)methanone (target compound 1-9)

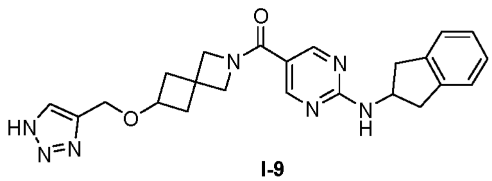

**I-9**

The synthetic scheme of the target compound 1 is illustrated as below:

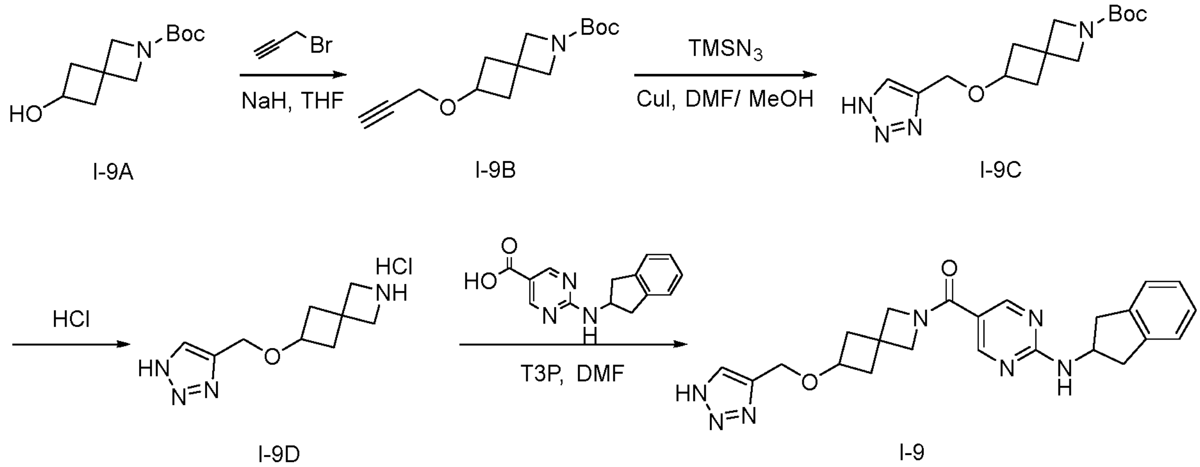


Step 1: Synthesis of tert-butyl 6-(prop-2-yn-1-yloxy)-2-azaspiro[3.3]heptane-2-carboxylate (I-9B)

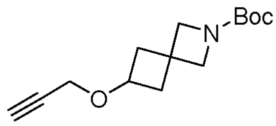

I-9B

Sodium hydride (11.25 g, 281 mmol) was dissolved in tetrahydrofuran (400 mL) and cooled to 0 to 5°C. Tert-butyl 6-hydroxy-2-azaspiro[tert-butyl][3.3]heptane-2-carboxylate (I-9A) (50 g, 234 mmol) was added, and then the temperature of the mixture was recovered to room temperature to react for 1h. 3-bromopropyne (41.8 g, 352 mmol) was added dropwise, and then the reaction was continued at 25 to 30°C for 20 h. The reaction was quenched by adding saturated ammonium chloride solution (1L) to the reaction solution, and the reaction solution was separated. The aqueous phase was extracted with ethyl acetate (500mL×2), and the organic phases were combined, dried over anhydrous sodium sulfate, and concentrated. The residue was separated and purified with a silica gel column (petroleum ether: ethyl acetate (V/V) = 10: 1) to obtain a yellow oily substance, tert-butyl 6-(prop-2-yn-1-yloxy)-2-azaspiro[3.3]heptane-2-carboxylate (I-9B) (60 g, yield 102%).

Step 2: Synthesis of *tert-butyl* 6-((1H-1,2,3-triazol-4-yl)methoxy)-2-azaspiro[3.3]heptane-2-carboxylate (I-9C)

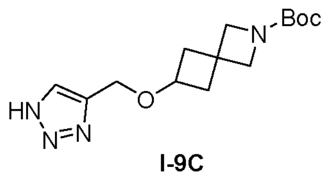

I-9C

Tert-butyl 6-(prop-2-yn-1-yloxy)-2-azaspiro[3.3]heptane-2-carboxylate (I-9B) (40 g, 159 mmol) and cuprous iodide (9.09 g, 47.7 mmol) were dissolved in N, N-dimethylformamide (400 mL) and methanol (40 mL). Azidotrimethylsilane (27.5 g, 239 mmol) was added dropwise into the reaction solution. In the reaction system, nitrogen replacement was performed for three times. The reaction solution was heated to 95°C and reacted for 18 h. The reaction solution was cooled to room temperature, added with water (1 L), and extracted with ethyl acetate (500 mL*3). The organic phases were combined, dried over anhydrous sodium sulfate, and concentrated. The residue was separated and purified with a silica gel column (petroleum ether: ethyl acetate (V/V) = 2: 1) to obtain a colorless oily substance, tert-butyl 6-((1H-1,2,3-triazol-4-yl)methoxy)-2-azaspiro[3.3]heptane-2-carboxylate (I-9C) (33.7g, yield 71.9%).

Step 3: Synthesis of 6-((1H-1,2,3-triazol-4-yl)methoxy)-2-azaspiro[3.3]heptane hydrochloride (I-9D)

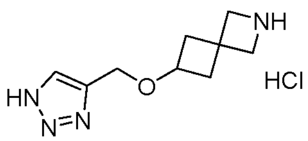

I-9D

Tert-butyl 6-((1H-1,2,3-triazol-4-yl)methoxy)-2-azaspiro[3.3]heptane-2-carboxylate (I-9C) (25 g, 85 mmol) was dissolved in 1,4-dioxane (150 mL), hydrogen chloride in dioxane solution (42.5 mL, 170 mmol, 4 M) was added, and the mixture was stirred for reaction at room temperature for 2 h. The solvent was removed under reduced pressure to obtain a crude product, and the crude product was directly used in the next step of reaction without purification.

Step 4: Synthesis of (6-((1H-1,2,3-triazol-4-yl)methoxy)-2-azaspiro[3.3]heptan-2-yl)(2-((2,3-dihydro-1H-inden-2-yl)amino)pyrimidin-5-yl)methanone (1-9)

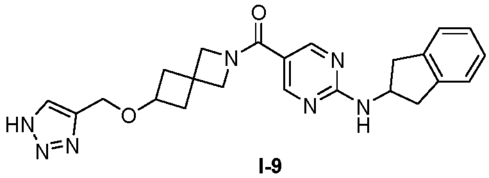

I-9

N,N-dimethylformamide (300 mL), N,N-diisopropylethylamine (50.6 g, 392 mmol), and 2-(2,3-dihydro-1H-inden-2-ylamino)pyrimidine-5-carboxylic acid (20 g, 78 mmol) were added into the crude product obtained in the previous step, i.e., 6-((1H-1,2,3-triazol-4-yl)methoxy)-2-azaspiro[3.3]heptane hydrochloride (I-9D) (19.52 g, 85 mmol). The reaction solution was cooled to about 0°C. 2,4,6-tripropyl-1,3,5,2,4,6-trioxotriphosphate-2,4,6-trioxide (74.8 g, 118 mmol, 50% N,N-dimethylformamide solution) was added dropwise, and then, the mixture reacted at 25 to 30°C for 20 h. The reaction was quenched by adding water (10mL) to the reaction solution, and the reaction solution was concentrated to dryness, then pulped by adding water (200 mL) and methanol (20 mL). After filtering, the solid was hot pulped twice by adding isopropyl acetate and methanol ((V/V) =10: 1), and then air-dried at 50°C for 20 h to obtain a white solid, (6-((1H-1,2,3-triazol-4-yl)methoxy)-2-azaspiro[3.3]heptan-2-yl)(2-((2,3-dihydro-1H-inden-2-yl)amino)pyrimidin-5-yl)methanone (1-9) (7.2 g, yield 21.3%).

$^1$H NMR (400 MHz, DMSO-$d_6$): δ8.53-8.49 (d, 2H), 8.09-8.07 (d, 1H), 7.78 (s, 1H), 7.19-7.16 (m, 2H), 7.13-7.10 (m, 2H), 4.67-4.59 (m, 1H), 4.40 (s, 3H), 4.34-4.29 (d, 1H), 3.97-3.91 (m, 3H), 3.29-3.19 (dd, 2H), 2.90-2.85 (dd, 2H), 2.47-2.41(m,2H), 2.04-1.99 (m, 2H).

LC-MS, M/Z (ESI): 432.4 (M+H).

Example 10: Preparation of target compound 1-10

(6-((1H-1,2,3-triazol-4-yl)methoxy)-2-azaspiro[3.3]heptan-2-yl)(2-((5-fluoro-2,3-dih ydro-1H-inden-2-yl)amino)pyrimidin-5-yl)methanone (target compound 1-10)

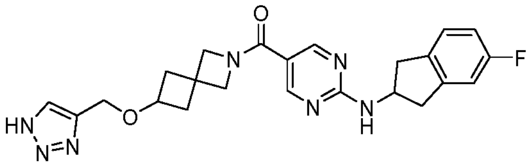

I-10

The synthetic scheme of the target compound 1-10 is illustrated as below:

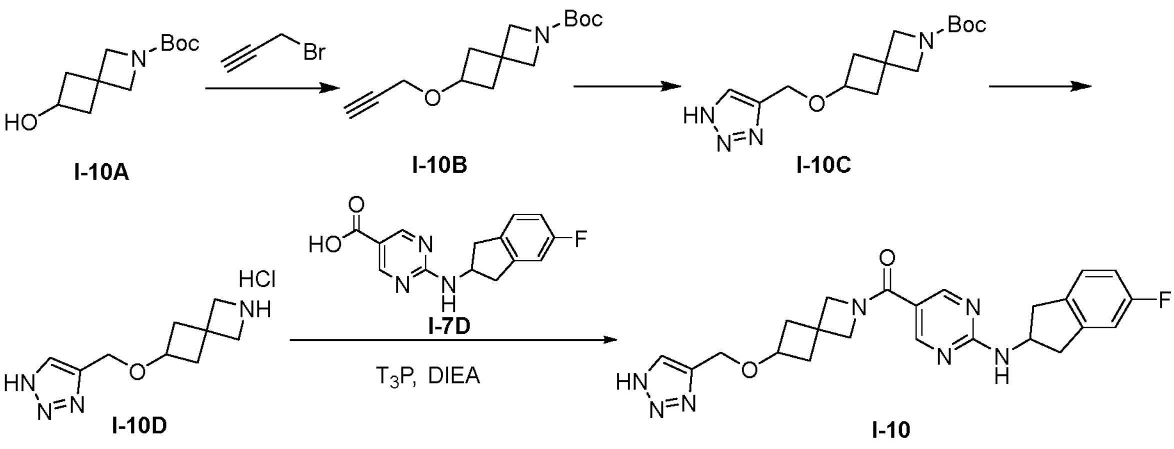


Step 1: Synthesis of tert-butyl 6-(prop-2-yn-1-yloxy)-2-azaspiro[3.3]heptane-2-carboxylate (I-10B)

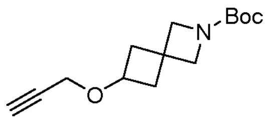

I-10B

Sodium hydride (60% purity, 11.25 g, 281 mmol) was dissolved in tetrahydrofuran (400 mL) and cooled to 0 to 5°C. Tert-butyl 6-hydroxy-2-azaspiro[3.3]heptane-2-carboxylate **(I-10A)** (50 g, 234 mmol) was added, and then temperature of the mixture was recovered to room temperature and the mixture reacted for 1h. 3-bromopropyne (41.8 g, 352 mmol) was added dropwise, and thereafter, the reaction continued at 25 to 30°C for 20 h. The reaction was quenched by adding saturated ammonium chloride solution (1 L) to the reaction solution, followed by liquid separation. The aqueous phase was extracted with ethyl acetate (500 mL×2), and the organic phases were combined, dried over anhydrous sodium sulfate, and concentrated. The residue was separated and purified with a silica gel column (petroleum ether: ethyl acetate (V/V)=10: 1) to obtain a yellow oily substance, tert-butyl 6-(prop-2-yn-1-yloxy)-2-azaspiro[3.3]heptane-2-carboxylate **(I-10B)** (60 g, yield 102%).

Step 2: Synthesis of tert-butyl 6-((1H-1,2,3-triazol-4-yl)methoxy)-2-azaspiro[3.3]heptane-2-carboxylate **(I-10C)**

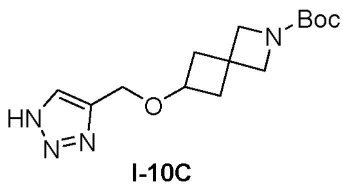

I-10C

Tert-butyl 6-(prop-2-yn-1-yloxy)-2-azaspiro[3.3]heptane-2-carboxylate **(I-10B)** (40 g, 159 mmol) and cuprous iodide (9.09 g, 47.7 mmol) were dissolved in N,N-dimethylformamide (400 mL) and methanol (40 mL), and azidotrimethylsilane (27.5 g, 239 mmol) was added dropwise to the reaction solution. In the reaction system, nitrogen replacement was performed for three times, the reaction solution was heated to 95°C to react for 18 h. The reaction solution was cooled to room temperature, added with water (1 L), and extracted with ethyl acetate (500 mL×3). The organic phases were combined, dried over anhydrous sodium sulfate, and concentrated. The residue was separated and purified with a silica gel column (petroleum ether: ethyl acetate (V/V) = 2: 1) to obtain a colorless oily substance, tert-butyl 6-((1H-1,2,3-triazol-4-yl)methoxy)-2-azaspiro[3.3]heptane-2-carboxylate **(I-10C)** (33.7 g, yield 71.9% ).

Step 3: Synthesis of 6-((1H-1,2,3-triazol-4-yl)methoxy)-2-azaspiro[3.3]heptane hydrochloride **(I-10D)**

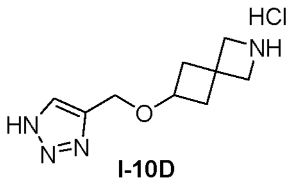

I-10D

Tert-butyl 6-((1H-1,2,3-triazol-4-yl)methoxy)-2-azaspiro[3.3]heptane-2-carboxylate **(I-10C)** (25 g, 85 mmol) was dissolved in 1,4-dioxane (150 mL), and 4M hydrogen chloride in 1,4-dioxane solution (42.5 mL, 170 mmol) was added. The mixture was stirred at room temperature for 2 h, and the solvent was removed under reduced pressure to obtain the crude product. The crude product was used directly in the next step of reaction without purification.

Step 4: Synthesis of (6-((1H-1,2,3-triazol-4-yl)methoxy)-2-azaspiro[3.3]heptan-2-yl)(2-((5-fluoro-2,3-dihydro-1H-inden-2-yl)amino)pyrimidin-5-yl)methanone (1-10)

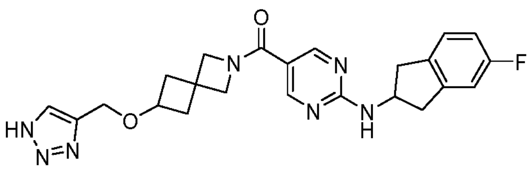

I-10

2-((5-fluoro-2,3-dihydro-1H-inden-2-yl)amino)pyrimidine-5-carboxylic acid (I-7D) (278 mg, 1.019 mmol) and 6-((1H-1,2,3-triazol-4-yl)methoxy)-2-azaspiro[3.3]heptane hydrochloride (I-10D) (235 mg, 1.019 mmol) were dissolved in dried N,N-dimethylformamide (5 mL), and N,N-diisopropylethylamine (658 mg, 5.09 mmol) was added. The reaction solution was cooled to about 0°C. $T_3P$ (972 mg, 1.528 mmol, 50% N,N-dimethylformamide solution) was added dropwise. After the dropwise addition was complete, the mixture reacted at room temperature for 3 h. The reaction was quenched by adding water (20 mL) into the reaction solution. The reaction solution was extracted with dichloromethane (30 mL×3). The organic phases were dried with anhydrous sodium sulfate, filtered, and concentrated to dryness. The residue was separated and purified with a silica gel plate (ethyl acetate: methanol (V/V)=10:1) to obtain a white solid, (6-((1H-1,2,3-triazol-4-yl)methoxy)-2-azaspiro[3.3]heptan-2-yl)(2-((5-fluoro-2,3-dihydro-1H-inden-2-yl)amino)pyrimidin-5-yl)methanone (1-10) (125.3 mg, yield 27.4%).

$^1H$ NMR (400 MHz, DMSO-$d_6$) δ 8.56-8.53 (d, 2H), 8.15-8.13 (d, 1H), 7.81(bs, 1H), 7.25-7.21 (m, 1H), 7.07-7.04 (m, 1H), 6.99-6.94 (m, 1H), 4.73-4.64 (m, 1H), 4.44 (s, 2H), 4.38-4.33 (d, 2H),4.06-3.96 (m, 3H), 3.30-3.20 (m, 2H), 2.95-2.84 (m, 2H), 2.48-2.45 (m, 2H), 2.08-2.00 (m, 2H).

LC-MS, M/Z: 450.4 $[M+H]^+$.

**Test Examples of Biological Activity and Related Properties**

Test Example 1: Autotaxin (ATX) enzyme activity inhibition test

The inhibitory effects of the compounds on the autotaxin enzyme were detected using the Autotaxin Inhibitor Screening Assay Kit (Cayman, 700580). First, the test compound was prepared as a 10 mM stock solution in DMSO solvent, and then the stock solution was diluted with DMSO into 8 gradient concentrations. Subsequently, the 8 concentrations were diluted with Autotaxin Assay buffer (1×) provided in the kit into 19× compound working solutions (DMSO content was 1.9%). The Autotaxin Assay Reagent (10×) was taken out and diluted by 10 times with Autotaxin Assay Buffer (1×). The Autotaxin Substrate was taken out, dissolved by adding 1.2 mL of Autotaxin Assay Buffer (1×), mixed evenly and let stand by at room temperature. In a 96-well plate, 150 μL of Autotaxin Assay Buffer (1×), 10 μL of the prepared and diluted 19× compound working solution, 10 μL of Autotaxin Assay Reagent (1×), and 20 μL of the dissolved Autotaxin Substrate were added to each of the wells for each concentration, and homogenously mixed. The 96-well plate was shaken in a constant temperature shaker at 37°C and incubated in the dark for 30 min, and then the plate was taken out and placed on a microplate reader to read OD405. The experimental results were input into GraphPad Prism software, and the $IC_{50}$ of each compound was calculated by fitting calculation.

According to the above experimental methods, the inhibitory effects of the control compound 1 and compounds of the present disclosure on ATX enzyme activity were determined. The results are listed in Table 1:

[Table 1] Results of inhibitory effects of the test compounds on ATX enzyme activity

| Test compound | $IC_{50}$ (nM) |
|---|---|
| Control compound 1 | 2.60 |
| Compound 1-1 | 2.05 |
| Compound 1-2 | 3.39 |
| Compound 1-3 | 6.71 |
| Compound 1-5 | 1.29 |
| Compound 1-6 | 1.43 |
| Compound 1-7 | 1.16 |
| Compound 1-8 | 2.04 |
| Compound 1-9 | 1.43 |

(continued)

| Test compound | $IC_{50}$ (nM) |
|---|---|
| Compound I-10 | 1.08 |

Experimental results revealed that $IC_{50}$ of the compounds of the present disclosure is similar as or lower than that of the control compound 1, indicating excellent inhibitory activities of the compounds of the present disclosure on ATX enzyme. In addition, the inhibitory activities of compounds 1-1, and 1-5 to I-10 on the ATX enzyme are superior over that of the control compound 1.

Test Example 2: Inhibition test of activity of ATX enzyme in human plasma

Whole blood was collected from healthy volunteers and anticoagulated with heparin. The blood collection tubes were centrifuged at 3,000 rpm for 10 min, and the plasma was taken and stored at -80°C for use.

The compound was serially diluted with DMSO according to the conventional concentration requirements, then 3 μL of each of the diluted compound solutions was added to a 96-well plate, and 147 μL of PBS was added to each well containing 3 μL of the compound solution. After mixing homogenously, 50 μL of the mixture was taken out and added to a new 96-well plate. The human plasma was taken out from the -80°C refrigerator and thawed through rapid shaking in a 37°C water bath. 50 μL of the human plasma was taken and added to the 96-well plate containing 50 μL of the diluted compound (the final system contained 1% DMSO). The group without the compound was set as the positive group. The 96-well plate was shaken and mixed uniformly, and incubated at 37°C for 3 h. A blank group was also provided, and the plasma of the blank group was stored at -80°C. The blank group is provided to determine the baseline concentration of endogenous LPA.

After the incubation was finished, the blank group was thawed on ice and transferred to an incubation plate. Excess acetonitrile containing the internal standard LPA17: 0 was added to the incubation plate to precipitate the plasma protein. After vortex centrifugation, the supernatant was taken and diluted, and a peak area of LPA18: 2 and a peak area of internal standard LPA17: 0 were detected using LC-MSMS mass spectrometry.

A ratio of the peak area of LPA18: 2 to the peak area of internal standard LPA17: 0 was calculated, and a formation inhibition rate of LPA18: 2 was calculated according to the following formula:

Inhibition rate (%) = 100-(compound group with different concentrations - blank group)/(positive group - blank group)*100

According to the inhibition rates of different concentrations of the compound, the $IC_{50}$ value of the compound for inhibiting activity of ATX enzyme in human plasma was calculated.

The inhibitory effect on activity of ATX enzyme in human plasma of control compound 1, control compound 2 and compounds of the present disclosure were tested according to the above experimental method, and the results are listed in Table 2.

[Table 2] Results of inhibitory effects of test compounds on activity of ATX enzyme in human plasma

| Test compound | $IC_{50}$ (nM) |
|---|---|
| Control compound 1 | 13.0 |
| Control compound 2 | 263 |
| Compound 1-1 | 8.73 |
| Compound 1-2 | 24.7 |
| Compound 1-3 | 42.1 |
| Compound 1-5 | 30.9 |
| Compound 1-6 | 41.2 |
| Compound 1-7 | 14.6 |
| Compound 1-9 | 2.74 |

(continued)

| Test compound | $IC_{50}$ (nM) |
|---|---|
| Compound I-10 | 11.8 |

It is known that the control compounds 1 and 2 have good inhibitory activity on ATX enzyme. The experimental results in Table 2 indicate that the compounds of the present disclosure have good inhibitory effect on ATX enzyme in human plasma, especially compounds 1-1 and 1-9 and I-10 have higher inhibitory effect on ATX enzyme in human plasma than the control compounds 1 and 2.

Test Example 3: Inhibition test of activity of ATX enzyme in rat plasma

Whole blood was collected from rats and anticoagulated with heparin. The blood collection tubes were centrifuged at 3,000 rpm for 15 min, and the plasma was placed on ice for use.
The compound was serially diluted with DMSO: 30% ACN=1:19 according to the conventional concentration requirements, and then placed on ice. 147 μL of plasma, which was placed on ice, was added to a 96-well plate, and then 3 μL of each of the gradient solutions of the compound (finally 1‰ DMSO) was added. A group containing none of the compounds was set as the positive group. The 96-well plate was mixed uniformly, and incubated at 37°C for 3 h. In addition, a blank group was also provided. At the beginning of the incubation, the plasma of blank group was added to with excessive acetonitrile containing the internal standard LPA17:0 to precipitate the plasma protein, vortexed to mix, and stored at 4°C. The blank group was provided to determine the baseline concentration of endogenous LPA.
After the incubation was finished, excessive acetonitrile containing the internal standard LPA17: 0 was added to a incubation plate to precipitate the plasma protein, the above-mentioned blank group, in which the proteins had been precipitated, was transferred to the incubation plate, after vortex centrifugation, the supernatant was taken and diluted. A peak area of LPA18: 2 and a peak area of internal standard LPA17: 0 were detected using LC-MSMS mass spectrometry.
A ratio of the peak area of LPA18: 2 to the peak area of internal standard LPA17: 0 was calculated, and a formation inhibition rate of LPA18: 2 was calculated according to the following formula:

Inhibition rate (%) = 100-(compound group with different concentrations - blank group)/(positive group - blank group)*100

According to the inhibition rates of different concentrations of the compound, the $IC_{50}$ value of the compound for inhibiting activity of ATX enzyme in human plasma, was calculated.
The inhibitory effect on activity of ATX enzyme in rat plasma of control compound 1, control compound 2 and compounds of the present disclosure were tested according to the above experimental method, and the results are listed in Table 3.

[Table 3] Results of inhibitory effects of test compounds on activity of ATX enzyme in rat plasma

| Test compound | $IC_{50}$ (nM) |
|---|---|
| Control compound 1 | 13.6 |
| Control compound 2 | 413 |
| Compound 1-1 | 6.49 |
| Compound 1-4 | 32.3 |
| Compound 1-5 | 10.7 |
| Compound 1-6 | 22.9 |
| Compound 1-7 | 14.7 |
| Compound 1-8 | 136 |
| Compound 1-9 | 2.83 |
| Compound I-10 | 4.8 |

The experimental results indicate that the compounds of the present disclosure have good inhibitory activity on ATX enzyme in rat plasma, especially compounds 1-1, I-5, I-9, and I-10 have higher inhibitory activity on ATX enzyme in rat plasma than the control compounds 1 and 2.

Test Example 4: Human Liver Microsome Stability Test

The human liver microsome stability test was performed by incubating the compound and human liver microsomes in vitro for detection. First, the test compound was prepared into a 10 mM stock solution in DMSO solvent, and then the compound was diluted to 0.5 mM with acetonitrile. Human liver microsomes (Corning) were diluted with PBS and prepared as a microsome/buffer solution, which was used to dilute the 0.5 mM compound solution into a working solution. In the working solution, a concentration of the compound was 1.5 μM, and a concentration of human liver microsomes was 0.75 mg/ml. A deep-well multi-well plate was taken, 30 μL of the working solution and 15 μL of pre-warmed NADPH solution (6 mM) were sequentially added to each well to initiate a reaction, and the reaction was incubated at 37°C. At 0, 5, 15, 30, and 45 min of the incubation, 135 μL of acetonitrile was added to the corresponding wells to terminate the reaction. After the reaction was terminated with acetonitrile at the last time point of 45 min, the deep-well multi-well plate was vortexed for 10 min (600 rpm/min), and then centrifuged for 15 min. After centrifugation, the supernatant was collected, and added with purified water in a ratio of 1: 1 to perform LC-MS/MS detection. Accordingly, a ratio of a peak area of compound to a peak area of internal standard at each time point was obtained, and the peak area ratios of the compound at 5, 15, 30, and 45 min were compared with the peak area ratio at 0 min to calculate the remaining percentage of the compound at each time point. $T_{1/2}$ was calculated by using Excel.

The human liver microsome stability of control compound 1, control compound 2 and compounds of the present disclosure were tested according to the above experimental method, and the results are listed in Table 4.

[Table 4] Results of human liver microsome stability test

| Compound | Remaining percentage (%) of compound after incubation for 30 min | $T_{1/2}$ (min) |
|---|---|---|
| Control compound 1 | 41.2 | 24.1 |
| Control compound 2 | 24.8 | 16 |
| Compound 1-1 | 85.6 | 99 |
| Compound 1-2 | 39.65 | 24 |
| Compound 1-9 | 95.5 | 242 |

Compared with the control compound 1 and control compound 2, the compounds of the present disclosure exhibited better liver metabolic stability, and they were metabolized more slowly in the human body, and had higher exposure. $T_{1/2}$ of the compounds of the present disclosure is better than that of the control compounds. Accordingly, the clinical dosage and frequency of administration can be reduced, the toxic and side effects of clinical administration can be lowered, and the clinical compliance was improved.

Test Example 5: Detection of inhibitory effect of compounds on hERG by using full-automatic electrophysiological patch clamp QPatch

Full-automatic electrophysiological patch clamp QPatch was used to detect the inhibitory effect of compounds on hERG. Cells used in this test were CHO cell line transfected with cDNA of hERG and stably expressing hERG channels (provided by Sophion Bioscience, Denmark), and the cell passage number was P24. The cells were cultured in a medium containing the following components (all purchased from Invitrogen): Ham's F12 medium, inactivated fetal bovine serum (10% (v/v)), hygromycin B (100 μg/ml), and Geneticin (100 μg/ml). CHO hERG cells were grown in a petri dish containing the above-mentioned medium and cultured in an incubator at 37°C and containing 5% $CO_2$.

An extracellular fluid (2 mM $CaCl_2$, 1 mM $MgCl_2$, 4 mM KCl, 145 mM NaCl, 10 mM Glucose, 10 mM HEPES, pH: about 7.4, osmotic pressure: about 305 mOsm) and an intracellular fluid (5.374 mM $CaCl_2$, 1.75 mM $MgCl_2$, 120 mM KCl, 10 mM HEPES, 5 mM EGTA, 4 mM Na-ATP, pH about 7.25, osmotic pressure about 295 mOsm) were prepared.

The test compound was prepared into a 10 mM stock solution in DMSO solvent, the compound was diluted to 3 mM, 1 mM, 0.3 mM, and 0.1 mM with DMSO, and then the compound was diluted to 30 μM, 10 μM, 3 μM, 1 μM, 0.3 μM and 0.1 μM with the extracellular fluid, so that except that the final concentration of DMSO in the 30 μM compound was 0.3%, the final concentration of DMSO in compound solutions of all other concentrations was 0.1%.

The CHO hERG cells, after being digested and resuspended, were added to the full-automatic QPatch system

(Sophion, Denmark), and subjected to a test according to the following preset procedure.

After reaching the ruptured whole cell configuration state in the initial stage, the whole cell current was recorded at room temperature (about 25°C) for at least 120 seconds in order to achieve stability. The stable cells were selected for the test. During the whole test, the cells were clamped at a voltage of -80 mV, the cell clamp voltage was depolarized to +20mV to activate the hERG potassium channels, and after 2.5 seconds, the cell clamp voltage was at -50 mV to eliminate inactivation and generate outward tail current. The peak tail current was used as the value of the hERG current. The voltage mode described above was applied to the cells for electrophysiological test every 15 seconds. An intracellular liquid containing 0.1% dimethyl sulfoxide (solvent) was added to the cells to establish a baseline, and then the current was allowed to stabilize for 3 min. After the compound solution was added, the cells were kept in the test environment until the effect of the compound reached a steady state or until reaching 4 min. In the test experiments with different concentration gradients of the compound, the compound was added from low to high concentration to the clamped cells. After the compound test was finished, the cells were washed with the extracellular fluid until the current returned to a stable state. The test data was analyzed by Qpatch analysis software provided by Sophion, Excel, and Graphpad Prism, etc.

According to the above experimental methods, the inhibitory effects of the control compound 1 and compounds of the present disclosure on hERG were determined. The results are listed in Table 5:

[Table 5] Results of inhibitory effects of compounds on hERG

| Compound | hERG $IC_{50}$ (μM) | hERG $IC_{50}$/ATX $IC_{50}$ |
|---|---|---|
| Control compound 1 | 6.69 | 6.69/2.60=2.6 |
| Compound 1-1 | 7.93 | 7.93/2.05=3.87 |
| Compound 1-9 | 13.4 | 13.4/1.43=9.37 |

Compared with the control compound 1, the compounds of the present disclosure exhibit weaker inhibitory effect on hERG. Taking the $IC_{50}$ value of the compounds for inhibiting activity of ATX enzyme into consideration, it indicated that the compounds of the present disclosure exhibit a better safety window for hERG inhibition, and have significant cardiac safety advantages.

Test Example 6: Pharmacokinetic test in rats

For the pharmacokinetic test of rats, 3 male SD rats (180-240 g) were used, and fasted overnight. The rats were orally administered by gavage (10 mg/kg). Blood was collected before the administration, and at 15 min, 30 min, 1 h, 2 h, 4h, 8 h and 24 h after the administration. The blood samples were centrifuged at 8,000 rpm at 4°C for 6 min, and plasma was collected and stored at -20°C. The plasma at each time point was taken and added with an acetonitrile solution containing internal standard in 3 to 5 times the amount, vortexed and mixed for 1 min, and centrifuged at 13,000 rpm at 4°C for 10 min. The supernatant was collected, added with water in 3 times the amount, and mixed. An appropriate amount of the mixture was taken for LC-MS/MS analysis. The main pharmacokinetic parameters were analyzed using WinNonlin 7.0 software with non-compartmental model.

[Table 6] Results of pharmacokinetic test in rats

| Test compound | Pharmacokinetic parameters of Rats (oral administration by gavage) | | |
|---|---|---|---|
| | Cmax (ng/mL) | Tmax (hr) | AUC0-t (h*ng/mL) |
| Control compound 1 | 2591 | 0.42 | 4874 |
| Control compound 2 | 582 | 0.5 | 2568 |
| Compound 1-1 | 3176 | 0.5 | 5720 |
| Compound 1-9 | 973 | 0.42 | 1414 |

The experimental results indicate that, compared with the control compounds 1 and 2, the compounds of the present disclosure exhibit better pharmacokinetic properties.

Test Example 7: Pharmacokinetic test in mice

For the pharmacokinetic test of mice, male CD-1 mice (20 g to 25 g) were used, and fasted overnight. The mice were orally administered by gavage (10 mg/kg). Blood was collected before the administration, and at 15 min, 30 min, 1 h, 2 h, 4h, 8 h and 24 h after the administration. The blood samples were centrifuged at 8,000 rpm at 4°C for 6 min, and plasma was collected and stored at -20°C. The plasma at each time point was taken and added with an acetonitrile solution containing internal standard in 3 to 5 times the amount, vortexed and mixed for 1 min, and centrifuged at 13,000 rpm at 4°C for 10 min. The supernatant was collected, added with water in 3 times the amount, and mixed. An appropriate amount of the mixture was taken for LC-MS/MS analysis. The main pharmacokinetic parameters were analyzed using WinNonlin 7.0 software with non-compartmental model.

[Table 7] Results of pharmacokinetic test in mice

| Test compound | Pharmacokinetic parameters of mice (oral administration by gavage) | | |
|---|---|---|---|
| | Cmax (ng/mL) | Tmax (hr) | AUC0-t (h*ng/mL) |
| Control compound 1 | 1496 | 0.25 | 2572 |
| Compound 1-1 | 1864 | 0.42 | 2393 |
| Compound 1-9 | 3874 | 0.33 | 4594 |

The experimental results indicate that, compared with the control compound 1, the compounds of the present disclosure exhibit better pharmacokinetic properties.

Test Example 8: Pharmacokinetic test in canines

For the pharmacokinetics test of canines, 3 male beagle dogs (8 to 10 kg) were used, and fasted overnight. The dogs were orally administered by gavage (5 mg/kg). The rest of the operation is the same as the pharmacokinetic test in rats.

[Table 8] Results of pharmacokinetic test in canines

| Test compound | Canine | | |
|---|---|---|---|
| | Cmax (ng/mL) | Tmax (hr) | AUC0-t (h*ng/mL) |
| Control compound 1 | 2386 | 1 | 10120 |
| Control compound 2 | 2098 | 2 | 14183 |
| Compound 1-1 | 3667 | 1.17 | 17621 |
| Compound 1-9 | 2094 | 1.17 | 9414 |

The test results indicate that, compared with the control compounds 1 and 2, the compounds of the present disclosure exhibit better pharmacokinetic properties.

Test example 9: Thermodynamic solubility test

The following solutions were prepared: phosphate buffer (PBS, pH 7.4), FeSSIF solution (pH 5.8, containing 10 mM sodium taurocholate, 2 mM lecithin, 81.65 mM sodium hydroxide, 125.5 mM sodium chloride, 0.8 mM sodium oleate, 5 mM glyceryl monooleate, 55.02 mM maleic acid), and FaSSGF solution (pH 1.6, 1L solution containing 80 μM sodium taurocholate, 20 μM lecithin, 0.1g pepsin, and 34.2 mM sodium chloride).

The compound was accurately weighed, and the prepared phosphate buffer (pH 7.4), FeSSIF solution (pH 5.8) and FaSSGF solution (pH 1.6) were added to prepare a solution with a concentration of 4 mg/mL, which was shaken at 1,000 rpm for 1 hour, and then incubated overnight at room temperature. After the incubation, the solution was centrifuged at 12,000 rpm for 10 min to remove undissolved particles, and the supernatant was transferred to a new centrifuge tube. The supernatant was diluted appropriately, then added with an acetonitrile solution containing the internal standard, and quantified using a standard curve prepared with the same matrix.

[Table 9] Results of thermodynamic solubility test

| Test compound | Solubility (μg/mL) | | |
|---|---|---|---|
| | FaSSGF (pH 1.6) | FeSSIF (pH 5.8) | PBS (pH 7.4) |
| Control compound 1 | 66.5 | 18.3 | 6.3 |
| Compound 1-4 | 331.8 | / | 147.2 |
| Compound 1-5 | 448.4 | 316.9 | 129.1 |
| Compound 1-6 | 165.2 | 49 | 18.6 |

The test results indicate that, compared with the control compound, the thermodynamic solubilities of the compounds of the present disclosure in simulated gastric juice, simulated intestinal fluid, and neutral conditions are significantly improved. Accordingly, it is expected that the intestinal absorption of the compounds in the human body will be greatly improved, and the oral administration exposure will be higher, such that the clinical administration dose can be reduced and clinical compliance can be improved.

Although the embodiments of the present disclosure are illustrated and described above, it can be understood that the above-mentioned embodiments are illustrative and should not be construed as limiting the present disclosure. Those skilled in the art can make changes, modifications, substitutions, and variations based on the above-mentioned embodiments within the scope of the present disclosure.

## Claims

1. A compound, the compound being a compound represented by Formula (I), or being a tautomer, stereoisomer, hydrate, solvate, pharmaceutical acceptable salt, or prodrug of the compound represented by Formula (I):

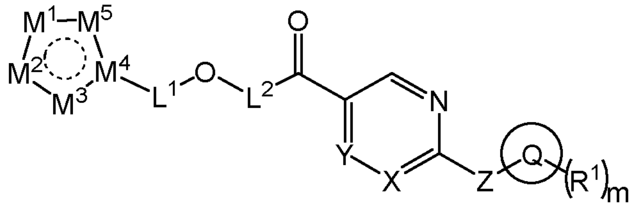

(I)

wherein Q is selected from $C_3$-$C_{10}$ cycloalkyl, a three- to ten-membered heterocyclic group, $C_6$-$C_{10}$ aryl, or a five- to ten-membered heteroaryl; the $C_3$-$C_{10}$ cycloalkyl, the three- to ten-membered heterocyclic group, the $C_6$-$C_{10}$ aryl, and the five- to ten-membered heteroaryl are optionally substituted by m groups $R^1$, m is an integer selected from 0 to 9;

each of the m groups $R^1$ is independently selected from hydrogen, halogen, -CN, -OH, -SH, $-NO_2$, $C_1$-$C_{10}$ alkyl, $C_3$-$C_{10}$ cycloalkyl, or $C_1$-$C_{10}$ alkoxy;

X and Y are independently selected from -N= or $-C(R^2)=$;

Z is selected from -O-, -S-, or $-N(R^3)-$;

$L^1$ is selected from a single bond or

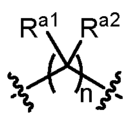;

$L^2$ is selected from a single bond or

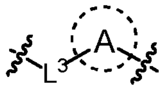;

$L^3$ is selected from a single bond or

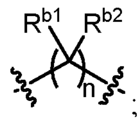;

$R^{a1}$, $R^{a2}$, $R^{b1}$, and $R^{b2}$ are each independently selected from hydrogen, halogen, cyano, -OH, or $C_1$-$C_3$ alkyl;

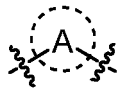

is selected from $C_3$-$C_8$ cycloalkyl, $C_4$-$C_8$ heterocycloalkyl, or a seven- to eight-membered N-spirocyclic group, and the $C_{3-8}$ cycloalkyl, the $C_{4-8}$ heterocycloalkyl, and the seven- to eight-membered N-spirocyclic group are optionally substituted with at least one $R^c$;
each of the at least one $R^c$ is independently selected from -H, halogen, -OH, -CN, or $C_1$-$C_3$ alkyl;
n is an integer selected from 0 to 3;
$M^1$, $M^2$, $M^3$, $M^4$, and $M^5$ are each independently selected from -N=, -N($R^4$)-, or -C($R^5$)=, at least one of $M^1$, $M^2$, $M^3$, $M^4$, or $M^5$ is -N= or -N($R^4$)-, and at least one of $M^1$, $M^2$, $M^3$, $M^4$, or $M^5$ is -C($R^5$)=;
$R^2$ and $R^5$ are each independently selected from hydrogen, halogen, -CN, -OH, -SH, or $C_1$-$C_3$ alkyl;
$R^3$ and $R^4$ are each independently selected from hydrogen or $C_1$-$C_3$ alkyl; and
the compound comprises none of the following compounds or enantiomers or stereoisomers thereof:

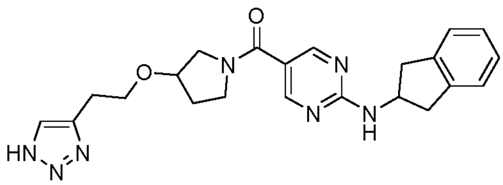 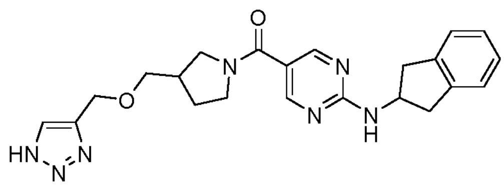

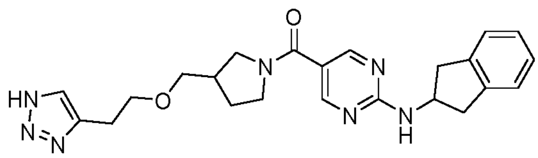 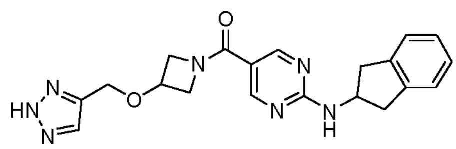

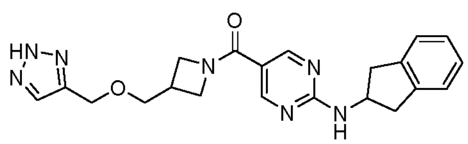 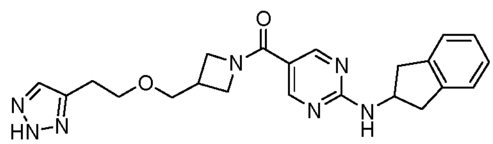

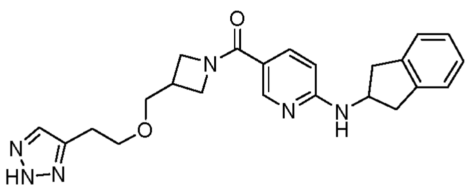 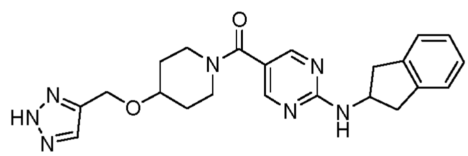

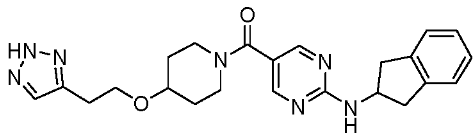
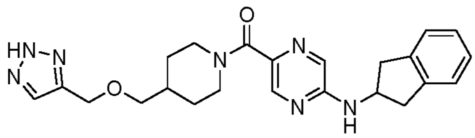

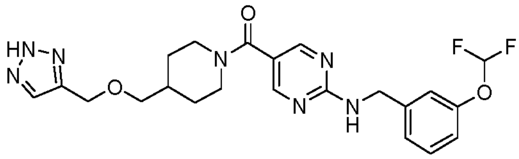
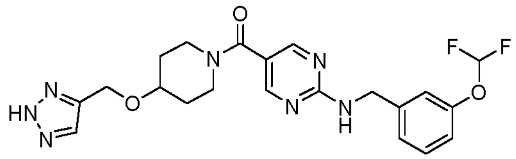

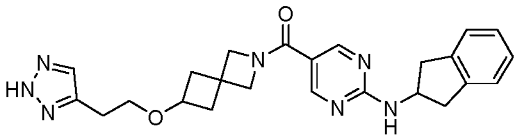
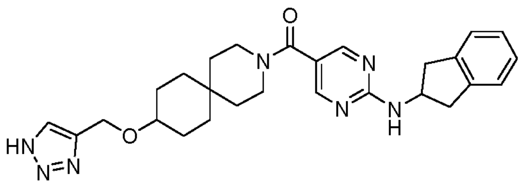

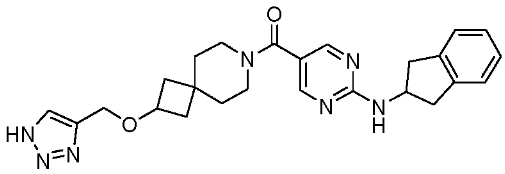
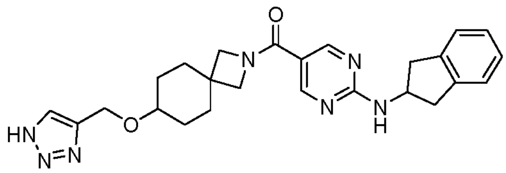
.

**2.** The compound according to claim 1, wherein in the compound represented by Formula (I):

Q is selected from cyclopropyl, cyclobutyl, cyclopentyl, cyclohexyl, azetidinyl, pyrrolidinyl, tetrahydrofuranyl, piperidinyl, piperazinyl, morpholinyl, phenyl, indenyl, naphthyl, pyrrolyl, pyrazolyl, imidazolyl, triazolyl, tetrazolyl, furyl, thienyl, thiazolyl, oxazolyl, pyridinyl, pyrimidinyl, pyrazinyl, pyridazinyl, benzimidazolyl, indolyl, or quinolinyl;
m is 0, 1, 2 or 3; and
$M^1$, $M^2$, and $M^3$ are each selected from -N= or -N($R^4$)-, and each of $M^4$ and $M^5$ is -C($R^5$)= .

**3.** The compound according to claim 1, wherein

each of the m groups $R^1$ is independently selected from hydrogen, halogen, or $C_1$-$C_3$ alkyl, and preferably, $R^1$ is selected from H, F, Cl, methyl, or ethyl;
optionally, when Q is indenyl, the indenyl is optionally unsubstituted with $R^1$ or substituted with one or two groups $R^1$;
optionally, when X is -N=, Y is -C($R^2$)=; or when X is -C($R^2$)=, Y is -N=;
optionally, Z is selected from -O-, -S-, or -N($R^3$)-; and when Z is -N($R^3$)-, $R^3$ is hydrogen or $C_1$-$C_3$ alkyl;
optionally, when $L^1$ is

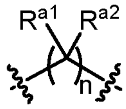,

n is 1 or 2;
optionally, when $L^1$ is

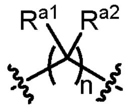,

$R^{a1}$ and $R^{a2}$ are each independently selected from hydrogen, F, Cl, -CN, methyl, or ethyl;
optionally, when $L^1$ is

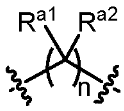,

$R^{a1}$ and $R^{a2}$ are each independently selected from hydrogen, F, Cl, -CN, methyl, or ethyl, and at least one $R^{a1}$ is hydrogen;
optionally, when $L^3$ is

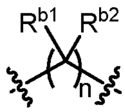,

n is 1 or 2;
optionally, when $L^3$ is

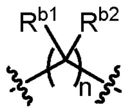,

$R^{b1}$ and $R^{b2}$ are each independently selected from hydrogen, -F, -Cl, -CN, methyl, or ethyl;
optionally, when $L^3$ is

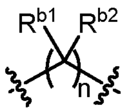,

$R^{b1}$ and $R^{b2}$ are each independently selected from hydrogen, -F, -Cl, -CN, methyl, or ethyl, and at least one $R^{b1}$ is hydrogen;
optionally, when said $L^2$ is

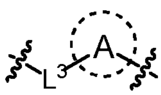, 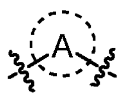

is selected from the following groups unsubstituted or optionally substituted with at least one $R^c$: piperidinyl, or a seven- to eight-membered N-spirocyclic group;
optionally, when said $L^2$ is

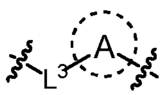, 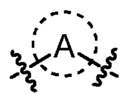

is piperidinyl unsubstituted or optionally substituted with at least one $R^c$, said $L^3$ and N on the piperidinyl are independently in an ortho, meta or para position relative to each other;
optionally, when said $L^2$ is

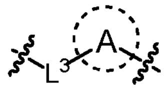 , 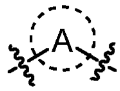

is piperidinyl unsubstituted or optionally substituted with at least one $R^c$, the at least one $R^c$ and N on the piperidinyl are independently in an ortho or meta position relative to each other;
optionally, when said $L^2$ is

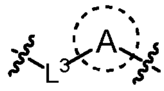 , 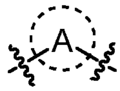

is piperidinyl unsubstituted or optionally substituted with at least one $R^c$, the at least one $R^c$ is selected from -H, -F, -Cl, methyl, or ethyl;
optionally, when said $L^2$ is

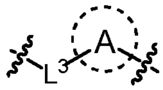

and $L^3$ is a single bond,

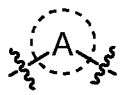

is selected from seven- to eight-membered N-spirocyclic groups unsubstituted or optionally substituted with at least one $R^c$;
optionally, when said

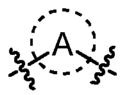

is selected from the seven- to eight-membered N-spirocyclic groups unsubstituted or optionally substituted with at least one $R^c$, the N-spirocyclic group is selected from

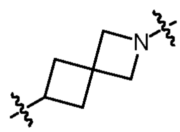

or

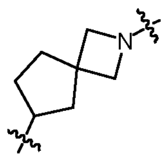

;

and
optionally, $M^1$, $M^2$, and $M^3$ are each selected from -N= or -N($R^4$)-, $M^4$ and $M^5$ are both -C($R^5$)=, $R^4$ is hydrogen, and $R^5$ is hydrogen.

**4.** The compound according to claim 1, wherein the compound represented by Formula (I) is a compound represented by the following Formula (I-0):

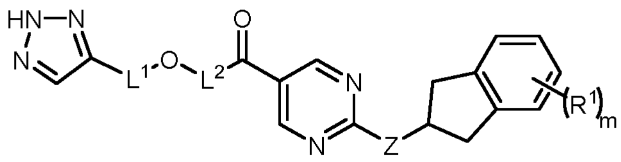

(I-0)

wherein $R^1$ is -F or methyl;

optionally, m is 0, 1, or 2;
optionally, Z is -NH-;
optionally, $L^1$ is selected from

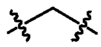

or

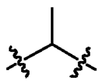;

optionally, $L^2$ is selected from

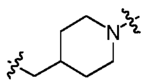, 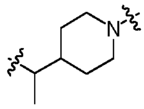, 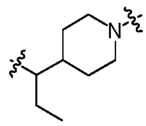, 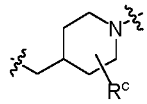,

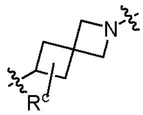,

or

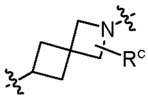,

wherein $R^c$ is selected from -H, -F, -Cl, methyl, or ethyl.

**5.** A compound represented by Formula (1-1), or a stereoisomer, tautomer, hydrate, solvate, pharmaceutical acceptable salt, or prodrug of the compound represented by Formula (I-1):

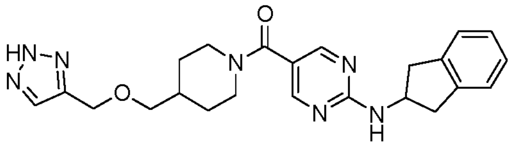

I-1

.

6. A compound represented by Formula (1-9), or a stereoisomer, tautomer, hydrate, solvate, pharmaceutical acceptable salt, or prodrug of the compound represented by Formula (1-9):

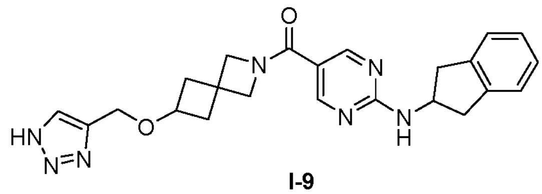

I-9

.

7. The compound according to claim 1, wherein the compound represented by Formula (I) is any one of the following compounds:

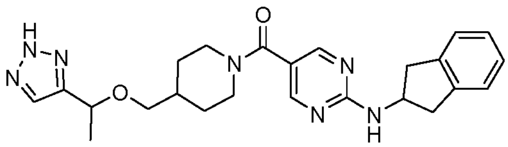

I-2

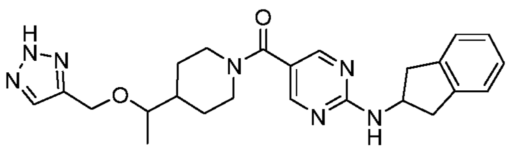

I-3

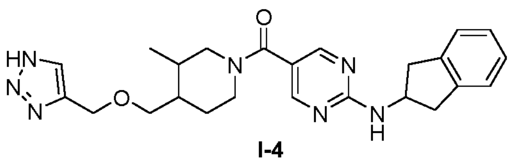

I-4

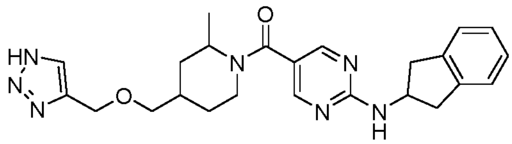

I-5

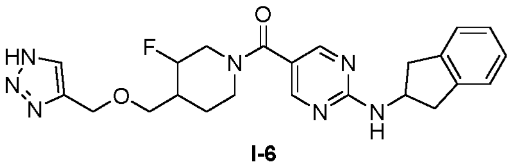

I-6

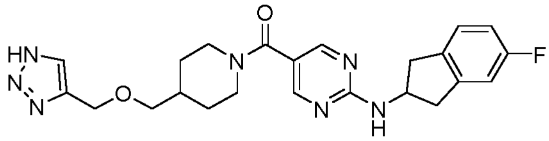

I-7

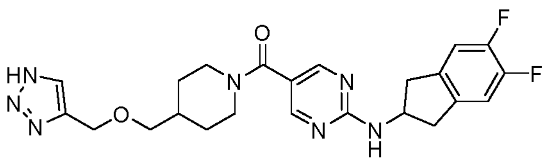

I-8

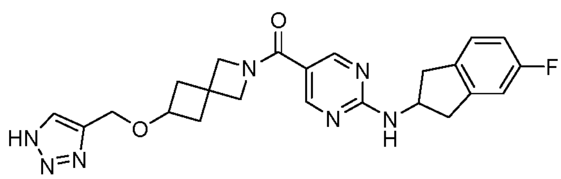

I-10

.

8. A pharmaceutical composition, comprising an effective dose of the compound according to any one of claims 1 to 7.

9. Use of the compound according to any one of claims 1 to 7, the tautomer, stereoisomer, hydrate, solvate, pharmaceutical acceptable salt, or prodrug thereof in manufacture of a medicament for treating an ATX-related disease.

10. The use according to claim 9, wherein the ATX-related disease is selected from cancer, metabolic diseases, kidney diseases, liver diseases, fibrosis diseases, interstitial lung diseases, proliferative diseases, inflammatory diseases, pain, autoimmune diseases, respiratory diseases, cardiovascular diseases, neurodegenerative diseases, dermatological disorders, and/or diseases related to abnormal angiogenesis.

**11.** The use according to claim 9, wherein the ATX-related disease is selected from interstitial lung diseases, pulmonary fibrosis, liver fibrosis, or renal fibrosis, and preferably, the ATX-related disease is idiopathic pulmonary fibrosis.

**12.** The use according to claim 9, wherein the ATX-related disease is a metabolic disease, and preferably, selected from type II diabetes or non-alcoholic steatohepatitis.

**13.** The use according to claim 9, wherein the ATX-related disease is selected from neuropathic pain or inflammatory pain, and preferably, the ATX-related disease is osteoarthritis-related pain.

**14.** The use according to claim 9, wherein the ATX-related disease is cancer.

**15.** A method for treating an ATX-related disease, comprising: administering the pharmaceutical composition according to claim 8 to a patient suffering from the ATX-related disease.

**16.** The method according to claim 15, wherein the ATX-related disease is selected from cancer, metabolic diseases, kidney diseases, liver diseases, fibrosis diseases, interstitial lung diseases, proliferative diseases, inflammatory diseases, pain, autoimmune diseases, respiratory diseases, cardiovascular diseases, neurodegenerative diseases, dermatological disorders, and/or diseases related to abnormal angiogenesis.

**17.** The method according to claim 15, wherein the ATX-related disease is selected from interstitial lung diseases, pulmonary fibrosis, liver fibrosis, or renal fibrosis, and preferably, the ATX-related disease is idiopathic pulmonary fibrosis.

**18.** The method according to claim 15, wherein the ATX-related disease is a metabolic disease, and preferably, selected from type II diabetes or non-alcoholic steatohepatitis.

**19.** The method according to claim 15, wherein the ATX-related disease is selected from neuropathic pain or inflammatory pain, and preferably, the ATX-related disease is osteoarthritis-related pain.

**20.** The method according to claim 15, wherein the ATX-related disease is cancer.

**INTERNATIONAL SEARCH REPORT**

International application No.
**PCT/CN2020/113474**

**A. CLASSIFICATION OF SUBJECT MATTER**

C07D 255/00(2006.01)i；C07D 255/02(2006.01)i；C07D 401/02(2006.01)i；C07D 401/12(2006.01)i；C07D 403/12(2006.01)i；A61K 31/506(2006.01)i；A61P 35/00(2006.01)i；A61P 19/02(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 自分泌运动因子, 自霉素, 溶血磷脂酶D, 三唑, 嘧啶, 茚, 湖北生物医药产业技术研究院, triazol, pyrimidin, inden, autotaxin, ATX, LysoPLD, Hubei bio-pharmaceutical industrial technological institute

**C. DOCUMENTS CONSIDERED TO BE RELEVANT**

| Category* | Citation of document, with indication, where appropriate, of the relevant passages | Relevant to claim No. |
|---|---|---|
| X | "CAS: 1320657-08-6"<br>*REGISTRY,* 21 August 2011 (2011-08-21), | 1, 3 |
| A | "CAS: 1428046-13-2"<br>*REGISTRY,* 11 April 2013 (2013-04-11), | 1-20 |
| A | CN 103958481 A (INHIBITAXIN LTD.) 30 July 2014 (2014-07-30)<br>description paragraphs [0009] and [0235], and page 24, table 1 | 1-20 |
| A | CN 106220572 A (INHIBITAXIN LTD.) 14 December 2016 (2016-12-14)<br>entire document | 1-20 |
| A | CN 106103446 A (F. HOFFMANN-LA ROCHE AG) 09 November 2016 (2016-11-09)<br>entire document | 1-20 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 November 2020** | **09 December 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/113474**

**Box No. II** **Observations where certain claims were found unsearchable (Continuation of item 2 of first sheet)**

This international search report has not been established in respect of certain claims under Article 17(2)(a) for the following reasons:

1. ☑ Claims Nos.: **15-20**
because they relate to subject matter not required to be searched by this Authority, namely:

[1] A method of treating an ATX-related disease as claimed in claims 15-20. Claims 15-20 relate to a treatment method directly targeted at a human or animal body. Therefore, claims 15-20 do not comply with requirement of PCT Rule 39.1 (iv). The search of claims 15-20 is made based on the pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/113474**

| Patent document cited in search report | Publication date (day/month/year) | Patent family member(s) | Publication date (day/month/year) |
|---|---|---|---|
| CN 103958481 A | 30 July 2014 | WO 2013061297 A1 | 02 May 2013 |
| | | US 2016287584 A1 | 06 October 2016 |
| | | JP 6158817 B2 | 05 July 2017 |
| | | CA 2853231 C | 04 August 2020 |
| | | EP 3243815 A1 | 15 November 2017 |
| | | US 9273011 B2 | 01 March 2016 |
| | | EP 2771325 B1 | 28 June 2017 |
| | | ES 2648901 T3 | 08 January 2018 |
| | | US 2014275100 A1 | 18 September 2014 |
| | | HK 1200455 A1 | 07 August 2015 |
| | | CN 103958481 B | 30 June 2017 |
| | | JP 2014530902 A | 20 November 2014 |
| | | ES 2748656 T3 | 17 March 2020 |
| | | US 10016420 B2 | 10 July 2018 |
| | | CN 106220572 B | 13 December 2019 |
| | | CN 106220572 A | 14 December 2016 |
| | | HK 1246783 A1 | 14 September 2018 |
| | | EP 2771325 A1 | 03 September 2014 |
| | | IN 3063DEN2014 A | 15 May 2015 |
| | | CA 2853231 A1 | 02 May 2013 |
| | | EP 3243815 B1 | 10 July 2019 |
| CN 106220572 A | 14 December 2016 | WO 2013061297 A1 | 02 May 2013 |
| | | US 2016287584 A1 | 06 October 2016 |
| | | JP 6158817 B2 | 05 July 2017 |
| | | CA 2853231 C | 04 August 2020 |
| | | EP 3243815 A1 | 15 November 2017 |
| | | US 9273011 B2 | 01 March 2016 |
| | | EP 2771325 B1 | 28 June 2017 |
| | | ES 2648901 T3 | 08 January 2018 |
| | | US 2014275100 A1 | 18 September 2014 |
| | | HK 1200455 A1 | 07 August 2015 |
| | | CN 103958481 B | 30 June 2017 |
| | | JP 2014530902 A | 20 November 2014 |
| | | ES 2748656 T3 | 17 March 2020 |
| | | CN 103958481 A | 30 July 2014 |
| | | US 10016420 B2 | 10 July 2018 |
| | | CN 106220572 B | 13 December 2019 |
| | | HK 1246783 A1 | 14 September 2018 |
| | | EP 2771325 A1 | 03 September 2014 |
| | | IN 3063DEN2014 A | 15 May 2015 |
| | | CA 2853231 A1 | 02 May 2013 |
| | | EP 3243815 B1 | 10 July 2019 |
| CN 106103446 A | 09 November 2016 | AU 2015238537 B2 | 01 August 2019 |
| | | WO 2015144605 A1 | 01 October 2015 |
| | | PT 3122750 T | 30 October 2019 |
| | | EA 201691799 A1 | 30 January 2017 |
| | | US 2020216457 A1 | 09 July 2020 |
| | | HU E046820 T2 | 30 March 2020 |
| | | US 2019144457 A1 | 16 May 2019 |
| | | EP 3122750 A1 | 01 February 2017 |

Form PCT/ISA/210 (patent family annex) (January 2015)

**INTERNATIONAL SEARCH REPORT**
**Information on patent family members**

International application No.
**PCT/CN2020/113474**

| Patent document cited in search report | Publication date (day/month/year) | Patent family member(s) | | | Publication date (day/month/year) |
|---|---|---|---|---|---|
| | | CN | 106103446 | B | 30 July 2019 |
| | | JP | 2017508774 | A | 30 March 2017 |
| | | CL | 2016002310 | A1 | 24 February 2017 |
| | | HR | P20191936 | T1 | 10 January 2020 |
| | | MX | 370659 | B | 19 December 2019 |
| | | IL | 246708 | A | 28 November 2019 |
| | | US | 9802944 | B2 | 31 October 2017 |
| | | PH | 12016501632 | B1 | 06 February 2017 |
| | | CA | 2937616 | A1 | 01 October 2015 |
| | | CN | 110204550 | A | 06 September 2019 |
| | | US | 10654857 | B2 | 19 May 2020 |
| | | RS | 59477 | B1 | 31 December 2019 |
| | | UA | 118582 | C2 | 11 February 2019 |
| | | CR | 20160419 | A | 07 November 2016 |
| | | SI | 3122750 | T1 | 31 December 2019 |
| | | AR | 099824 | A1 | 24 August 2016 |
| | | AU | 2019257457 | A1 | 21 November 2019 |
| | | DK | 3122750 | T3 | 04 November 2019 |
| | | EP | 3122750 | B1 | 04 September 2019 |
| | | US | 2018111941 | A1 | 26 April 2018 |
| | | AU | 2015238537 | A1 | 28 July 2016 |
| | | SG | 11201607839U | A | 28 October 2016 |
| | | ES | 2753549 | T3 | 13 April 2020 |
| | | LT | 3122750 | T | 11 November 2019 |
| | | MX | 2016012550 | A | 14 December 2016 |
| | | JP | 2019189640 | A | 31 October 2019 |
| | | EP | 3590939 | A1 | 08 January 2020 |
| | | PE | 20161134 | A1 | 29 October 2016 |
| | | KR | 20160128345 | A | 07 November 2016 |
| | | IL | 246708 | D0 | 31 August 2016 |
| | | TW | 201625624 | A | 16 July 2016 |
| | | PL | 3122750 | T3 | 31 March 2020 |
| | | US | 2017008900 | A1 | 12 January 2017 |
| | | PH | 12016501632 | A1 | 06 February 2017 |
| | | MA | 39792 | B1 | 31 December 2019 |
| | | JP | 6553637 | B2 | 31 July 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910841159
- CN 201910846545
- WO 2014202458 A1
- WO 2014110000 A1
- WO 2014139882 A1


### Non-patent literature cited in the description


- **HIDENOBU KANDA ; REBECCA NEWTON ; RUSSELL KLEIN et al.** Autotaxin, an ectoenzyme that produces lysophosphatidic acid, promotes the entry of lymphocytes into secondary lymphoid organs[J. *Nature Immunology.,* 2008, vol. 9 (4), 415-423
- **TOBY M MAHER et al.** Rationale, design and objectives of two phase III, randomised, placebocontrolled studies of GLPG1690, a novel autotaxin inhibitor, in idiopathic pulmonary fibrosis (ISABELA 1 and 2). *BMJ Open Respiratory Research,* 2019, vol. 21 (1), 6
- McGraw-Hill Dictionary of Chemical Terms. McGraw-Hill Book Company, 1984
- **ELIEL, E ; WILEN, S.** Stereochemistry of Organic Compounds. John Wiley & Sons, Inc, 1994
- **MAEHR.** *J. Chem. Ed.,* 1985, vol. 62, 114-120